(19) Europäisches Patentamt
European Patent Office
Office européen des brevets

(11) EP 4 220 160 A1

(12) **EUROPEAN PATENT APPLICATION**
published in accordance with Art. 153(4) EPC

(43) Date of publication:
**02.08.2023 Bulletin 2023/31**

(21) Application number: **21872092.8**

(22) Date of filing: **27.08.2021**

(51) International Patent Classification (IPC):
***G01N 33/53*** *(2006.01)* ***G01N 33/531*** *(2006.01)*
***G01N 33/543*** *(2006.01)* ***G01N 33/545*** *(2006.01)*

(52) Cooperative Patent Classification (CPC):
**G01N 33/53; G01N 33/531; G01N 33/543; G01N 33/545**

(86) International application number:
**PCT/JP2021/031621**

(87) International publication number:
**WO 2022/064960 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.09.2020 JP 2020159008**
**01.03.2021 PCT/JP2021/007726**

(71) Applicant: **National University Corporation Kobe University**
**Kobe-shi, Hyogo 657-8501 (JP)**

(72) Inventors:
- **TAKEUCHI, Toshifumi**
**Kobe-shi, Hyogo 657-8501 (JP)**
- **SUNAYAMA, Hirobumi**
**Kobe-shi, Hyogo 657-8501 (JP)**
- **TAKANO, Eri**
**Kobe-shi, Hyogo 657-8501 (JP)**

(74) Representative: **Engelhard, Markus**
**Boehmert & Boehmert**
**Anwaltspartnerschaft mbB**
**Pettenkoferstrasse 22**
**80336 München (DE)**

(54) **BASE MATERIAL FOR MANUFACTURING SENSOR FOR ANALYZING DETECTION OBJECT, SENSOR FOR ANALYZING DETECTION OBJECT, AND METHOD FOR ANALYZING DETECTION OBJECT**

(57) The present disclosure provides a biosensor having improved sensitivity. The present disclosure also provides a base material for manufacturing a sensor for analyzing a detection object, said base material comprising a molecularly imprinted polymer having: a molecularly imprinted recess in which a target of the detection object is received; and a group for binding a specific bondable molecule to the target, said group being provided inside the molecularly imprinted recess. The base material is characterized in that the group for binding the specific bondable molecule is a group suited for a small substance, and/or is characterized in that the molecularly imprinted recess has a shape or structure suited for a small substance.

EP 4 220 160 A1

## Description

[Technical Field]

The present disclosure relates to the technique of sensitively detecting a detection subject on a base material. More specifically, the present disclosure relates to a base material for making a sensor for analyzing a detection subject and a manufacturing method thereof, a sensor for analyzing a detection subject and a manufacturing method thereof, and a method of analyzing a detection subject.

[Background Art]

Small Extracellular Vesicles (sEVs) such as exosome are one of the endoplasmic reticula released from a cell, which is lipid bilayer vesicle with a diameter of 20 to 200 nm. A small extracellular vesicle encapsulates a protein and a nucleic acid such as miRNA or mRNA therein, and has a protein on the surface thereof. Since a small extracellular vesicle is characterized by such a substance, it is considered that it is possible to presume what kind of cell a discharged cell is by analyzing the characteristic of the small extracellular vesicle. In addition, the presence of a small extracellular vesicle is confirmed in various body fluids, which can be relatively easily collected.

A small extracellular vesicle discharged from a cancer cell comprises a substance derived from a tumor. Therefore, it is expected that it is possible to diagnose cancer by analyzing a substance comprised in a small extracellular vesicle in a body fluid. Furthermore, since a small extracellular vesicle is actively discharged by a cell, it is expected that some sort of characteristic is exhibited even at an early stage of cancer.

[Summary of Invention]

[Solution to Problem]

When the present inventors intentionally introduced a small substance instead of an antibody in a biosensor, which comprises a polymer (molecularly imprinted polymer: MIP) having a hole that had been formed by molecular imprinting technique and is selectively introduced with an antibody in said hole, it was discovered that, regardless of the natural binding capacity (hereafter, "binding capacity" refers to affinity) of said small substance being low, the binding capacity per target would be increased to a surprising level which surpasses the case of introducing an antibody. The present disclosure was completed by carry out further examinations based on this finding.

The present disclosure comprises a base material for making a sensor for analyzing a detection subject and a manufacturing method thereof, a sensor for analyzing a detection subject and a manufacturing method thereof, and a method of analyzing a detection subject. For example, the present disclosure presents inventions in the following forms.

While an antibody used for a biosensor greatly contributes to the performance of the biosensor by its excellent specificity and affinity, the present disclosure provides improvement in the sensitivity thereof.

The present disclosure provides a biosensor with improved sensitivity.

(Item 1)

A base material for use in making a sensor for use in analyzing a detection subject, comprising a molecularly imprinted polymer having:

a molecular imprint recessed part that receives a target of a detection subject; and
a group for use in binding a binding molecule to the target provided within the molecular imprint recessed part,
wherein the group for use in binding a binding molecule is a group adapted to a small substance.

(Item 2)

A base material for use in making a sensor for use in analyzing a detection subject, comprising a molecularly imprinted polymer having:

a molecular imprint recessed part that receives a target of a detection subject; and
a group for use in binding a binding molecule to the target provided within the molecular imprint recessed part,
wherein the molecular imprint recessed part has a shape or configuration adapted to a small substance.

(Item 3)

The base material for use in making a sensor for use in analyzing a detection subject of any one of the items above, further having a group for use in binding a signal substance within the molecular imprint recessed part.

(Item 4)

The base material for use in making a sensor for use in analyzing a detection subject of any one of the items above, wherein the group for use in binding a binding molecule and the group for use in binding a signal substance are independent from one another in the molecular imprint recessed part surface.

(Item 5)

The base material for use in making a sensor for use in analyzing a detection subject of any one of the items above, wherein the group for use in binding a binding molecule and the group for use in binding a signal substance configure the functional group shown in Formula (1) below within the molecular imprint recessed part.

[Chemical 1] $-R^2-L^1-R^1$ (1)

<$R^1$ represents the group for use in binding a binding molecule, $L^1$ represents a direct binding or linking group, and $R^2$ represents a linking group comprising the group for use in binding a signal substance.>

(Item 6)

The base material for use in making a sensor for use in analyzing a detection subject of any one of the items above, wherein a linking group comprising the group for use in binding a signal substance is represented by Formulas (2) and (3) below.

[Chemical 2] $-NR^3-$ (2)

[Chemical 3]

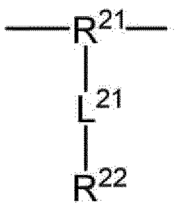

(3)

<$R^3$ represents an alkyl group with a carbon number of 1 to 8 that may comprise hydrogen or a polyalkylene glycol chain, $R^{21}$ represents a nitrogen atom or hydrocarbon, $L^{21}$ represents a direct binding or linking group, and $R^{22}$ represents the group for use in binding a signal substance.>

(Item 7)

The base material for use in making a sensor for use in analyzing a detection subject of any one of the items above, wherein the group for use in binding a binding molecule is a group selected from the group consisting of an amino group, a carboxyl group, a boronic acid group, a cis-diol group, an aldehyde group, a ketone group, an oxyamino group, a biotin (biocytin, desthiobiotin) group, a nickel-nitrilotriacetic acid-driven group, a thiol group, and a pyridyl disulfide group.

(Item 8)

The base material for use in making a sensor for use in analyzing a detection subject of any one of the items above, wherein the group for use in binding a binding molecule is a group selected from the group consisting of a nickel-nitrilotriacetic acid-driven group, a thiol group, and a pyridyl disulfide group.

(Item 9)

The base material for use in making a sensor for use in analyzing a detection subject of any one of the items above, wherein the group for use in binding a binding molecule of Item 7 is adapted to a small substance via an Fc domain binding protein of an antibody, avidin, streptavidin, NeutrAvidin, or a polymer, dendrimer, or oligoDNA comprising one or more of the binding group of any one of the items above.

(Item 10)

The base material for use in making a sensor for use in analyzing a detection subject of any one of the items above, wherein the Fc domain binding protein of an antibody is protein A, protein G, protein L, or protein A/G.

(Item 11)

The base material for use in making a sensor for use in analyzing a detection subject of any one of the items above, having an interaction group for a mold of the molecular imprint recessed part or the detection subject, within the molecular imprint recessed part.

(Item 12)

The base material for use in making a sensor for use in analyzing a detection subject of any one of the items above, wherein the binding molecule is a specific binding molecule.

(Item 13)

The base material for use in making a sensor for use in analyzing a detection subject of any one of the items above, wherein the specific binding molecule is selected from the group consisting of an antibody mimetic, a phospholipid binding protein, an artificial (polymer) receptor, and lectin.

(Item 14)

The base material for use in making a sensor for use in analyzing a detection subject of any one of the items above, wherein the detection subject is a microparticle having a film structure.

(Item 15)

The base material for use in making a sensor for use in analyzing a detection subject of any one of the items above, wherein a mold of the molecular imprint recessed part is a particle core or a biomolecule.

(Item 16)

The base material for use in making a sensor for use in analyzing a detection subject of any one of the items above, wherein a mold of the molecular imprint recessed part is a silica particle or serum protein.

(Item 17)

The base material for use in making a sensor for use in analyzing a detection subject of any one of the items above, wherein a mold of the molecular imprint recessed part is a serum protein, the detection subject is a microparticle having a film structure, and the target is a molecule expressed on a surface of the microparticle.

(Item 18)

The base material for use in making a sensor for use in analyzing a detection subject of any one of the items above, comprising a base plate and a polymer film provided on a surface of the base plate,
wherein the imprint polymer configures the polymer.

(Item 19)

The base material for use in making a sensor for use in analyzing a detection subject of any one of the items above, wherein the imprint polymer is particle-like.

(Item 20)

The base plate for use in making a sensor for use in analyzing a detection subject of any one of the items above, wherein the imprint polymer that is particle-like is immobilized on a base plate.

(Item 21)

A sensor for use in analyzing a detection subject, comprising:

the base material for use in making a sensor for use in analyzing a detection subject of any one of the items above; and
a specific binding group for a target of the detection subject bound to the group for use in binding a specific binding molecule,
wherein the specific binding molecule is a small substance group.

(Item 22)

The sensor for use in analyzing a detection subject of any one of the items above, further comprising a signal group bound to the group for use in binding a signal substance.

(Item 23)

An analysis method of a detection subject, comprising:

the step of contacting a sample comprising a detection subject to the sensor for use in analyzing a detection subject of any one of the items above and binding the detection subject to the specific binding group; and
the step of detecting a bond between the specific binding group and the detection subject.

(Item 24)

A method of manufacturing a base material for use in making a sensor for use in analyzing a detection subject, comprising:

the monolayer formation step **(1-1)** of forming a monolayer having a group for use in binding a binding molecule to a target of a detection subject and a polymerization initiating group on a surface at a base plate;
the step **(1-2)** of introducing a mold to the group for use in binding a binding molecule via a first reversible linking group;
the step **(1-3)** of adding a polymerizable monomer, using the polymerizable monomer as a substrate, using the polymerization initiating group as a polymerizable initiating agent, and synthesizing a molecularly imprinted polymer for a part of a surface of the mold to form a polymer film on the base plate surface; and
the step **(1-4)** of cleaving the first reversible linking group for conversion to the group for use in binding a binding molecule as well as removal of the mold,
wherein the group for use in binding a binding molecule is a group adapted to a small substance.

(Item 25)

A method of manufacturing a base material for use in making a sensor for use in analyzing a detection subject, comprising:

the monolayer formation step **(1-1)** of forming a monolayer having a group for use in binding a binding molecule to a target of a detection subject and a polymerization initiating group on a surface at a base plate;
the step **(1-2)** of introducing a mold to the group for use in binding a binding molecule via a first reversible linking group;
the step **(1-3)** of adding a polymerizable monomer, using the polymerizable monomer as a substrate, using the polymerization initiating group as a polymerizable initiating agent, and synthesizing a molecularly imprinted polymer for a part of a surface of the mold to form a polymer film on the base plate surface; and

the step **(1-4)** of cleaving the first reversible linking group for conversion to the group for use in binding a binding molecule as well as removal of the mold,
wherein the molecular imprint recessed part has a shape or configuration adapted to a small substance.

(Item 26)

A method of manufacturing the base material for use in making a sensor for use in analyzing a detection subject of any one of the items above, comprising:

the modification step of modifying the surface of the mold via a polymerizable functional group via a second reversible linking group,
wherein the polymerizable monomer and the polymerizable functional group are used as a substrate and the polymerization initiating group is used as a polymerizable initiating agent to synthesize a molecularly imprinted polymer for a part of the surface of the mold in the step **(1-3),** and
wherein the first reversible linking group and the second reversible linking group are cleaved for conversion to the group for use in binding a binding molecule and a group for use in binding a signal substance, respectively, as well as removal of the mold in the step **(1-4).**

(Item 27)

A method of manufacturing a base material for use in making a sensor for use in analyzing a detection subject, comprising:

the step **(1-11)** of preparing a mold of a molecular imprint wherein a surface is modified with a polymerizable functional group via a first reversible linking group;
the step **(1-12)** of using the polymerizable functional group as a substrate and synthesizing a molecularly imprinted polymer for a part of the surface of the mold; and
the step **(1-13)** of cleaving the first reversible linking group and removing the mold from the molecularly imprinted polymer to generate the group for use in binding a binding molecule,
wherein the group for use in binding a binding molecule is adapted to a small substance.

(Item 28)

A method of manufacturing a base material for use in making a sensor for use in analyzing a detection subject, comprising:

the step **(1-11)** of preparing a mold of a molecular imprint wherein a surface is modified with a polymerizable functional group via a first reversible linking group;
the step **(1-12)** of using the polymerizable functional group as a substrate and synthesizing a molecularly imprinted polymer for a part of the surface of the mold; and
the step **(1-13)** of cleaving the first reversible linking group and removing the mold from the molecularly imprinted polymer to generate the group for use in binding a binding molecule,
wherein the molecular imprint recessed part has a shape or configuration adapted to a small substance.

(Item 29)

A method of manufacturing a base material for use in making a sensor for use in analyzing a detection subject, comprising:

the step **(1-21)** of preparing a mold of a molecular imprint wherein a surface is modified with a polymerizable functional group via a third reversible linking group;
the step **(1-22)** of using the polymerizable functional group as a substrate and synthesizing a molecularly imprinted polymer for a part of the surface of the mold;
the step **(1-23)** of cleaving the third reversible linking group and removing the mold from the molecularly imprinted polymer; and
the step **(1-24)** of introducing a molecule having functional group comprising a group for use in binding a binding molecule for a target of a detection subject and a group for use in binding a signal substance via third reversible linking group,

wherein the group for use in binding a binding molecule is a group adapted to a small substance.

(Item 30)

A method of manufacturing a base material for use in making a sensor for use in analyzing a detection subject, comprising:

the step (**1-21**) of preparing a mold of a molecular imprint wherein a surface is modified with a polymerizable functional group via a third reversible linking group;
the step **(1-22)** of using the polymerizable functional group as a substrate and synthesizing a molecularly imprinted polymer for a part of the surface of the mold;
the step **(1-23)** of cleaving the third reversible linking group and removing the mold from the molecularly imprinted polymer; and
the step **(1-24)** of introducing a molecule having functional group comprising a group for use in binding a binding molecule for a target of a detection subject and a group for use in binding a signal substance via third reversible linking group,
wherein the molecular imprint recessed part has a shape or configuration adapted to a small substance.

(Item 31)

The method of manufacturing a base material for use in making a sensor for use in analyzing a detection subject of any one of the items above, further using a monomer comprising an interaction group for the mold as the substrate in the step **(1-22).**

(Item 32)

A method of manufacturing the base material for use in making a sensor for use in analyzing a detection subject of any one of the items above, wherein the binding molecule is a specific binding molecule.

(Item 33)

A method of manufacturing a sensor for use in analyzing a detection subject, comprising:

the step **(1)** of carrying out the method of manufacturing a base material for use in making a sensor for use in analyzing a detection subject of any one of the items above; and
the step **(2)** of binding the binding molecule to the group for use in binding a binding molecule.

(Item 34)

A method of manufacturing a sensor for use in analyzing a detection subject, comprising:

the step **(1)** of carrying out the method of manufacturing a base material for use in making a sensor for use in analyzing a detection subject of any one of the items above;
the step **(2)** of binding the binding molecule to the group for use in binding a binding molecule; and
the step **(3)** of binding a signal substance to the group for use in binding a signal substance.

(Item 35)

A method of diagnosing and examining a subject using the sensor for analysis of one of the items above, the diagnosis method comprising:

A) the step of collecting a sample (comprising, for example, urine, cerebrospinal fluid, sweat, nasal mucus, saliva, blood, or tear) of a subject;
B) the step of contacting the sample with the sensor for analysis;
C) the step of detecting a signal from the sensor for analysis; and
D) the step of analyzing the signal and then diagnosing or examining the subject (e.g., determining presence/absence or progression of a disease).

(Item 36)

A program that causes a computer to execute a method of diagnosing and examining a subject using a diagnosis or examination system comprising the sensor for analysis of any one of the items above, the method comprising:

A) the step of providing a sample (comprising, for example, urine, cerebrospinal fluid, sweat, nasal mucus, saliva, blood, or tear) obtained from the subject to the diagnosis or examination system;
B) the step of activating the diagnosis or examination system to contact the sample with the sensor for analysis;
C) the step of the diagnosis or examination system analyzing a signal from the sensor for analysis; and
D) the step of the diagnosis or examination system analyzing the signal and then diagnosing or detecting the subject (determining presence/absence or progression of a disease from a detection result).

(Item 37)

A record medium storing a program that causes a computer to execute a method of diagnosing or examining a subject using the sensor for analysis of any one of the items above, the method comprising:

A) the step of providing a sample (comprising, for example, urine, cerebrospinal fluid, sweat, nasal mucus, saliva, blood, or tear) obtained from the subject to the diagnosis or examination system;
B) the step of activating the diagnosis or examination system to contact the sample with the sensor for analysis;
C) the step of the diagnosis or examination system analyzing a signal from the sensor for analysis; and
D) the step of the diagnosis or examination system analyzing the signal and then diagnosing or detecting the subject (determining presence/absence or progression of a disease from a detection result).

(Item 38)

A diagnosis or examination system, the system comprising:

A) a providing unit that provides a sample (comprising, for example, urine, cerebrospinal fluid, sweat, nasal mucus, saliva, blood, or tear) obtained from the subject;
B) an activation unit that activates so as to contact the sample to the sensor for analysis;
C) a detection unit that detects a signal from the sensor for analysis; and
D) a diagnosis unit that analyzes the signal and then provides diagnosis to the subject (determines presence/absence or progression of disease from a detection result).

Item. 1

A base material for use in making a sensor for use in analyzing a detection subject, comprising a molecularly imprinted polymer having:

a molecular imprint recessed part that receives a target molecule of a detection subject; and
a group for use in binding a specific binding molecule to the target molecule provided within the molecular imprint recessed part,
wherein the group for use in binding a specific binding molecule is a specific binding protein or peptide with a molecular weight of 45,000 or less.

Item. 2

The base material for use in making a sensor for use in analyzing a detection subject of item 1, further having a group for use in binding a signal substance within the molecular imprint recessed part.

Item. 3

The base material for use in making a sensor for use in analyzing a detection subject of item 2, wherein the group for use in binding a specific binding molecule and the group for use in binding a signal substance are independent from one another in the molecular imprint recessed part surface.

Item. 4

The base material for use in making a sensor for use in analyzing a detection subject of item 2, wherein the group for use in binding a specific binding molecule and the group for use in binding a signal substance configure the functional group shown in Formula (1) below within the molecular imprint recessed part.

[Chemical 4] $-R^2-L^1-R^1$ (1)

<$R^1$ represents the group for use in binding a specific binding molecule, $L^1$ represents a direct binding or linking group, and $R^2$ represents a linking group comprising the group for use in binding a signal substance.>

Item. 5

The base material for use in making a sensor for use in analyzing a detection subject of item 4, wherein a linking group comprising the group for use in binding a signal substance is represented by Formulas (2) and (3) below.

[Chemical 5] $-NR^3-$ (2)

[Chemical 6]

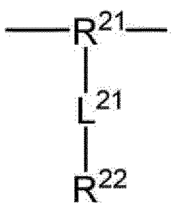

(3)

<$R^3$ represents an alkyl group with a carbon number of 1 to 8 that may comprise hydrogen or a polyalkylene glycol chain, $R^{21}$ represents a nitrogen atom or hydrocarbon, $L^{21}$ represents a direct binding or linking group, and $R^{22}$ represents the group for use in binding a signal substance.>

Item. 6

The base material for use in making a sensor for use in analyzing a detection subject of any of items 1 to 5, having an interaction group for a mold of the molecular imprint recessed part or the detection subject within the molecular imprint recessed part.

Item. 7

The base material for use in making a sensor for use in analyzing a detection subject of any of items 1 to 6, wherein the specific binding molecule is selected from the group consisting of a nanobody, an antibody mimetic, a phospholipid binding protein, and lectin.

Item. 8

The base material for use in making a sensor for use in analyzing a detection subject of any of items 1 to 7, wherein the detection subject is a microparticle having a film structure.

Item. 9

The base material for use in making a sensor for use in analyzing a detection subject of any of items 1 to 8, wherein a mold of the molecular imprint recessed part is a plasma protein, the detection subject is a microparticle having a film structure, and the target molecule is a molecule expressed on the microparticle.

Item. 10

The base material for use in making a sensor for use in analyzing a detection subject of any of items 1 to 9, comprising a base plate and a polymer film provided on a surface of the base plate,
wherein the imprint polymer configures the polymer.

Item. 11

The base material for use in making a sensor for use in analyzing a detection subject of any of items 1 to 9, wherein the imprint polymer is particle-like.

Item. 12

The base plate for use in making a sensor for use in analyzing a detection subject of item 11, wherein the imprint polymer that is particle-like is immobilized on a base plate.

Item. 13

A sensor for use in analyzing a detection subject, comprising:

the base material for use in making a sensor for use in analyzing a detection subject of any one of items 1 to 12; and
a specific binding group for a target molecule of the detection subject bound to the group for use in binding a specific binding molecule,
wherein the specific binding molecule is a specific binding protein or peptide with a molecular weight of 45,000 or less.

Item. 14

The sensor for use in analyzing a detection subject of item 13, further comprising a signal group bound to the group for use in binding a signal substance.

Item. 15

An analysis method of a detection subject, comprising:

the step of contacting a sample comprising a detection subject to the sensor for use in analyzing a detection subject of item 13 or 14 and binding the detection subject to the specific binding group; and
the step of detecting a bond between the specific binding group and the detection subject.

Item. 16

A method of manufacturing a base material for use in making a sensor for use in analyzing a detection subject, comprising:

the monolayer formation step **(1-1)** of forming a monolayer having a group for use in binding a specific binding molecule to a target of a detection subject and a polymerization initiating group on a surface at a base plate;
the step **(1-2)** of introducing a mold to the group for use in binding a specific binding molecule via a first reversible linking group;
the step **(1-3)** of adding a polymerizable monomer, using the polymerizable monomer as a substrate, using the polymerization initiating group as a polymerizable initiating agent, and synthesizing a molecularly imprinted polymer for a part of a surface of the mold to form a polymer film on the base plate surface; and
the step **(1-4)** of cleaving the first reversible linking group for conversion to the group for use in binding a specific binding molecule as well as removal of the mold,
wherein the specific binding molecule is a specific binding protein or peptide with a molecular weight of 45,000 or less.

Item. 17

A method of manufacturing the base material for use in making a sensor for use in analyzing a detection subject of item 16, comprising:

the modification step of modifying the surface of the mold via a polymerizable functional group via a second reversible linking group,

wherein the polymerizable monomer and the polymerizable functional group are used as a substrate and the polymerization initiating group is used as a polymerizable initiating agent to synthesize a molecularly imprinted polymer for a part of the surface of the mold in the step **(1-3),** and

wherein the first reversible linking group and the second reversible linking group are cleaved for conversion to the group for use in binding a specific binding molecule and a group for use in binding a signal substance, respectively, as well as removal of the mold in the step **(1-4)**.

Item. 18

A method of manufacturing a base material for use in making a sensor for use in analyzing a detection subject, comprising:

the step **(1-11)** of preparing a mold of a molecular imprint wherein a surface is modified with a polymerizable functional group via a first reversible linking group;

the step **(1-12)** of using the polymerizable functional group as a substrate and synthesizing a molecularly imprinted polymer for a part of the surface of the mold; and

the step **(1-13)** of cleaving the first reversible linking group and removing the mold from the molecularly imprinted polymer to generate the group for use in binding a specific binding molecule,

wherein the specific binding molecule is a specific binding protein or peptide with a molecular weight of 45,000 or less.

Item. 19

A manufacturing method a base material for use in making a sensor for use in analyzing a detection subject, comprising:

the step **(1-21)** of preparing a mold of a molecular imprint wherein a surface is modified with a polymerizable functional group via a third reversible linking group;

the step **(1-22)** of using the polymerizable functional group as a substrate and synthesizing a molecularly imprinted polymer for a part of the surface of the mold;

the step **(1-23)** of cleaving the third reversible linking group and removing the mold from the molecularly imprinted polymer; and

the step **(1-24)** of introducing a molecule having functional group comprising a group for use in binding a specific binding molecule for a target molecule of a detection subject and a group for use in binding a signal substance via third reversible linking group,

wherein the specific binding molecule is a specific binding protein or peptide with a molecular weight of 45,000 or less.

Item. 20

The method of manufacturing a base material for use in making a sensor for use in analyzing a detection subject of item 19, further using a monomer comprising an interaction group for the mold as the substrate in the step **(1-22).**

Item. 21

A method of manufacturing a sensor for use in analyzing a detection subject, comprising:

the step **(1)** of carrying out the method of manufacturing a base material for use in making a sensor for use in analyzing a detection subject of any of items 16 to 20; and

the step **(2)** of binding the specific binding molecule to the group for use in binding a specific binding molecule. Item. 22

A method of manufacturing a sensor for use in analyzing a detection subject, comprising:

the step **(1)** of carrying out the method of manufacturing a base material for use in making a sensor for use in analyzing a detection subject of any of items 17, 19, and 20;

the step **(2)** of binding the specific binding molecule to the group for use in binding a specific binding molecule; and

the step **(3)** of binding a signal substance to the group for use in binding a signal substance.

[Advantageous Effects of Invention]

The present disclosure provides a biosensor with improved sensitivity regardless of the natural binding capacity of an introduced specific binding molecule being low (relatively great dissociation constant Kd). Although the specific mechanism of achieving such excellent effect is not certain, it is considered that, due to the fact that the introduced specific binding molecule is a relatively small molecule, it becomes possible to distribute a plurality of specific binding molecules in a fine hole formed by molecular imprinting technique, whereby a detection subject is captured with a plurality of bonds upon sensing, raising the binding capacity of the specific binding molecule per detection subject to a surprising level (significantly reduced dissociation constant Kd).

[Brief Description of Drawings]

[Figure 1A] Figure **1A** is a schematic diagram of an example of the base material for making an analysis sensor of a detection subject of the present disclosure.

[Figure 1B-1] Figure **1B-1** is a schematic diagram of an example of the analysis sensor of a detection subject of the present disclosure corresponding to Figure **1A.**

[Figure 1B-2] Figure **1B-2** is a schematic diagram of another example of the analysis sensor of a detection subject of the present disclosure corresponding to Figure **1A.**

[Figure 2A] Figure **2A** is a schematic diagram of an example of the base material for making an analysis sensor of a detection subject of the present disclosure.

[Figure 2B-1] Figure **2B-1** is a schematic diagram of an example of the analysis sensor of a detection subject of the present disclosure corresponding to Figure **2A.**

[Figure 2B-2] Figure **2B-2** is a schematic diagram of another example of the analysis sensor of a detection subject of the present disclosure corresponding to Figure **2A.**

[Figure 3A] Figure **3A** is a schematic diagram of an example of the base material for making an analysis sensor of a detection subject of the present disclosure.

[Figure 3B] Figure **3B** is a schematic diagram of the analysis sensor of a detection subject of the present disclosure corresponding to Figure **3A.**

[Figure 4A] Figure **4A** is a schematic diagram of an example of the base material for making an analysis sensor of a detection subject of the present disclosure.

[Figure 4B] Figure **4B** is a schematic diagram of the analysis sensor of a detection subject of the present disclosure corresponding to Figure **4A.**

[Figure 5] Figure **5** is (A) a schematic diagram of the case wherein an example of the base material for making an analysis sensor of a detection subject of the present disclosure of Figure **1A** is like a film provided on a base plate, and a schematic diagram of the analysis sensor of a detection subject of the present disclosure corresponding thereto.

[Figure 6] Figure **6** is (A) a schematic diagram of the case wherein an example of the base material for making an analysis sensor of a detection subject of the present disclosure of Figure **1A** is a particle, and (B) a schematic diagram of the analysis sensor of a detection subject of the present disclosure corresponding thereto.

[Figure 7] Figure **7** is (A) a schematic diagram of the case wherein an example of the base material for making an analysis sensor of a detection subject of the present disclosure of Figure **1A** is a molecule immobilized on a base plate, and (B) a schematic diagram of the analysis sensor of a detection subject of the present disclosure corresponding thereto.

[Figure 8] Figure **8** is (A) a schematic diagram of the case wherein an example of the base material for making an analysis sensor of a detection subject of the present disclosure of Figure 2A is like a film provided on a base plate, and (B1), (B2) a schematic diagram of the analysis sensor of a detection subject of the present disclosure corresponding thereto.

[Figure 9] Figure **9** is (A) a schematic diagram of the case wherein an example of the base material for making an analysis sensor of a detection subject of the present disclosure of Figure **2A** is a particle, and (B) a schematic diagram of the analysis sensor of a detection subject of the present disclosure corresponding thereto.

[Figure 10] Figure **10** is (A) a schematic diagram of the case wherein an example of the base material for making an analysis sensor of a detection subject of the present disclosure of Figure **4A** is a particle, and (B) a schematic diagram of the analysis sensor of a detection subject of the present disclosure corresponding thereto.

[Figure 11] Figure **11** is (A) a schematic diagram of the case wherein an example of the base material for making an analysis sensor of a detection subject of the present disclosure of Figure **3A** is a particle immobilized on a base plate, and (B) a schematic diagram of the analysis sensor of a detection subject of the present disclosure corresponding thereto.

[Figure 12-1aJ Figure **12-1a** schematically shows an embodiment of preparing a mold in a manufacturing method of the base material **10a-C'** for making a sensor for analyzing a detection subject that is base plate-type which is shown in Figure **8(A).**

[Figure 12-1b] Figure **12-1b** schematically shows another embodiment of preparing a bold in a manufacturing method of the base material **10a-C'** for making a sensor for analyzing a detection subject that is base plate-type which is shown in Figure **8(A).**

[Figure 12-2] Figure **12-2** schematically shows the step **(1-1)** in a manufacturing method of the base material **10a-C'** for making a sensor for analyzing a detection subject that is base plate-type which is shown in Figure **8(A).**

[Figure 12-3a] Figure **12-3a** schematically shows the step **(1-2)** (the case of using the mold $\mathbf{60_1}$ obtained in Figure **12-1a**) in a manufacturing method of the base material **10a-C'** for making a sensor for analyzing a detection subject that is base plate-type which is shown in Figure **8(A).**

[Figure 12-3b] Figure **12-3b** schematically shows the step **(1-2)** (the case of using the mold $\mathbf{60_2}$ obtained in Figure **12-1b**) in a manufacturing method of the base material **10a-C'** for making a sensor for analyzing a detection subject that is base plate-type which is shown in Figure **8(A).**

[Figure 12-4] Figure **12-4** schematically shows the step **(1-**3) in a manufacturing method of the base material **10a-C'** for making a sensor for analyzing a detection subject that is base plate-type which is shown in Figure **8(A).**

[Figure 12-5] Figure **12-5** shows the step **(1-4)** in a manufacturing method of the base material **10a-C'** for making a sensor for analyzing a detection subject that is base plate-type which is shown in Figure **8(A).**

[Figure 12-6] Figure **12-6** schematically shows a manufacturing method of the sensor **10a-C** for analyzing a detection subject that is base plate-type which is shown in Figure **8(B).**

[Figure 13-1a] Figure **13-1a** schematically shows the step **(1-11)** (the case of using the mold $\mathbf{60_1}$) in a manufacturing method of the base material **10-P'** for making a sensor for analyzing a detection subject that is particle-like which is shown in Figure **6(A).**

[Figure 13-1b] Figure **13-1b** schematically shows the step **(1-11)** (the case of using the mold $\mathbf{60_2}$) in a manufacturing method of the base material **10-P'** for making a sensor for analyzing a detection subject that is particle-like which is shown in Figure **6(A).**

[Figure 13-2] Figure **13-2** schematically shows the step **(1-12)** in a manufacturing method of the base material **10-P'** for making a sensor for analyzing a detection subject that is particle-like which is shown in Figure **6(A).**

[Figure 13-3] Figure **13-3** schematically shows an example of the step **(1-13)** in a manufacturing method of the base material **10-P'** for making a sensor for analyzing a detection subject that is particle-like which is shown in Figure **6(A).**

[Figure 13-4] Figure **13-4** schematically shows the manufacturing method of the sensor **10-P** for analyzing a detection subject that is particle-like which is shown in Figure **6(B).**

[Figure 14-1] Figure **14-1** schematically shows the step **(1-21)** in a manufacturing method of the base material **10c-P'** for making a sensor for analyzing a detection subject that is particle-like which is shown in Figure **10(A).**

[Figure 14-2] Figure **14-2** schematically shows the step **(1-22)** in a manufacturing method of the base material **10c-P'** for making a sensor for analyzing a detection subject that is particle-like which is shown in Figure **10(A).**

[Figure 14-3] Figure **14-3** schematically shows the step **(1-23)** in a manufacturing method of the base material **10c-P'** for making a sensor for analyzing a detection subject that is particle-like which is shown in Figure **10(A).**

[Figure 14-4] Figure **14-4** schematically shows the step **(1-24)** in a manufacturing method of the base material **10c-P'** for making a sensor for analyzing a detection subject that is particle-like which is shown in Figure **10(A).**

[Figure 14-5] Figure **14-5** schematically shows the manufacturing method of the sensor **10c-P** for analyzing a detection subject that is particle-like which is shown in Figure **10(B).**

[Figure 15] Figure **15** schematically shows the step of fixing a sensor base material for analyzing a detection subject that is particle-like to a base plate during Figure **14-3** and Figure **14-4.**

[Figure 16-1] Figure **16-1** schematically shows an example (in an environment of high concentration of a mold **(60c)** of a molecular imprint recessed part **21c**) of double targeting using the sensor **10c-P** for analyzing a detection subject that is particle-like which is shown in Figure **10(B).**

[Figure 16-2] Figure **16-2** schematically shows an example (in an environment of low concentration of a mold **(60c)** of a molecular imprint recessed part **21c**) of double targeting using the sensor **10c-P** for analyzing a detection subject that is particle-like which is shown in Figure **10(B).**

[Figure 17A] Figure **17A** shows the relationship between a relative fluorescence intensity and an exosome concentration, using the analysis sensor **10a-C** (introducing Alexa Fluor 647 as a signal substance) of a detection subject that is base plate-type of Example 1.

[Figure 17B] Figure **17B** shows the relationship between a relative fluorescence intensity and an exosome concentration, using the analysis sensor that is not introduced with an affibody of Comparative Example 1.

[Figure 18] Figure **18** shows the relationship between a relative fluorescence intensity and an exosome concentration, using the analysis sensor **10a-C** (introducing rhodamine as a signal substance) of a detection subject (exosome) that is base plate-type of Example 2.

[Figure 19] Figure **19** shows the relationship between a relative fluorescence intensity and an exosome concentration, using the analysis sensor of a detection subject (exosome) that is base plate-type of Example 3 (provided that the sensor is a type of analysis sensor **$10^1$** product of a detection subject that is base plate-type utilizing a signal base plate fused to a specific binding group).

[Figure 20] Figure **20** shows a TEM image of a particle-like molecularly imprinted polymer of the analysis sensor **10-PC** of a detection subject that is particle-like and immobilized on a base plate of Example 4.

[Figure 21] Figure **21** shows the relationship between an amount of change of response and exosome solution concentration, using the analysis sensor **10-PC** of a detection subject (exosome) that is particle-like and immobilized on a base plate of Example 4.

[Figure 22] Figure **22** shows the relationship between a relative fluorescence intensity and an exosome concentration, using the analysis sensor **10b-PC** of a detection subject (exosome) that is particle-like and immobilized on a base plate of Example 5.

[Figure 23] Figure **23** shows relative fluorescence intensity change of the case of adding exosome derived from an HER2 overexpression-type breast cancer cell line regarding the analysis sensor **10b-PC** of a detection subject (exosome) that is particle-like and immobilized on a base plate of Example 5 and a sensor for analysis wherein an anti-HSA affibody is introduced instead of an anti-HER2 affibody.

[Figure 24] Figure **24** shows a TEM image of a particle-like molecularly imprinted polymer of the analysis sensor **10c-P** of a detection subject that is particle-like and immobilized on a base plate of Example 6.

[Figure 25] Figure **25** shows double targeting in solutions with different concentration ratio of exosome and HSA, using the analysis sensor **10c-P** of a detection subject (exosome) that is particle-like and immobilized on a base plate of Example 6.

[Description of Embodiments]

The present disclosure is explained below in further detail.

Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The terms and general technique used in the present disclosure are appropriately explained herein.

<1. A base material for making a sensor for analyzing a detection subject>

The base material for making a sensor for analyzing a detection subject of the present disclosure is a base material that becomes a material for making a sensor for analysis of the invention discussed below. This base material for making a sensor for analysis is configured to enable a user to easily carry out customization to a sensor capable of even more sensitive detection of a detection subject.

<1-1. Basic configuration (sensing precursor part)>

The base material for making an analysis sensor of a detection subject of the present disclosure comprises a molecularly imprinted polymer having a molecular imprint recessed part that receives a target of a detection subject and a group for binding a specific binding molecule to said target, which is provided within said molecular imprint recessed part; characterized in that said group for binding the specific binding molecule is a group adapted to a small substance, and/or characterized in that the molecular imprint recessed part has a shape or configuration adapted to a small substance. This basic configuration configures a sensing precursor part (i.e., a portion configuring a sensing part when customized to a sensor for analysis) of a base material for making an analysis sensor of a detection subject.

As used herein, "target" refers to a targeted molecule or substance. For example, a target may be a molecule or a molecular aggregate.

As used herein, "small substance" refers to any substance smaller than an antibody, which can be interchangeable with "small binding molecular substance". Since the criterion of the molecular weight is about 150,000, a molecular weight that is less than said number (i.e., less than 150,000) may be included in this concept. Preferable molecular weight includes about 140,000 or less, about 130,000 or less, about 120,000 or less, about 110,000 or less, about 100,000 or less, about 90,000 or less, about 80,000 or less, about 70,000 or less, about 70,000 or less, about 60,000

or less, about 50,000 or less, about 40,000 or less, about 30,000 or less, about 22,000 or less, about 20,000 or less, about 19,000 or less, about 18,000 or less, about 17,000 or less, about 16,000 or less, about 15,000 or less, about 14,000 or less, about 13,000 or less, about 12,000 or less, about 11,000 or less, about 10,000 or less, about 9,000 or less, about 8,000 or less, about 7,000 or less, about 6,000 or less, and the like. The lower limit of the molecular weight of a small molecule can include, about 200 or more, about 300 or more, about 400 or more, about 500 or more, and about 600 or more.

Any type of substance can be used as a small substance, which can typically include protein, polypeptide, peptide, sugar, lipid, nucleic acid, nucleic acid derivative, artificial (polymer) receptor, other organic molecules, complex molecules thereof, and the like, preferably includes protein, polypeptide, and peptide, more preferably protein or peptide.

As used herein, "artificial (polymer) receptor" refers to a molecularly imprinted polymer nanoparticle (particle size of about 10 to 30 nm) that recognizes a target, which is used for a sandwich assay or the like instead of an antibody. Evan M. Peck and Bradley D. Smith, Chapter 1: Applications of Synthetic Receptors for Biomolecules, in Synthetic Receptors for Biomolecules: Design Principles and Applications, 2015, pp. 1-38 can be referred to as such artificial receptor.

As used herein, "group for binding" or "molecular imprint recessed part" being "adapted to..." refers to the concept of being a configuration that is appropriately detected upon carrying out a detection process that sets a substance that is subjected to adaptation as a subject when used in a manufacturing base material for a sensor or a sensor wherein the "base for binding" or "molecular imprint recessed part" is comprised, regarding a substance that is subjected to adaptation (e.g., small substance, a molecule with the molecular weight of 45,000, or the like).

Typically, for example, "group for binding that is adapted to a small substance" is a group for binding that is appropriate for a small substance smaller than an antibody, wherein groups such as amino group, carboxy group, boronic acid group, cis-diol group, aldehyde group, ketone group, oxyamino group, hydrazide group, biotin (biocytin, desthiobiotin) group, nickel-nitrilotriacetic acid-driven group, thiol group, pyridyl disulfide group, metal complex, and the like may be preferably utilized. In addition, said group for binding may be adapted to a small substance via Fc domain binding proteins of an antibody such as protein A, protein G, protein L, and protein A/G, proteins such as avidin, streptavidin, and neutravidin, polymers comprising one or more of groups such as amino group, carboxy group, boronic acid group, cis-diol group, aldehyde group, ketone group, oxyamino group, hydrazide group, biotin (biocytin, desthiobiotin) group, nickel-nitrilotriacetic acid-driven group, thiol group, pyridyl disulfide group, and metal complex, dendrimer, oligo DNA, and the like.

Typically, for example, "a molecular imprint recessed part has a shape or configuration adapted to a small substance" refers to the concept of having a shape or configuration appropriate for detection when the molecular imprint recessed part is disposed in a manufacturing base material for a sensor or a sensor.

Some examples of the base material for making an analysis sensor of a detection subject of the present disclosure are schematically shown as base materials 10', 10a', 10b', and 10c' for making an analysis sensor of a detection subject in Figure 1A, Figure 2A, Figure 3A, and Figure **4A,** respectively. The base materials for making an analysis sensor of a detection subject of the present disclosure shown in these diagrams are used to make sensors **10,** $\mathbf{10_1}$**, 10a,** $\mathbf{10a_1}$**, 10b,** and **10c** for analyzing a detection subject schematically shown in Figure **1B-1,** Figure **1B-2,** Figure **2B-1,** Figure **2-2,** Figure **3B,** and Figure **4B,** respectively.

The base material **10'** for making an analysis sensor of a detection subject shown in Figure **1A** comprises a molecularly imprinted polymer **20** having a molecular imprint recessed part **21** that receives a target of a detection subject, and a group **22'** for binding a specific binding molecule to said target, which is provided within said molecular imprint recessed part **21.**

The base material **10a'** for making an analysis sensor of a detection subject shown in Figure **2A** comprises a molecularly imprinted polymer **20a** having a molecular imprint recessed part **21** that receives a target of a detection subject and a group **22'** for binding a specific binding molecule to said target and a group **23'** for signal substance binding, which are provided within the molecular imprint recessed part **21.** In other words, the base material **10a'** for making an analysis sensor of a detection subject is the same as the base material **10'** for making an analysis sensor of a detection subject of Figure **1A** excluding the point that the group **23'** for signal substance binding is further provided within said molecular imprint recessed part **21.** Furthermore, in the base material **10a'** for making an analysis sensor of a detection subject, in a molecular imprint recessed part **21** surface, the group **22'** for binding a specific binding molecule to a target and the group **23'** for binding a signal substance are independent from one another (i.e., spaced apart from one another in the molecular imprint recessed part **21** surface direction).

The base material **10b'** for making an analysis sensor of a detection subject shown in Figure **3A** comprises a molecularly imprinted polymer **20b** having a molecular imprint recessed part **21** that receives a target of a detection subject, and a group **22b'** for binding a specific binding molecule to said target and a group **23b'** for signal substance binding, which are provided within said molecular imprint recessed part **21.** In other words, the base material **10b'** for making an analysis sensor of a detection subject is the same as the base material **10a'** for making an analysis sensor of a detection subject of Figure **2A** in that the group **23b'** for signal substance binding is further provided within the

molecular imprint recessed part **21.** However, unlike the base material **10a'** for making an analysis sensor of a detection subject of Figure **2A,** in the base material **10b'** for making an analysis sensor of a detection subject, the group **22b'** for binding a specific binding molecule to a target and the group **23b'** for binding a signal substance configure a predetermined functional group **24b** within the molecular imprint recessed part **21.**

The base material **10c'** for making an analysis sensor of a detection subject shown in Figure **4A** comprises a molecularly imprinted polymer **20c** having a molecular imprint recessed part **21c** that receives a target of a detection subject, a group **22b'** for binding a specific binding molecule to a target and a group **23b'** for binding a signal substance, which are provided within said molecular imprint recessed part **21c,** and an interaction group **25** for a mold of the molecular imprint recessed part **21c.** In other words, the base material **10c'** for making an analysis sensor of a detection subject is the same as the base material **10b'** for making an analysis sensor of a detection subject of Figure **3A** in that the group **23b'** for signal substance binding is further comprised, and in that the group **22b'** for binding a specific binding molecule to a target and the group **23b'** for binding a signal substance configure a predetermined functional group **24b.** However, unlike the base material **10b'** for making an analysis sensor of a detection subject of Figure **3A,** the base material **10c'** for making an analysis sensor of a detection subject further comprises the interaction group **25** and the molecular imprint recessed part **21c** is limited to those formed by a mold that interacts with the interaction group **25.**

When the base materials **10', 10a', 10b',** and **10c'** for making an analysis sensor of a detection subject shown in Figure **1A,** Figure **2A,** Figure **3A,** and Figure **4A** are used to make sensors **10,** $\mathbf{10_1}$, **10a,** $\mathbf{10a_1}$, **10b,** and **10c** for analyzing a detection subject schematically shown in Figure **1B-1,** Figure **1B-2,** Figure **2B-1,** Figure **2B-2,** Figure **3B,** and Figure **4B,** respectively, at least a specific binding group **42** (small substance group) for a target is bound to the groups **22'** and **22b'** for binding a specific binding molecule to a target, and a signal group **43** is bound to the groups **23'** and **23b'** for binding a signal substance.

<1-2. Shape>

More specific examples of the base material **10'** for making an analysis sensor of a detection subject of the present disclosure shown in Figure **1A** are shown as base materials **10-C', 10-P',** and **10-PC'** for making an analysis sensor of a detection subject in Figure **5(A),** Figure **6(A),** and Figure **7(A).** The base materials **10-C', 10-P',** and **10-PC'** for making an analysis sensor of a detection subject of the present disclosure are used for making sensors **10-C**, **10-P,** and **10-PC** for analyzing a detection subject schematically shown in Figure **5(B),** Figure **6(B),** and Figure **7(B),** respectively.

The base material **10-C'** for making an analysis sensor of a detection subject of the present disclosure shown in Figure **5(A)** is an example of the case wherein the base material **10'** for making an analysis sensor of a detection subject of the present disclosure is like a base plate (like a chip). In other words, the base material **10-C'** for making an analysis sensor of a detection subject comprises a base plate **30** and a polymer film provided on a surface of the base plate **30,** wherein an imprint polymer **10** configures said polymer film.

The base material **10-P'** for making an analysis sensor of a detection subject of the present disclosure shown in Figure **6(A)** is an example of the case wherein the base material **10'** for making an analysis sensor of a detection subject of the present disclosure is particle-like. In other words, in the base material **10-P'** for making an analysis sensor of a detection subject, the imprint polymer **10** is in a particle-like form.

The base material **10-PC'** for making an analysis sensor of a detection subject of the present disclosure shown in Figure **7(A)** is an example of the case wherein the base material 10' for making an analysis sensor of a detection subject of the present disclosure is particle-like and immobilized on a base plate (chip). In other words, the base material **10-P'** for making an analysis sensor of a detection subject shown in Figure **6(A)** is immobilized on the base plate **30** in the base material **10-PC'** for making an analysis sensor of a detection subject shown in Figure **7(A)**

A preferable example of the shape of a molecularly imprinted polymer includes a particle-like shape. The point of a molecularly imprinted polymer being particle-like not only enables a sensor for analyzing a detection subject to be in a base plate (chip)-like shape by being immobilized on a base plate like the base material **10-PC'** for making an analysis sensor of a detection subject shown in Figure **7(A)** discussed above, but also enables the form wherein a sensor for analyzing a detection subject is free (e.g., dispersed in a dispersion medium) by being in a non-immobilized state like the base material **10-P'** for making an analysis sensor of a detection subject shown in Figure **6(A)** discussed above, which is excellent in terms of versatility.

While the average particle size of a molecularly imprinted polymer of the case of being particle-like differs in accordance with the size, purpose, and the like of a detection subject or a target of the detection subject, the size includes, in terms of, for example, volume average particle size measured by dynamic light scattering method (DLS), for example, 5 to 500 nm, preferably 8 to 170 nm, more preferably 10 to 100 nm, further preferably 15 to 50 nm, even more preferably 20 to 30 nm. In addition, in terms of Z average particle size measured by DLS, the size includes, for example, 5 to 500 nm, preferably 50 to 350 nm, more preferably 80 to 200 nm, further preferably 100 to 140 nm, even more preferably 120 to 170 nm. Furthermore, in terms of number average particle size measured by a transmission electron microscope

(TEM), the size includes, for example, 5 to 150 nm, preferably 10 to 100 nm, more preferably 10 to 35 nm or 45 to 90 nm, further preferably 20 to 30 nm. In addition, the number average particle size measured by a transmission electron microscope (TEM) uses the average value of the long axis and the short axis of the particle as the grain size in the transmission electron microscope, using the average value of said grain size of 100 randomly selected particles.

Any of base materials **10a', 10b',** and **10c'** for making an analysis sensor of a detection subject of the present disclosure shown in Figure **2A,** Figure **3A,** and Figure **4A** may also be configured as being base plate (chip)-like, being particle-like, or being particle-like and immobilized on a base plate. Some of the examples thereof are schematically shown as base materials **10a-C', 10a-P', 10c-P',** and **10b-PC'** for making an analysis sensor of a detection subject in Figure **8(A),** Figure **9(A),** Figure **10(A),** and Figure **11(A),** respectively. The base materials for making an analysis sensor of a detection subject of the present disclosure shown in these diagrams are used to make the sensors for analyzing a detection subject schematically shown in Figure **5(B),** Figure **6(B),** Figure **7(B),** Figure **8(B1)** and **(B2),** Figure **9(B),** Figure **10(B),** Figure **11(B),** respectively.

When base materials **10-C', 10-P', 10-PC', 10a-C', 10a-P', 10c-P',** and **10b-PC'** for making an analysis sensor of a detection subject shown in Figure **5(A),** Figure **6(A),** Figure **7(A),** Figure **8(A),** Figure **9(A),** Figure **10(A),** and Figure **11(A)** are used to make the analysis sensors **10-C**, **10-P, 10-PC, 10a-C,** **$10a_1$-C**, **10a-P, 10c-P,** and **10b-PC** of a detection subject shown in Figure **5(B),** Figure **6(B),** Figure **7(B),** Figure **8(B1)** and **(B2),** Figure **9(B),** Figure **10(B),** and Figure **11(B),** at least a specific binding group **42** (small substance group) for a target is bound to groups **22'** and **22b'** for binding a specific binding molecule to a target, and a signal group **43** is bound to groups **23'** and **23b'** for binding a signal substance.

<1-3. Each constituent element>

Each element configuring these base materials for making an analysis sensor of a detection subject is discussed in detail below.

<1-3-1. Molecular imprint recessed part>

Molecular imprint recessed parts **21** and **21c** are portions that provide a sensor field in the sensor for analyzing a detection subject of the present disclosure, which is formed to receive a target of a detection subject. Specifically, the molecular imprint recessed parts **21** and **21c** are portions wherein a polymer is molded by a mold (molds **60** and **60c** discussed below) used in a molecularly imprinted polymerization method, having the shape that corresponds to a part of the surface shape of said mold. The recessed parts **21** and **21c** at least should only be formed in a size that can receive a target of a detection subject. In addition, the recessed parts **21** and **21c** have the shape of configuration wherein the molecular imprint recessed part is adapted to a small substance.

The molecular imprint recessed parts **21** and **21c** being in a "size that can receive a target of a detection subject" refers to the point that, when the specific binding group **42** for a target is introduced to form a sensor for analysis, the molecular imprint recessed parts **21** and **21c** are opened to a sufficient size to the extent that enables at least a part of the detection subject (e.g., at least a specific binding site for the specific binding group **42** in the target **72** of the detection subject **70** discussed below) to enter within the molecular imprint recessed parts **21** and **21c** to approach and bind to a specific binding molecule.

Therefore, the recessed parts **21** and **21c** may use a substance that is the same size as a detection subject or a target of the detection subject, or a substance of which size is greater than the detection subject, as a mold. Preferably, the recessed parts **21** and **21c** use a substance of which size is greater than a detection subject or a target of the detection subject as a mold.

<1-3-2. A group for binding a specific binding molecule to a target>

The groups **22'** and **22b'** for binding a specific binding molecule to a target are groups that enables introduction of a specific binding molecule to a target by binding a specific binding group for a target (the specific binding group **42** in Figure **1B-1,** Figure **1B-2,** Figure **2B-1,** Figure **2B-2,** Figure **3B,** and Figure **4B**). A user can freely select a target, and can introduce a specific binding molecule for the selected target to the groups **22'** and **22b'** for binding.

Herein, a specific binding molecule is a binding molecule having high affinity/selectivity to a target.

In addition, when the base material for making a sensor for analysis of the present disclosure comprises a base plate **30** (e.g., in the case of base materials **10-C', 10-PC', 10a-C', 10b-PC',** and the like for making an analysis sensor of a detection subject of the present disclosure shown in Figure **5(A),** Figure **7(A),** Figure **8(A),** and Figure **11(A)** in the depicted embodiment), it is also possible to carry out customization so as to enable analysis of a detection subject using a plurality of types of specific binding groups in one base plate **30** by introducing one type of specific binding molecule to one of the recessed part **21** and **21c** on one base plate **30** and introducing another type of specific binding molecule

to the other of the recessed part **21** and **21c** (i.e., one type of specific binding molecule and a molecule with a different specific binding group).

In addition, the specific method of introducing a plurality of types of specific binding molecules to one base plate **30** includes a method of fractionating a region on the base plate **30** into a plurality of regions and introducing different specific binding molecules to each fractionated region, and the like. The method of fractionation includes: a method of providing on a base plate **30** a portion where a polymer film (e.g., the film-like molecularly imprinted polymers **20** and **20a** of Figure **5(A)** and Figure **8(A)**) or polymer particle (e.g., the particle-like molecularly imprinted polymers **20** and **20b** of Figure **7(A)** and Figure **11(A)**) consisting of a molecularly imprinted polymer is absent; a method of providing a portion where a recess part **21** is absent on a polymer film (e.g., the film-like molecularly imprinted polymers **20** and **20a** of Figure **5(A)** and Figure **8(A)**) consisting of a molecularly imprinted polymer; a method of projecting a wall with a polymer or the like on a polymer film (e.g., the film-like molecularly imprinted polymers **20** and **20a** of Figure **5(A)** and Figure **8(A)**) consisting of a molecularly imprinted polymer; and the like.

As a specific example of such customization, a specific binding molecule that binds to a surface protein of a specific small extracellular vesicle is introduced to one of the recessed parts **21** and **21c** and a specific binding molecule that binds to a specific protein (protein not expressed on the surface of a small extracellular vesicle) is introduced to the other of the recessed parts **21** and **21c** to enable analysis of both a small extracellular vesicle and said protein on one base material **30.** In addition, as another specific example of customization, a specific binding molecule that binds to a surface protein A of a specific small extracellular vesicle is introduced to one of the recessed parts **21** and **21c** and a specific binding molecule that binds to a surface protein B of said specific small extracellular vesicle is introduced to the other recessed part **31** to enable analysis of different types of surface proteins on a small extracellular vesicle on one base material **30.** In addition, a specific binding molecule for a substance expressed on an extracellular vesicle **A'** derived from a cell strain **A** is introduced to one recessed part and a specific binding molecule for a substance expressed on an extracellular vesicle **B'** derived from a cell strain **B** is introduced the other to enable analysis of a small extracellular vesicle derived from different cell strains on one base material. These specific examples can be applied not only to different types of surface proteins, but also to different types of surface targets (e.g., protein, sugar chain, and the like).

In the present disclosure, a plurality of groups **22'** and **22b'** for binding a specific binding molecule to a target are provided per one molecule imprint recessed part **21** or **21c.**

The groups **22'** and **22b'** for binding a specific binding molecule are selected from groups commonly known as binding functional groups without any particular restriction.

A group that can form a reversible linking group (the first reversible linking group **22** discussed below) is selected among groups generally known as binding functional groups as the group **22'** for binding a specific binding molecule of the groups **22'** and **22b'** for binding a specific binding molecule. A reversible linking group refers to a group of direct binding or bivalent or more wherein cutting (cleavage) and binding are reversible. It does not matter whether the binding form of a reversible portion of the reversible linking group (the first reversible linking group **22** discussed below) is a covalent bond or a non-covalent bond. The specific example of the group **22'** for binding a specific binding molecule includes the groups shown in column **1-1** of Table **1** below, and the specific example of a corresponding reversible linking group (the first reversible linking group **22** discussed below) includes, but not limited to, the groups shown in column **1-3** of Table **1** below.

[Table 1-1]

| 1-1 | 1-2 | 1-3 | 1-4 |
|---|---|---|---|
| Binding functional group | Binding functional group | Reversible linking group | Form of bond of a reversible portion |
| (Group 22' for binding a specific binding molecule to a target molecule) | (Binding group 22") | (First reversible linking group 22) | |
| (Group 23' for binding a signal substance) | (Binding group 23") | (Second reversible linking group 23) | |
| (Binding group 26b') | (Binding group 26b") | (Third reversible linking group 26b) | |
| thiol group, disulfide group, pyridyl disulfide group | thiol group, disulfide group, pyridyl disulfide group | disulfide group | Covalent bond |
| boronyl group | sugar group (cis-diol group) | boronic acid cis diol ester | Covalent bond |
| carbonyl group/ aldehyde group | amino group | imino bond group | Covalent bond |
| amino group | carbonyl group/ aldehyde group | | |
| carboxyl group | hydroxyl group | carboxylic acid ester group | Covalent bond |
| hydroxyl group | carboxyl group/ carboxylic acid-active ester group | | |
| aminooxy group | carboxy group/ aldehyde group | oxime group | Covalent bond |
| carbonyl group/ aldehyde group | aminooxy group | | |
| carboxy group/ aldehyde group | hydroxyl group | acetal group | Covalent bond |
| hydroxyl group | carboxy group/ aldehyde group | | |

[Table 1-2]

| | | | |
|---|---|---|---|
| metal complex (Ni-NTA (nickel-nitrilotriacetic acid-driven group) or the like) | polyhistidine (His) tag (6 × His, 8 × His, 10 × His or the like) | coordinate bond | non-covalent bond |
| basic group (amino group, cyclic secondary amino group (e.g., pyrrolidyl group), piperidi group, pyridyl group, imidazole group, guanidine group, or the like) | acidic group (carboxyl group/ carboxylic acid-active ester group, or the like) | hydrogen bond | non-covalent bond |
| hydroxyl group | | | |
| acidic group | basic group | | non-covalent bond |
| (carboxyl group, or the like) | amino group, cyclic secondary amino group (e.g., pyrrolidyl group, piperidi group), pyridyl group, imidazole group, guanidine group, or the like), hydroxyl group | | |
| aromatic groups (phenyl group such as aminophenyl group, naphthyl group such as aminonaphthyl group, pyridyl group, or the like) | aromatic groups (phenyl group such as aminophenyl group, naphthyl group such as aminonaphthyl group, pyridyl group, or the like) | hydrophobic bond | non-covalent bond |
| hydrazide | carbonyl group/ aldehyde group | hydrazone bond | covalent bond |
| carbonyl group/ aldehyde group | hydrazide | | |
| avidin (NeutrAvidin, streptavidin) | biotin (biocytin, desthiobiotin) | avidin-biotin bond | non-covalent bond |
| biotin (biocytin, desthiobiotin) | avidin (NeutrAvidin, streptavidin) | | |

[Table 1-3]

| | | | |
|---|---|---|---|
| glutathione (GSH) | glutathione-S-transferase (GST) | glutathione (GSH)-glutathione-S-transferase (GST) bond | non-covalent bond |
| glutathione-S-transferase (GST) | glutathione (GSH) | | |
| Phos-tag | phosphate ion | coordinate bond | non-covalent bond |
| phosphate ion | Phos-tag | | |

Among these binding functional groups, the group **22'** for binding a specific binding molecule is preferably a reversible linking group with a covalent bond or a coordinate bond. A reversible linking group with a covalent bond or a coordinate bond is relatively stable compared to a hydrogen bond and a hydrophobic bond, which is stably maintained also at the time of polymerization reaction, which makes formation of the molecular imprint recessed parts **21** and **21c** easy, but is also easily cut by a reducing agent, heating with a relatively low temperature, hydrolysis under a relatively mild condition, light irradiation, or the like, and thus removal of a mold after polymerization reaction is also easy.

Furthermore, among these binding functional groups, a suitable example of the group **22'** for binding a specific binding molecule includes a metal complex, more preferably a nickel complex, further preferably a nickel-nitrilotriacetic acid-driven group, and the like. The case wherein such group **22'** for binding a specific binding molecule is suitable includes the case wherein a molecularly imprinted polymer of a base material for making an analysis sensor of a detection subject configures a polymer film (e.g., the case of the base materials **10-C', 10a-C'** and the like for making an analysis sensor of a detection subject shown in Figure **5(A)** and Figure **8(A)**).

In addition, among these binding functional groups, other suitable examples of the group **22'** for binding a specific binding molecule includes a thiol group or a pyridyl disulfide group, more preferably a thiol group. The case wherein such group **22'** for binding a specific binding molecule is suitable includes the case wherein a molecularly imprinted polymer of a base material for making an analysis sensor of a detection subject is polymer-like (e.g., the case of base materials **10-P', 10-PC', 10a-P'** and the like for making an analysis sensor of a detection subject shown in Figure **6(A),** Figure **7(A)** and Figure **9(A)).**

Between the groups 22' and **22b'** for binding a specific binding antibody, a group that can form a reversible or non-reversible linking group (the linking group **22b** discussed below) is selected among groups commonly known as binding functional groups as the group **22b'** for binding a specific binding molecule. From the viewpoint of stably binding a specific binding molecule (the specific binding molecule **R42** discussed below), the group **22b'** for binding a specific binding molecule is preferably a group that can form a reversible or non-reversible linking group (the linking group **22b** disclosed below) with a covalent bond. The specific example of such group **22b'** for binding a specific binding molecule includes, but not limited to, the groups shown in column **2-1** of Table **2** below.

[Table 2-1]

| 2-1 | 2-2 |
|---|---|
| Binding functional group | Corresponding binding functional group |
| (Group 22b' for binding a specific binding molecule to a target molecule) | (Binding group 22b") |
| (Group binding group 23b' for binding a signal substance) | (Binding group 23b") |
| (Binding groups BG22", BG23", BG1', and BG6") | (Binding groups BG22', BG23', BG1", and BG6') |
| amino group (monovalent amino group, bivalent amino group, or the like) | carboxylic acid-active ester group (active ester group using N-hydroxysuccinimide, nitrophenol, or pentafluorophenol; carbamic acid active ester group such as NHS carbamate); carboxyl group; aldehyde group; isocyanate group; isothiocyanate group; epoxy group; maleimide group; or the like |
| amino group | carboxyl group |
| carboxyl group | amino group |
| sugar group (cis-diol group) | boronyl group |
| boronyl group | sugar group (cis-diol group) |
| thiol group | unsaturated carbon group lacking an enectron such as maleimide group or acrylate ester; iodoacetamide group; thiol group; pyridyl disulfide group; alkene (vinyl sulfone) /alkyne group for radical addition click chemistry; a thioester group for a native chemical ligation method; or the like |

[Table 2-2]

| | |
|---|---|
| unsaturated carbon group lacking an enectron such as maleimide group or acrylate ester; iodoacetamide group; thiol group; pyridyl disulfide group; alkene (vinyl sulfone) /alkyne group for radical addition click chemistry; a thioester group for a native chemical ligation method; or the like | thiol group |
| carbonyl group/ aldehyde group | amino group |
| amino group | carbonyl group/ aldehyde group |
| aminooxy group | carbonyl group/ aldehyde group |
| carbonyl group/ aldehyde group | aminooxy group |
| carbonyl group/ aldehyde group | hydroxyl group |
| hydroxyl group | carbonyl group/ aldehyde group |
| hydroxyl group, phenolic hydroxyl group | carboxylic acid-active ester group |
| hydrazide | carbonyl group/ aldehyde group |
| carbonyl group/ aldehyde group | hydrazide |
| avidin (NeutrAvidin, streptavidin) | biotin (biocytin, desthiobiotin) |
| biotin (biocytin, desthiobiotin) | avidin (NeutrAvidin, streptavidin) |
| glutathione (GSH) | glutathione-S-transferase |
| glutathione-S-transferase | glutathione (GSH) |

In the present disclosure, chemical modification may be carried out to a binding group made by being chemically modified.

Among these binding functional groups, suitable examples of the group **22b'** for binding a specific binding molecule includes a thiol group or a pyridyl disulfide group, more preferably a pyridyl disulfide group.

<1-3-3. A group for binding a signal substance>

The groups **23'** and **23b'** for binding a signal substance are groups that can introduce a signal substance by binding a signal group (the signal group **43** of Figure **2B-1,** Figure **2B-2,** Figure **3B** and Figure **4B**). A user can freely select a signal substance, and introduction to the groups **23'** and **23b'** is possible.

In the present disclosure, it is possible to provide a plurality of groups **23'** and **23b'** for binding a signal substance per one molecular imprint recessed part **21** or **21c.**

Each of the groups **23'** and **23b'** for binding a signal substance is selected from those commonly known as binding functional groups with no particular restriction as long as the groups are different from the groups **22'** and 22b' for binding a specific binding molecule.

Among the groups **23'** and **23b'** for binding a signal substance, a group that can form a reversible linking group (the second reversible linking group **23** discussed below) is selected among groups commonly known as binding functional groups as the group **23'** for binding a signal substance. It does not matter whether the binding form of the reversible portion of the reversible linking group (the second reversible linking group **23** discussed below) is a covalent bond or a non-covalent bond. A specific example of the group **23'** for binding a signal substance includes the groups shown in column **1-1** of Table **1** above, and a specific example of a corresponding reversible linking group (the second reversible linking group **23** discussed below) includes the groups shown in column **1-3** of Table **1** above.

Among these binding functional groups, the group **23'** for binding a signal substance is preferably a group that forms a reversible linking group with a covalent bond or a coordinate bond. A reversible linking group with a covalent bond or a coordinate bond is relatively stable compared to a hydrogen bond and a hydrophobic bond, which is stably maintained also at the time of polymerization reaction, which makes formation of the molecular imprint recessed parts **21** and **21c** easy, but is also easily cut by a reducing agent, heating with a relatively low temperature, hydrolysis under a relatively mild condition, light irradiation, or the like, and thus removal of a mold after polymerization reaction is also easy.

Furthermore, among these binding functional groups, an appropriate example of the group **23'** for binding a signal substance includes a group that forms a reversible linking group with a covalent bond, more preferably a thiol group or a pyridyl disulfide group, further preferably a thiol group.

Among groups **23'** and **23b'** for binding a signal substance, a group that can form a reversible or non-reversible linking group (the linking group **23b** discussed below) is selected among groups commonly known as binding functional groups as the group **23b'** for binding a signal substance. From the viewpoint of stably binding a signal substance (the signal substance **R43** discussed below), the group **23b'** for binding a signal substance is preferably a group that can form a reversible or non-reversible linking group (the linking group **23b** discussed below) with a covalent bond. A specific example of such a group **23b'** for binding a signal substance includes the groups shown in column **2-1** of Table **2** above.

Among these binding functional groups, a suitable example of the group **23b'** for binding a signal substance includes an amino group.

<1-3-4. Functional group>

The above-mentioned group **22b'** for binding a specific binding molecule and the group **23b'** for binding a signal substance configure a functional group **24b.** Since the functional group **24b** comprises both the group 22b' for binding a specific binding molecule and the group 23b' for binding a signal substance in one group, it is easy to closely provide these groups to a base material for making an analysis sensor of a detection subject. The act of closely providing the group 22b' for binding a specific binding molecule and the group 23b' for binding a signal substance in the base material for making an analysis sensor of a detection subject is, in other words, the act of closely providing a specific binding group **42** and a signal group **43** in an analysis sensor of a detection subject, whereby it is possible to more sensitively obtain signal change when the analysis sensor of a detection subject recognizes a detection subject.

In the depicted embodiment, while the group **22b'** for binding a specific binding molecule is positioned on the tip side of the functional group **24b** and the binding group **23b'** of a signal substance is positioned on the base end side **of** the functional group **24b,** the positional relationship between the group **22b'** for binding a specific binding molecule and the binding group **23b'** of a signal substance in the functional group **24b** is not limited thereto as long as the positional relationship enables specific binding of the specific binding group **42** bound to the group **22b'** for binding a specific binding molecule to a target of the detection subject and causes the signal group **43** bound to the group **23b'** for binding a signal substance to receive an environmental change upon specific binding. For example, it is possible to reverse with an embodiment wherein the positional relationship between the group **22b'** for binding a specific binding molecule and the binding group **23b'** of a signal substance is depicted. However, from the viewpoint of obtaining a higher recognition capacity by achieving an easier specific binding between a target of a detection subject and the specific binding group **42** and/or from the viewpoint of more highly sensitively obtaining a signal change by more certainly changing the environment of the signal group **43** upon the specific binding between a target of a detection subject and a specific binding group **42,** it is preferable that the group **22b'** for binding a specific binding molecule is positioned on the tip side of the functional group **24b** and the binding group **23b'** of a signal substance is positioned on the base end side of the functional group **24b** as depicted.

A more specific structure of the functional group **24b** includes the group represented by Formula **(1)** below.

[Chemical 7] $-R^2-L^1-R^1$ (1)

In Formula **(1),** $R^1$ represents a group for binding a specific binding molecule, $L^1$ represents a direct binding or linking group, and $R^2$ represents a linking group comprising a group for binding a signal substance.

The group $R^1$ for binding a specific binding molecule is as discussed in "1-3-2. A group for binding a specific binding molecule to a target" above.

The direct binding or linking group $L^1$ is interposed between the group $R^1$ for binding a specific binding molecule and the linking group $R^2$ comprising a group for binding a signal substance. It is more preferable that $L^1$ is a linking group from the viewpoint of more effectively suppressing interference of the signal group 43 that binds to a group for binding a signal substance comprised in the linking group $R^2$ and specific recognition of a detection subject relative to the specific binding group **42** that binds to the group $R^1$ for binding a specific binding molecule when a sensor for analyzing a detection subject recognizes a detection subject.

When $L^1$ is a linking group, said linking group includes an alkylene group (e.g., C1 to 8 alkylene group, or

polyoxy C1 to 3 alkylene C1 to 8 alkylene group) that may comprise a polyoxyalkylene group, carbonyl group, ester bond (-COO- or -OCO-), amide bond (-CONH- or -NHCO-), and a group consisting of any combination thereof. From the viewpoint of molecular design and the like, said linking group includes, preferably a combination of an alkylene group that may comprise a polyoxyalkylene group and an ester bond and/or an amide bond, more preferably a combination of an alkylene group that may comprise a polyoxyalkylene group and an ester bond or an amide bond, further preferably a combination of one or two or more alkylene group (the total of the carbon number of all alkylene groups comprised in said linking group is, for example, 1 to 8, preferably 2 to 6, more preferably 3 to 5, especially preferably 4) and an ester bond or an amide bond.

The group for binding a signal substance in the linking group $R^2$ comprising the group for binding a signal substance is as discussed in "1-3-3. A group for binding a signal substance" above. The linking group $R^2$ comprising a group for binding a signal substance is a non-branched or branched bivalent group.

When the linking group $R^2$ is non-branched, a signal substance binding group itself makes a linking group $R^2$ (i.e., the linking group $R^2$ consists of a signal substance binding group). Such a non-branched linking group $R^2$, i.e., a signal substance binding group, includes the bivalent amino group represented by Formula **(2)** below.

[Chemical 8] $-NR^3-$ (2)

In Formula **(2),** $R^3$ represents hydrogen or an alkyl group with the carbon number of 1 to 8 that may comprise a polyoxyalkylene group (e.g., C1 to 8 alkylene group, or polyoxy C1 to 3 alkylene C1 to 8 alkylene group), preferably hydrogen.

When the linking group $R^2$ is branched, the signal substance binding group is a monovalent group that exists in a branch. Such a linking group $R^2$ includes the bivalent group represented by Formula **(3)** below.

[Chemical 9]

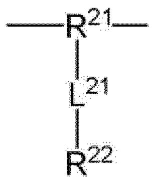

(3)

In Formula **(3),** $R^{21}$ represents nitrogen atom or carbohydrate, $L^{21}$ represents a direct binding or linking group, and $R^{22}$ represents a group for binding a signal substance.

When nitrogen atom or carbohydrate $R^{21}$ is carbohydrate, $R^{21}$ is carbohydrate represented by $-C-R^{211}$. Although not particularly limited, $R^{211}$ includes, for example, hydrogen atom or an alkyl group with the carbon number of 1 to 8 that may comprise a polyoxyalkylene group (e.g., C1 to 8 alkyl group, or polyoxy C1 to 3 alkylene C1 to 8 alkyl group), preferably hydrogen atom.

From the viewpoint of more effectively suppressing interference of the signal group **43** that binds to the group $R^{22}$ for binding a signal substance and specific recognition of a detection subject with respect to the specific binding group **42** that binds to the group $R^1$ for binding a specific binding molecule when a sensor for analyzing a detection subject recognizes a detection subject, the direct binding or linking group $L^{21}$ is more preferably a linking group.

When the direct binding or linking group $L^{21}$ is a linking group, said linking group includes an alkylene group (e.g., C1 to 8 alkylene group, or polyoxy C1 to 3 alkylene C1 to 8 alkylene group) that may comprise a polyoxyalkylene group, carbonyl group, ester bond (-COO- or -OCO-), amide bond (-CONH- or -NHCO-), and a group consisting of any combination thereof. From the viewpoint of molecular design and the like, said linking group includes preferably an alkylene group that may comprise a polyoxyalkylene group, more preferably an alkylene group with the carbon number of 1 to 6 (preferably 1 to 4, more preferably 1 to 3, especially preferably 2).

The group $R^{22}$ for binding a signal substance is as discussed in "1-3-3. A group for binding a signal substance" above.

A specific example that is not limited to the group represented by the Formula **(1)** above includes the following group.

[Compound 10]

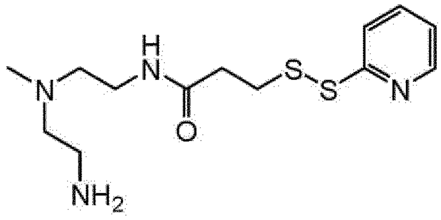

(1a)

<1-3-5. Interaction group>

The interaction group **25** in the base material **10c** for making an analysis sensor of a detection subject shown in Figure **4A** is a group that interacts with an interaction site (the interaction site **65c** discussed below) in a mold of the molecular imprint recessed part **21c.** The mold (the mold having the interaction site **65c** discussed below) corresponding to the interaction group **25** includes a biomolecule such as protein or glycoprotein.

A specific example of the interaction group **25** is designed in accordance with the surface state of a corresponding mold, i.e., an interaction group of the mold. For example, the specific example includes a group that can form a hydrogen bond, electrostatic interaction, or weak covalent bond, or the like due to the interaction with a specific amino acid residue or sugar residue, or the like configuring a protein, or glycoprotein, or the like that forms the mold, and a group that can form a hydrophobic bond with a specific hydrophobic region of a protein that forms a mold, and the like.

A specific example of the interaction group **25** includes <1> a substituted or unsubstituted amino group, <2> a substituted or unsubstituted aromatic group, <3> an amidino group ($-C(=NH)NH_2$), <4> a guanidino group ($-NHC(=NH)NH_2$), <5> a carboxyl group, <6> a sulfo group, and/or <7> a bornyl group ($-B(OH)_2$). The aromatic group in <2> the substituted or unsubstituted aromatic group comprises <21> a substituted or unsubstituted nitrogen-containing aromatic group and <22> a substituted or unsubstituted nitrogen-free aromatic group, and includes <25> a substituted or unsubstituted aryl group (<251> a nitrogen-containing aryl group and <252> a nitrogen-free aryl group) and <26> a substituted or unsubstituted arylene group (<261> a nitrogen-containing arylene group and <262> a nitrogen-free arylene group). The interaction group 25 may be a group consisting of any of these groups, and may be a group comprising a combination of two or more types selected from these groups. The combination of the interaction group 25, a corresponding interaction site (the interaction site **65c** discussed below), and the type of non-covalent bond formed by the interaction thereof is shown in the Table below, but the combination is not limited thereto.

[Table 3]

| 3-1 | 3-2 | 3-3 |
|---|---|---|
| Interaction group 25 | Interaction site 65c in a mold | Type of non-covalent bond |
| basic acid (substituted or unsubstituted amino group, substituted or unsubstituted nitrogen-containing aromatic group, amidino group, guanidion group, or the like) | acidic amino acid residue (carboxyl group or the like) | Non-covalent bond (hydrogen bond, electrostatic interaction) |
| acidic group (carboxyl group, sulfo group) | acidic amino acid residue (amino group or the like) | Non-covalent bond (hydrogen bond, electrostatic interaction) |
| substituted or unsubstituted aromatic group (substituted or unsubstituted aryl group, arylene group, or the like) | aromatic amino acid residue (phenyl group, indol group, imidazoyl group, or the like) | Non-covalent bond (hydrophobic interaction) |
| substituted or unsubstituted aromatic group (substituted or unsubstituted aryl group, arylene group, or the like) | hydrophobic region | Non-covalent bond (hydrophobic interaction) |
| bornyl group | sugar group (cis diol group) | Weak covalent bond (boronic acid cis diol ester group) |

In <1> the substituted or unsubstituted amino group, a substituent in the substituted amino group includes: a straight chain or branched alkyl group, substituted or unsubstituted aralkyl group with the carbon number of 1 to 8; an

alkenyl group with the carbon number of 4 to 6 that configures a cyclic secondary amino group; a substituted or unsubstituted aryl group; and the like. A specific example of the substituted or unsubstituted aryl group includes groups included in <251> the substituted or unsubstituted nitrogen-containing aryl group and <252> the substituted or unsubstituted nitrogen-free aryl group discussed below. A specific example of <1> the substituted or unsubstituted amino group includes: amino group (unsubstituted); secondary amino groups such as N-methylamino group, N-ethylamino group, N-n-propylamino group, N-isopropylamino group, N-n-butylamino group, N-isobutylamino group, N-tert-butylamino group, N-benzylamino group, N-phenylamino group, N-mesylamino group, and N-tosylamino group; secondary amino groups such as N,N-dimethylamino group, N,N-diethylamino group, N,N-dibenzylamino group, N-ethyl-N-methylamino group, N,N-di-n-propylamino group, N,N-diisopropylamino group, N,N-diphenylamino group, N-methyl-N-phenylamino group, N-mesyl-N-benzylamino group, and N-mesyl-N-methylamino group; cyclic secondary amino groups such as piperidyl group, and pyrrolidyl group; and the like. Among these substituted or unsubstituted amino groups, preferably a cyclic secondary amino group is included, and more preferably a pyrrolidyl group is included.

Among <2> the substituted or unsubstituted aromatic groups, the nitrogen-containing aryl group in <251> the substituted or unsubstituted nitrogen-containing aryl group includes a nitrogen-containing aryl group with the carbon number of 2 to 12. The substituent in the substituted nitrogen-containing aryl group includes a straight chain or branched alkyl group with the carbon number of 1 to 8, and the like. A specific example of <251> the substituted or unsubstituted nitrogen-containing aryl group includes a pyridyl group, pyrimidyl group, pyridazyl group, pyrazyl group, imidazolyl group, triazolyl group, methylpyridyl group (2-methylpyridyl group, 3-methylpyridyl group, 4-methylpyridyl group and the like), dimethylpyridyl group (2,6-dimethylpyridyl group and the like), methylethylpyridyl group (2-methyl-6-ethylpyridyl group and the like), methylimidazolyl group (1-methylimidazolyl group and the like), dimethylimidazolyl group (1,2-dimethylimidazolyl group and the like), ethylimidazolyl group (1-ethylimidazolyl group and the like), propylimidazolyl group (1-n-propylimidazolyl group and the like), brilimidazolyl group (1-n-butylimidazolyl group and the like), pentylimidazolyl group (1-n-pentylimidazolyl group and the like), hexylimidazolyl group (1-n-hexylimidazolyl and the like), and the like.

Among <2> the substituted or unsubstituted aromatic groups, the nitrogen-free aryl group in <252> the substituted or unsubstituted nitrogen-free aryl group includes a phenyl group and naphthyl group (1-naphthyl group, 2-naphthyl group, and the like) with the carbon number of 6 to 16, and the like. The substituent in the substituted aryl group includes a straight chain or branched alkyl group, nitro group, halogen group (fluoro group, chloro group, bromo group, and the like) with the carbon number of 1 to 8, and the like. A specific example of <252> the substituted or unsubstituted aryl group includes a phenyl group, naphthyl group, tolyl group (o-tolyl group, m-tolyl group, p-tolyl group and the like), ethylphenyl group (4-ethylphenyl group, 3-ethylfail group, 2-ethylphenyl group, and the like), propylphenyl group (4-n-propylphenyl group, and the like), isopropylphenyl group (4-isopropylphenyl group, 2-isopropylphenyl group, and the like), butylphenyl group(4-n-butylphenyl group, 4-isobutylphenyl group, 4-t-butylphenyl group, 3-t-butylphenyl group, 2-t-butylphenyl group, and the like), pentylphenyl group (4-n-pentylphenyl group, 4-isopentylphenyl group, 2-neopentylphenyl group, 4-t-pentylphenyl group, and the like), hexylphenyl group (4-n-hexylphenyl group and the like), 4-(2-ethylbutyl)phenyl group, 4-n-heptylphenyl group, 4-n-octylphenyl group, 4-(2-ethylhexyl)phenyl group, 4-t-octylphenyl group, 4-ethyl-1-naphthyl group, 6-n-butyl-2-naphthyl group, dimethylphenyl group (2,4-dimethylphenyl group, 2,5-dimethylphenyl group, 3,4-dimethylphenyl group, 3,5-dimethylphenyl group, 2,6-dimethylphenyl group, and the like), trimethylphenyl group (2,3,5-trimethylphenyl group, 2,3,6-trimethylphenyl group, 3,4,5-trimethylphenyl group and the like), diethylphenyl group (2,4-diethylphenyl group, 2,6-diethylphenyl group, and the like), 2,5-diisopropylphenyl group, 2,6-diisobutylphenyl group, di-t-butylphenyl group (2,4-di-t-butylphenyl group, 2,5-di-t-butylphenyl group, and the like), 4,6-di-t-butyl-2-methylphenyl group, 5-t-butyl-2-methylphenyl group, 4-t-butyl-2,6-dimethylphenyl group, fluorophenyl group (4-fluorophenyl group, 3-fluorophenyl group, 2-fluorophenyl group, and the like), chlorophenyl group (4-chlorophenyl group, 3-chlorophenyl group, 2-chlorophenyl group, and the like), bromophenyl group (4-bromophenyl group, 2-bromophenyl group, and the like), chloronaphthyl group (4-chloro-1-naphthyl group, 4-chloro-2-naphthyl group, and the like), 6-bromo-2-naphthyl group, dichlorophenyl group (2,3-dichlorophenyl group, 2,4-dichlorophenyl group, 2,5-dichlorophenyl group, 3,4-dichlorophenyl group, 3,5-dichlorophenyl group, and the like), 2,5-dibromophenyl group, 2,4,6-trichlorophenyl group, dichloronaphthyl group (2,4-dichloro-1-naphthyl group, 1,6-dichloro-2-naphthyl group, and the like), chloromethylphenyl group (2-chloro-4-methylphenyl group, 2-chloro-5-methylphenyl group, 2-chloro-6-methylphenyl group, 3-chloro-4-methylphenyl group, 2-methyl-3-chlorophenyl group, 2-methyl-4-chlorophenyl group, 3-methyl-4-chlorophenyl group, and the like), trifluoromethylphenyl group (4-trifluoromethylphenyl group, and the like), nitrophenyl group (4-nitrophenyl group and the like), and the like.

In addition, a group comprising a combination of two or more types selected from the groups of the above-mentioned <1> to <7> includes a group comprising a combination of a monovalent group among the groups of <1> to <7>, which is <1> the substituted or unsubstituted amino group, <251> the nitrogen-containing aryl group, <252> the nitrogen-free aryl group, <3> the amidino group, <4> the guanidino group, <5> the carboxyl group, <6> the sulfo group, or <7> the bornyl group and <26> the substituted or unsubstituted arylene group (<261> the nitrogen-containing arylene group, <262> nitrogen-free arylene group).

Among <26> the substituted or unsubstituted arylene groups, <261> the nitrogen-containing arylene group is

a group wherein one hydrogen bound to a carbon configuring an aromatic ring is removed from a group included as <251> the substituted or unsubstituted nitrogen-containing aryl group described above. Among <26> the substituted or unsubstituted arylene groups, <262> the nitrogen-free arylene groups is a group wherein a hydrogen bound to a carbon configuring aromatic ring is removed from a group included as <252> the substituted or unsubstituted nitrogen-free aryl group described above.

A specific group comprising a combination of two or more types selected from the groups <1> to <7> above includes, an amidinoaryl group which is a combination of <3> an amidino group and <26> a substituted or unsubstituted arylene group, a carboxyaryl group which is a combination of <5> a carboxyl group and <26> a substituted or unsubstituted arylene group, a sulfoaryl group which is a combination of <6> a sulfo group and <26> a substituted or unsubstituted arylene group, a boronylaryl group which is a combination of <7> a boronyl group and <26> a substituted or unsubstituted arylene group, and the like. Preferable groups thereamong are shown below.

[Chemical 11]

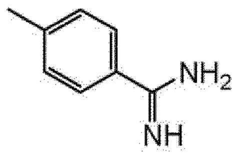

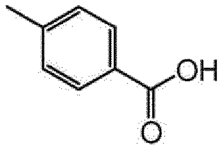

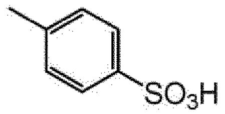

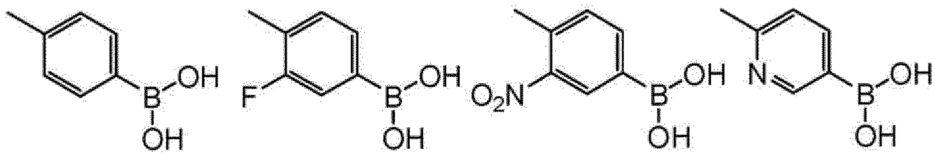

<1-3-6. A polymer configuring a molecularly imprinted polymer>

The constituent component of a polymer configuring molecularly imprinted polymers **20, 20a, 20b** and **20c** is not particularly limited. For example, said polymer comprises a constituent unit selected from the group consisting of a unit derived from a biocompatible monomer, a unit derived from N-substituted or unsubstituted (meth)acrylamide, and a unit derived from a functional monomer having the above-mentioned interaction group **25.** In addition, the present specification describes a polymer synthesized by imprinting polymerization using a mold as "molecularly imprinted polymer" for convenience, wherein the present disclosure also allows a matter that is not a molecule (e.g., a microparticle having a film structure) as a mold, and thus the "molecularly imprinted polymer" also encompasses a polymer synthesized by imprinting polymerization using a matter that is not a molecule as a mold.

(A unit derived from a biocompatible monomer)

A polymer configuring molecularly imprinted polymers **20, 20a, 20b** and **20c** preferably at least comprises a component derived from a biocompatible monomer. Biocompatibility refers to a property that does not induce adherence of a biological substance. Non-specific adsorption to the molecularly imprinted polymers **20, 20a, 20b** and **20c** surface can be favorably suppressed by comprising a component derived from a biocompatible monomer (e.g., exemplified as the biocompatible monomer **M28** discussed below). A biocompatible monomer preferably includes a hydrophilic monomer, more preferably includes a zwitterionic monomer.

The zwitterionic monomer comprises both an anion group derived from an acidic functional group (e.g., a phosphate group, a sulfate group, and a carboxyl group, and the like) and a cationic group derived from a basic functional group (e.g., a primary amino group, a secondary amino group, a tertiary amino group, and a quaternary ammonium group, and the like) in one molecule. For example, phosphobetaine, sulfobetaine, and carboxybetaine, and the like are included.

More specifically, phosphobetaine includes a molecule having a phosphorylcholine group on a side chain, preferably includes 2-methacryloyloxyethyl phosphorylcholine (MFC) and the like.

Sulfobetaine includes N,N-dimethyl-N-(3-sulfopropyl)-3'-methacryloylaminopropanaminium inner salt (SPB), N,N-dimethyl-N-(4-sulfobutyl)-3'-methacryloylaminopropanaminium inner salt (SBB), and the like.

Carboxybetaine includes N,N-dimethyl-N-(1-carboxymethyl)-2'-methacryloyloxyethaneaminium inner salt (CMB), N,N-dimethyl-N-(2-carboxyethyl)-2'-methacryloyloxyethaneaminium inner salt (CEB), and the like.

Among these zwitterionic monomers, preferably phosphobetaine is included, and more preferably 2-methacryloyloxyethyl phosphorylcholine (MPC) is included.

The ratio of the unit derive from a biocompatible monomer in the entire constituent unit of the molecularly imprinted polymers **20, 20a, 20b** and **20c** includes, for example, 1 mol% or greater and 100 mol% or less. The content of the unit derived from a biocompatible monomer is preferably at the above-mentioned lower limitation or greater in terms of suppressing non-specific adsorption in the molecularly imprinted polymers **20, 20a, 20b** and **20c** surface. In addition, a preferable range of the above-mentioned ratio of the unit derived from a biocompatible monomer may differ depending on the shape of the molecularly imprinted polymers **20, 20a, 20b** and **20c.** For example, when the molecularly imprinted polymers **20, 20a, 20b** and **20c** are film-like, the preferable range of the above-mentioned ratio of the unit derived from a biocompatible monomer includes 30 mol% or greater, more preferably 50 mol% or greater, further preferably 70 mol% or greater, even more preferably 80 mol% or greater, further even more preferably 90 mol% or greater, especially preferably 95 mol% or greater. In addition, when the molecularly imprinted polymers **20, 20a, 20b** and **20c** are particle-like, the preferable range of the above-mentioned ratio of the unit derived from a biocompatible monomer includes, 1 to 50 mol%, preferably 2 to 30 mol%, more preferably 3 to 10 mol%, further preferably 4 to 6 mol%, especially preferably 4.5 to 5.5 mol%.

(A unit derived from N-substituted or unsubstituted (meth)acrylamide)

A polymer configuring the molecularly imprinted polymers **20, 20a, 20b** and **20c** can comprise a unit derived from N-substituted or unsubstituted (meth)acrylamide. In particular, when the molecularly imprinted polymers **20, 20a, 20b** and **20c** are particle-like, it is preferable to comprise a unit derived from N-substituted or unsubstituted (meth)acrylamide in the constituent polymer.

N-substituted or unsubstituted (meth)acrylamide (e.g., exemplified as the polymerizable monomer **M29** discussed below) is shown in the following Formula **(4).**

[Chemical 12]

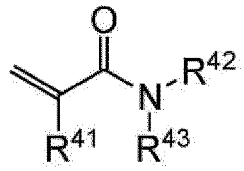

(4)

In Formula **(4),** $R^{41}$ represents hydrogen or a methyl group, preferably hydrogen, and $R^{42}$ and $R^{43}$ each independently represents hydrogen; a straight chain-like or branched chain-like alkyl group with a carbon number of 1 to 6; a straight chain-like or branched chain-like hydroxyalkyl group with a carbon number of 1 to 6; or a straight chain-like or branched chain-like aminoalkyl group with a carbon number of 1 to 6, wherein they may be the same or different from one another; and $R^{42}$ and $R^{43}$ may form a saturated 5 to 7 membered ring comprising a nitrogen atom carrying them as well as an oxygen atom.

A straight chain-like or branched chain-like alkyl group with a carbon number of 1 to 6 includes a methyl group, ethyl group, n-propyl group, isopropyl group, n-butyl group, sec-butyl group, tert-butyl group, n-pentyl group, n-hexyl group, and the like. A straight chain-like or branched chain-like hydroxyalkyl group with a carbon number of 1 to 6 includes hydroxymethyl group, 1-hydroxyethyl group, 2-hydroxyethyl group, 1-hydroxy-n-propyl group, 2-hydroxy-n-propyl group, 3-hydroxy-n-propyl group, 1-hydroxyisopropyl group, 2-hydroxyisopropyl group, 1-hydroxy-n-butyl group, 1-hydroxy-n-pentyl group, 1-hydroxy-n-hexyl group, and the like. A straight chain-like or branched chain-like aminoalkyl group with a carbon number of 1 to 6 includes aminomethyl group, 1-aminoethyl group, 2-aminoethyl group, 1-amino-n-propyl group, 2-amino-n-propyl group, 3-amino-n-propyl group, 1-aminoisopropyl group, 2-aminoisopropyl group, 1-amino-n-butyl group, 1-amino-n-pentyl group, 1-amino-n-hexyl group, and the like. A saturated 5 to 7 membered ring comprising a nitrogen atom and an oxygen atom includes a morpholine ring, and the like.

Among these N-substituted or unsubstituted (meth)acrylamide, N-substituted (meth)acrylamide is preferably included, more preferably N-mono-substituted (meth)acrylamide is included, further preferably N-alkyl(meth)acrylamide(the alkyl group is the above-mentioned straight chain-like or branched chain-like alkyl group with a carbon number of 1 to 6) is included, even more preferably N-isopropyl(meth)acrylamide is included, and especially preferably N-isopropyl acrylamide is included.

When a unit derived from N-substituted or unsubstituted (meth)acrylamide is comprised in a polymer configuring the molecularly imprinted polymers **20, 20a, 20b** and **20c,** the ratio of the unit derived from N-substituted or unsubstituted (meth)acrylamide in the entire constituent unit of the molecularly imprinted polymers **20, 20a, 20b** and **20c** includes, for example, 1 to 99 mol%, preferably 10 to 98 mol%, more preferably 30 to 96 mol%, further preferably 50 to 94 mol%,

even more preferably 70 to 92 mol%, further even more preferably 80 to 90 mol%, especially preferably 85 to 90 mol%.

(A unit derived form a functional monomer)

A polymer configuring the molecularly imprinted polymer **20c** comprises a unit derived from a functional monomer. The functional monomer is not particularly limited as long as the monomer has the interaction group **25** discussed in "1-3-5. Interaction group" above (e.g., exemplified as the monomer **M25** comprising an interaction group for a mold discussed below).

The monomer shown in Formula **(5)** below is included as an unlimited example of a functional monomer.

[Chemical 13]

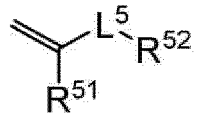

(5)

In Formula **(5),** $R^{51}$ represents hydrogen or methyl group, preferably hydrogen, $L^5$ represents direct binding or linking group, $R^{52}$ represents the interaction group **25** discussed in "1-3-5. Interaction group" above.

When the direct binding or linking group $L^5$ is a linking group, said linking group includes, for example, with the carbon number of 1 to 6, preferably 2 to 6, an alkylene group, amino group (-NH-), ether group (-O-), carbonyl group (-C(=O)-), ester group (-C(=O)-O- or -O-C(=O)-), amide group (-C(=O)-NH- or -NH-C(=O)-), sulfoxide group (-S(=O)-), sulphonyl group (-S(=O)$_2$-), and a group wherein two or more of the above are bound. Among these linking groups, an ester group (-C(=O)-O) is preferably included.

The specific unlimited example of the monomer represented by Formula **(5)** above includes the following monomer.

[Chemical 14]

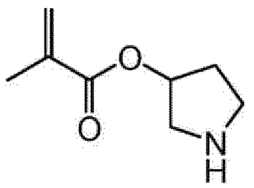

(5a)

In the molecularly imprinted polymer **20c,** a unit derived from a functional monomer at least exists on the recessed part **21c** surface. In the molecularly imprinted polymer **20c,** a unit derived from a functional monomer may exist not only on the recessed part **21c** surface, but also inside. The ratio of the unit derived from a functional monomer in the entire constituent unit of a polymer configuring the molecularly imprinted polymer **20c** is not particularly limited, and depends on the surface state of a corresponding mold (number of the sites interacting with the interaction **25**). As an example, a ratio of a unit derived from a functional monomer in the entire constituent unit of a polymer configuring the molecularly imprinted polymer **20c** includes, for example, 1 to 30 mol%, preferably 3 to 20 mol%, more preferably 5 to 10 mol%, further preferably 6 to 8 mol%.

(Other units derived from a monomer)

A unit derived from a monomer that provides a group for binding a specific binding molecule (the monomer **M22** comprising a group for binding a specific binding molecule and a polymerizable functional group discussed below) and/or a unit derived from a monomer that provides a group for binding a signal substance (the monomers **M23$_1$** and **M23$_2$** that provide a group for binding a signal substance discussed below) can also exist on the recessed part **21** surface of a polymer configuring the molecularly imprinted polymers **20, 20a** and **20b.** The case wherein a unit derived from a monomer that provides a group for binding a specific binding molecule exists includes the case wherein the molecularly imprinted polymers **20, 20a** and **20b** are particle-like. The case wherein a unit derived from a monomer that provides a group for binding a signal substance includes the case wherein the molecularly imprinted polymers **20, 20a** and **20b** are film-like.

<1-3-7. Base plate>

The material of the base plate **30** may be, for example, a material selected from the group consisting of metal, glass, and resin. The metal includes gold, silver, copper, aluminum, tungsten, molybdenum, and the like. The resin includes poly(meth)acrylate, polystyrene, ABS(acrylonitrilebutadiene-styrene copolymer), polycarbonate, polyester, polyethylene, polypropylene, nylon, polyurethane, silicone resin, fluorocarbon resin, methylpentene resin, phenol resin, melamine resin, epoxy resin, vinyl chloride resin, and the like.

The base plate **30** may be formed by combining a plurality of materials selected from the above-mentioned materials. For example, the base material **20** may be provided with a metal film on the surface of the glass or resin. In addition, it does not matter whether the shape of the base material **20** is plate-type or particle-like. A preferable example includes a gold base plate, glass base plate, gold nanoparticle, silicon dioxide particle (silica particle, glass bead, and the like), and the like.

<1-4. Other forms>

The base material for making a sensor for analyzing a detection subject of the present disclosure should only be configured so that a user would customize at least one of a specific binding molecule and a signal substance. Therefore, as another embodiment, a specific binding agent **42** for a target may already be bound to the groups **22'** and **22b'** for binding a specific binding molecule. In this case, a user can freely select and introduce a signal substance.

Furthermore, as another embodiment, a signal group **43** may already be bound to the groups **23'** and **23b'** for binding a signal substance. In this case, a user can freely target a detection subject, freely select a detection subject to be targeted, and can introduce a specific binding molecule for a target of a detection subject.

<2. A sensor for analyzing a detection subject>

The sensor for analysis of the present disclosure comprises: the above-mentioned base material for making a sensor for analyzing a detection subject; and a specific binding group for a target of said detection subject bound to said group for binding a specific binding molecule, wherein said group for binding a specific binding molecule is a group that is adapted to a small substance. Alternatively, the sensor for analysis of the present disclosure comprises: the above-mentioned base material for making a sensor for analyzing a detection subject; and a specific binding group for a target of said detection subject bound to said group for binding a specific binding molecule, wherein a molecular imprint recessed part has a shape or configuration adapted to a small substance. In addition, the sensor for analysis of the present disclosure also comprises a signal group bound to a group for binding a signal substance when the above-mentioned base material for making a sensor for analyzing a detection subject has a group for binding a signal substance.

Examples of the sensor for analysis of the present disclosure are shown as analysis sensors **10, $10_1$, 10a, $10a_1$, 10b** and **10c** of a detection subject in Figure **1B-1,** Figure **1B-2,** Figure **2B-1,** Figure **2B-2,** Figure **3B** and Figure **4B.** As depicted, regarding the analysis sensors **10, $10_1$, 10a, $10a_1$, 10b** and **10c** of a detection subject, at least the specific binding group **42** for a target is bound to the groups **22'** and **22'b** for binding a specific binding molecule to a target of the base materials **10'**, **10a', 10b'** and **10c'** for making an analysis sensor of a detection subject, and the signal group **43** is bound to the groups **23'** and **23b'** for binding a signal substance.

More specific examples of the sensor for analysis of the present disclosure are shown as analysis sensors **10-C, 10-P, 10-PC, 10a-C, $10a_1$-C, 10a-P, 10c-P** and **10b-PC** of a detection subject in Figure **5(B),** Figure **6(B),** Figure **7(B),** Figure **8(B1)** and **(B2),** Figure **9(B),** Figure **10(B),** and Figure **11(B).** As depicted, at least the specific binding group **42** for a target is bound to the groups **22'** and **22b'** for binding a specific binding molecule to a target of the analysis sensors **10-C, 10-P, 10-PC, 10a-C, 10a-P, $10a_1$-C, 10c-P** and **10b-PC** of a detection subject, and the signal group **43** is bound to the groups **23'** and **23b'** for binding a signal substance.

<2-1. Detection subject>

The detection subject of the sensor for analysis of the present disclosure comprises a target, which is not particularly limited in principle as long as the target has specific binding capacity with respect to the specific binding group **42.**

Regarding the relationship between a detection subject and a target: the detection subject may be the target itself (i.e., detection subject consists of the target; hereinafter, referred to as Embodiment **1**); or a part of the detection subject (e.g., the detection subject **70** discussed below) may be configured with the target (e.g., the target **72** discussed below) (hereinafter, referred to as Embodiment **2**).

The chemical species of a detection subject (Embodiment **1**) or a target (Embodiment **2**) of a detection subject is not particularly limited, and includes, for example, a low molecular organic compound and a high molecular compound

that are not derived from an organism, a low molecular organic compound and a high molecular compound that are derived from an organism. Among these chemical species, preferably a low molecular organic compound and high molecular compound that are derived from an organism are included, more preferably a low molecular organic compound and a high molecular compound derived from an animal are included, and specifically, sugar or sugar chain; lipid (e.g., phospholipid and the like); glycoprotein or glycopeptide; protein or peptide; nucleotide or polynucleotide, and the like are included.

The origin organism includes humans and animals other than a human, wherein the animals other than a human includes vertebrate animals, preferably includes mammals, which includes, for example, a mouse, rat, monkey, dog, cat, cow, horse, pig, hamster, rabbit, and goat, and the like.

The functional species of a detection subject (Embodiment **1**) or a target (Embodiment **2**) of a detection subject is not particularly limited, and includes plasma protein, disease marker, antigen, antibody, receptor, prion, microparticle having a film structure, and the like. In addition, when the microparticle having a film structure is set as a detection subject (Embodiment **2**), the target includes: an antigen, antibody, receptor, and/or disease marker expressed on the surface of the microparticle having a film structure; an antigen, antibody, receptor and/or disease marker, wherein the microparticle having a film structure is expressed inside the film structure; and an antigen, antibody, and/or disease marker discharged by the microparticle having a film structure; and the like.

A plasma protein includes albumin, γ-globulin, fibrinogen, and the like. A disease marker includes a kidney function marker, liver function marker, inflammation marker, tumor marker, and the like, and more specifically includes urinary albumin, aspartate aminotransferase (AST), alanine aminotransferase (ALT), transferrin, ceruloplasmin, lactate dehydrogenase (LDH), alkaline phosphatase (ALP), γ-glutamyl transpeptidase (γ-GTP), C-reactive protein (CRP), α-fetoprotein (AFP), sugar chain antigen 19-9 (CA19-9) carcinoembryonic antigen (CEA), prostate-specific antigen (PSA), exosome-specific antigen (CD63, CD9, CD81, CD37, CD53, CD82, CD13, CD11, CD86, ICAM-1, Rab5, Annexin V, LAMP1, and the like).

A microparticle that has a film structure includes an extracellular vesicle, intracellular vesicle, organelle, and cell. A film structure includes a lipid bilayer structure. An extracellular vesicle includes exosome, microvesicle, apoptotic body, and the like. An intracellular vesicle includes lysosome, endosome, and the like. An organelle includes mitochondria and the like. A cell includes a cancer cell such as circulating tumor cell (CTC), other disease-associated cells, and the like. Among these microparticles that have a film structure, preferably extracellular vesicle and cell are included, more preferably exosome and cancer-associated cell are included.

A target (exosome-specific antigen or exosome antigen) expressed on the surface of an exosome includes: proteins such as CD63, CD9, CD81, CD37, CD53, CD82, CD13, CD11, CD86, ICAM-1, Rab5, Annexin V, LAMP1, EpCAM, and HER2; lipid; sugar chain; and the like.

A target (cancer cell-specific antigen) expressed on the surface of a cancer cell includes: proteins such as Caveolin-1, EpCAM, FasL, TRAIL, Galectine3, CD151, Tetraspanin 8, EGFR, HER2, RPN2, CD44, and TGF-β; lipid; sugar chain; and the like.

As discussed below, when a signal substance **R43** is configured as one of the fluorescent dye pair that causes fluorescence resonance energy transfer (FRET), the target of a detection subject is coupled to the other of the fluorescent dye pair beforehand.

<2-2. A specific binding group for a target of a detection subject>

In the present disclosure, the specific binding group **42** is a small substance, preferably a specific binding protein group or peptide group with a molecular weight of 45,000 or less. Molecular weight refers to calculated molecular mass.

In one embodiment, a small substance group is typically a molecule with a molecular weight that is smaller than an antibody, which is a substance that is less than 150,000, preferably a substance that is 100,000 or less, more preferably 45,000 or less, further preferably 22,000 or less, even more preferably 16,000 or less, further even more preferably a group that is less than 10,000, and any substance is used, but preferably may be a protein, polypeptide, peptide.

In the present disclosure, by selecting a group with a small molecular weight as the specific binding group **42** provided within the molecular imprint recessed part, the binding capacity per target is significantly increased (i.e., the dissociation constant Kd significantly decreases) compared to the natural binding capacity to target of said specific binding group **42** (the binding capacity in the state of not being provided to the molecular imprint recessed part, e.g., a free state or the case of being immobilized in a plane-like manner). From the viewpoint of further even more increasing such effect, the molecular weight of the specific binding group **42** includes preferably 40,000 or less, more preferably 30,000 or less, further preferably 22,000 or less, even more preferably 16,000 or less, further even more preferably 10,000 or less, especially preferably 8,000 or less.

The specific binding group **42** is not particularly limited as long as the group has specific binding capacity to a

target of a detection subject and is a small substance group (provided that Fab', Fab, and scFv are excluded). The specific binding group **42** is a group introduced by a specific binding molecule (the specific binding molecule **R42** discussed below) that provides the specific binding group **42,** wherein a specific example of said specific binding molecule (the specific binding molecule **R42** discussed below) includes an antibody mimetic, phospholipid binding protein, and lectin, and the like. These specific binding molecules is commercially available or can be appropriately made or selected utilizing known information (e.g., library information and the like) of the subject technical field, and can be readily obtained by those skilled in the art.

A nanobody (VHH antibody) has a natural heavy chain antibody structure, and contains a single variable domain (VHH) and two constant domains (CH2 and CH3). A specific nanobody includes, but not limited to, an anti-EGFR nanobody, anti-HER2 nanobody, anti-MUC1 nanobody, anti-integrin nanobody, anti-TNF nanobody, and the like.

An antibody mimetic is any substance that can specifically bind to an antigen like an antibody but is not in structural association with an antibody, exemplified by an organic compound (not having an antibody binding domain). A specific antibody mimetic includes, but not limited to, an affibody (scaffold protein: a Z domain of a protein A, molecular weight: about 6,000, non-limited specific examples: anti-Her2 affibody, anti-EGFR affibody, anti-TNF-$\alpha$ affibody, anti-HSA affibody, anti-transferrin affibody, anti-fibrinogen affibody, anti-IgM affibody, and the like); aphylline (scaffold protein: $\gamma$B-crystallin or ubiquitin, molecular weight: about 20,000 or about 10,000); affimer (scaffold protein: cystatin, molecular weight: about 12,000 to 14,000); afitin (scaffold protein: Sac7d, molecular weight: about 7,000); alpha body (scaffold protein: triple helix coiled coil, molecular weight: about 10,000); anticalin (scaffold protein: lipocalin, molecular weight: about 20,000, non-limited specific examples: anti-CTLA-4 anticalin, anti-VEGF anticalin, and the like); abimer (scaffold protein: film receptor domain, molecular weight: about 9,000 to 18,000); DARPins (scaffold protein: ankyrin repeat motif, molecular weight: about 10,000 to 19,000); fynomer (scaffold protein: SH3 domain of Fyn, molecular weight: about 7,000); kunitz domain peptide (scaffold protein: kunitz domain of a protease inhibitor, molecular weight: about 6,000); monobody (also referred to as adnectin; scaffold protein: 10th III-type domain of fibronectin, molecular weight: about 10,000, non-limited specific examples: anti-EGFR1 monobody, monobody of anti-HER2, and the like); nanoCLAP (scaffold protein: carbohydrate binding module 32-2 (Clostridium perfringens NagH), molecular weight: about 16,000), nanobody (e.g., a recombinant protein of a VHH antibody that a camel and alpaca have), and the like.

A phospholipid binding protein is a protein that exhibits binding activity to phospholipid. A specific example of a phospholipid binding protein includes, but not limited to, evectin 2, annexin A2, annexin V, Tim4, and the like.

Lectin is a protein that exhibits binding activity to a sugar chain. A specific example of lectin includes, but not limited to, type-C lectin, kinase-like lectin, F box lectin, type-F lectin ficolin/mannose binding lectin, galectin, X-lectin, type-L lectin, type-M lectin, type-P lectin, type-R lectin, tachylectin, sialic acid binding lectin, and the like.

A substance having specific binding capacity for a target of a detection subject other than a protein and a peptide includes, for example, the following:

Sugar: The sugar includes $\alpha$-cyclodextrin (6 D-glucose coupled), $\beta$-cyclodextrin (7 D-glucose coupled), $\gamma$-cyclodextrin (8 D-glucose coupled), which are cyclic oligosaccharide wherein D-glucose is coupled by $\alpha$-1,4 glycoside binding. The inside of these cyclic structures are a hydrophobic cavity. Since a molecule that matches the cavity size is recognized and enclosed by hydrophobic interaction, the molecule can be used as a molecule having specific binding capacity for a target of a detection subject;

Glycosaminoglycan with a repetition structure of disaccharide provided with a sulfate group: for example, hyaluronic acid recognizes and binds CD44 which is a film protein, thereby enabling use as a molecule having specific binding capacity to CD44. In another example, heparin exists on a cell surface, and interacts with various extracellular matrix proteins having a heparin binding domain, thereby enabling use as a molecule having specific binding capacity to a protein with a heparin binding domain. In addition, many proteoglycans wherein a sugar chain is bound to a peptide are found, thereby enabling use as a molecule having specific binding capacity to a target of a detection subject.

Lipid: lipid, e.g., phospholipid with phosphate ester, can bind various functional groups with phosphate ester, wherein phospholipid can interact with various biomolecules, thereby enabling use as a molecule having specific binding capacity to a target of a detection subject.;

Nucleic acid: regarding a nucleic acid, nucleic acid aptamer is known as a nucleic acid molecule that specifically binds to a specific molecule. It can be freely manufacturing with the in vitro selection method, and there have been reports of those that specifically bind to various objects such as an organic small molecule, protein, nucleic acid cell, cell tissue, and microorganism, thereby enabling use as a molecule having specific binding capacity to a target of a detection subject.

Regarding the sensor for analysis of the present disclosure, even if the natural binding capacity to a target of a specific binding molecule (the specific binding molecule **R42** discussed below) that provides the specific binding group **42** is inferior (the dissociation constant Kd is greater than an antibody) to the natural binding capacity of an antibody, the binding capacity per target would be significantly increased (i.e., the dissociation constant Kd significantly decreases)

by providing the specific binding group **42** within the molecular imprint recessed parts **21** and **21c,** wherein the significantly improved binding capacity may even surpass the binding capacity of an antibody per target of when introducing said antibody within the molecular imprint recessed parts **21** and **21c.** In other words, according to the sensor for analysis of the present disclosure, it is possible to express sensitivity that exceeds a sensor for analysis wherein an antibody is introduced within the molecular imprint recessed part **21** and **21c** even when introducing the specific binding group **42** of which binding capacity is inferior to an antibody within the molecular imprint recessed parts **21** and **21c.** In view of the above, an example of a specific binding molecule (the specific binding molecule **R42** discussed below) that provides the specific binding group **42** in the sensor for analysis of the present disclosure includes a molecule wherein the natural binding capacity of itself is inferior (the dissociation constant Kd is greater than an antibody) to the natural binding capacity of an antibody. However, since the sensor for analysis of the present disclosure significantly increases the binding capacity of the specific binding group **42** per target, it is possible to use a molecule wherein the natural binding capacity is increased by providing a modification that increases the binding capacity such as mutation as a specific binding molecule (the specific binding molecule **R42** discussed below) that provides the specific binding group **42.**

The specific binding group **42** may be a group (specific binding group having a signal group) **44** with a signal group **43** further bound like the analysis sensor **10,** of a detection subject shown in Figure **1B-2.** A specific example of such specific binding group **44** that has a signal group includes: a modification protein or peptide wherein the specific modification group **42** has the signal group **43** as a modification group; a fusion protein or peptide wherein the specific binding group **42** and the signal group **43** which is a protein or a peptide are fused; and the like. As such, the natural binding capacity of the specific binding group **42** in the specific binding group **44** having a signal group is inferior to the natural binding capacity of the specific binding group **42** that does not have the signal group **43** (the specific binding group **42** in the sensors **10, 10a, 10b,** and **10c** for analysis of Figure **1B-1,** Figure **2B-1,** Figure **3B,** and Figure **4B**) due to the effect by the presence of the signal group **43.** However, since the sensor for analysis of the present disclosure is excellent in sensitivity improvement effect, it is possible to effectively improve binding capacity and achieve excellent sensitivity improvement effect even by using a specific binding group with low natural binding capacity like the specific binding group **44** that has a signal group. In the present disclosure, while it is possible to carry out analysis with excellent sensitivity (low dissociation constant) even when using the group **44** having the specific binding group **42** and the signal group **43** like the analysis sensor **10,** of a detection subject shown in Figure **1B-2,** from the viewpoint of improving reproducibility and/or lowering the detection limit value, it is preferable that the signal group **43** and the specific binding group **42** are spaced apart in the surface direction of the recessed part of the molecular imprint as shown in Figure **2B,** or have the functional group **24b** (see Figure **3A**) therebetween as shown in Figure **3B,** rather than being bound to one another.

Furthermore, the specific binding group **42** may be a group (specific binding group having a different signal group) $\mathbf{42_1}$ wherein a different signal group **sg** is further bound like the analysis sensor $\mathbf{10a_1}$ of a detection subject shown in Figure **2B-2.** While the signal group **43** bound to the specific binding group **42** shown in Figure **1B-2** above is used for sensing of the analysis sensor **10,** of a detection subject, the different signal group **sg** bound to this specific binding group **42** shown in Figure **2B-2** are different in that they are not used for sensing of the analysis sensor $\mathbf{10a_1}$ of a detection subject. Since the molecular weight of the specific binding group **42** is small, the different signal group **sg** is merely provided from the viewpoint of making the handling of the specific binding molecule (the specific binding molecule **R42** discussed below) that provides the specific binding group **42** easy, wherein not the different signal group **sg** but the signal group **43** is used for the sensing of the analysis sensor $\mathbf{10a_1}$ of a detection subject shown in Figure **2B-2.**

A specific example of such specific binding group $\mathbf{42_1}$ having a different signal group includes: a modification protein or peptide wherein the specific binding group **42** has the different signal group **sg** as a modification group; a fusion protein or peptide wherein the specific binding group **42** and the different signal group **sg** which is a protein or a peptide are fused; and the like. A such, the natural binding capacity of the specific binding group **42** in the specific binding group $\mathbf{42_1}$ having a different signal group is inferior to the natural binding capacity of the specific binding group **42** that does not have the different signal **sg** (e.g., the specific binding group **42** in the sensors **10, 10a, 10b,** and **10c** for analysis of Figure **1B-1,** Figure **2B-1,** Figure **3B,** and Figure **4B**) due to the effect of the existence of the different signal group **sg.** However, since the sensor for analysis of the present disclosure is excellent in sensitivity improvement effect, it is possible to effectively improve the binding capacity and achieve excellent sensitivity improvement effect even when using a specific binding group with low natural binding capacity like the specific binding group $\mathbf{42_1}$ having a different signal group.

<2-3. Signal group>

The signal group **43** functions as a group that reads binding information between a target of a detection subject and the specific binding group **42** for said target. The signal group **43** is not particularly limited as long as the detected signal intensity changes and the spectrum changes (e.g., the peak shifts) due to the binding of a target in the molecular imprint recessed parts **21** and **21c.** For example, a signal substance that provides the signal group **43** (the signal

substance **R43** discussed below) includes a fluorescent substance, radioactive element-containing substance, magnetic substance, and the like. From the viewpoint of easiness of detection and the like, it is preferable that the signal substance is a fluorescent substance. A fluorescent substance includes: fluorescent dye such as fluorescein dye, cyanine dye such as indocyanine dye (DY647, Cy5, DY490, and the like), rhodamine dye (CR110 and TAMRA), coumarin dye, EvoBlue dye, oxazine dye (ATTO), carbopyronin dye, and Alexa dye; fluorescent protein such as GFP; nanoparticle such as gold colloid and quantum dot; and the like. A radioactive element-containing substance includes sugar, amino acid, and nucleic acid labeled with a radioactive isotope such as $^{18}F$, MRI probe labeled with $^{19}F$, and the like. A magnetic substance includes a matter having a magnetic body such as ferrichrome, ferrite nanoparticle, nanomagnetic particle, and the like. Among the above-mentioned signal substances, rhodamine dye is preferable from the viewpoint of further improving detection limitation and/or sensitivity.

In addition, the signal group **43** can be configured as one of the fluorescent dye pair that causes fluorescence resonance energy transfer (FRET). The fluorescent dye pair that causes FRET is not particularly limited, and is also not limited in terms of which of the donor dye and accepter dye is selected as the signal group **43.** Preferably, donor dye can be selected as the signal group **43.** A specific example of the combination of donor dye/accepter dye configuring the pair of fluorescent dyes causing FRET includes fluorescein isothiocyanate (FITC)/tetramethylrhodamine isothiocyanate (TRITC), Alexa Fluor647/Cy5.5, HiLyte Fluor647/Cy5.5, R-phycoerythrin (R-PE)/allophycocyanin(APC), and the like.

<2-4. Usage>

The sensor for analysis of the present disclosure is used for sensing of a detection subject. While a more specific usage is determined in accordance with the type of the specific binding group, the sensor for analysis of the present disclosure can be used, for example, for the purpose of diagnosis, or the purpose of therapy monitoring or the like based on kidney function, liver function, the presence/absence or degree of inflammation; presence/absence or degree of tumor, or the like.

In addition, the sensor for analysis of the present disclosure may be formulated to be liquid-like or solid-like (e.g., powder-like) as a sensing reagent in the case of the form wherein a molecularly imprinted polymer is a particle and is not immobilized on a base plate. The sensing reagent comprises another pharmacologically accepted component. A solid body and/or liquid that does not affect the stability and recognition capacity of a sensing reagent is appropriately selected as the another component, which includes, for example, water, salt such as NaCl, buffering agent, stabilizing agent, antioxidizing agent, antiseptic agent, pH regulating agent, surface active agent, diluting agent, binding agent, lubricating agent, and the like.

A reagent for sensing can be obtained by formulation based on a normal formulation method using the sensor for analysis of the present disclosure and the above-mentioned another component.

Other than those mentioned above, an example of the usage of the sensing reagent includes in vitro imaging and *in vivo* imaging utilizing the feature of a particle not being immobilized on a base plate.

An example of the in vitro imaging includes a method of making a sensing reagent corresponding to a detection subject in a cell or on the surface and adding the sensing reagent to said cell to carry out imaging of the kinetics of the biomolecule in the cell or on the surface with a signal detection means such as a fluorescence microscope.

An example of the *in vivo* imaging includes a method of making a sensing reagent corresponding to the detection subject existing in a body, administering the sensing reagent to an experimentation animal, a circulatory organ of an animal such as a human, e.g., blood, and carrying out imaging of the biokinetics of a detection subject with a signal detection means. The sensing reagent used for the *in vivo* imaging preferably configures the sensor **10c-P** for analysis shown in Figure **10(B)** as an effective component when the detection subject is something other than a plasma protein (especially albumin). In this case, the sensor **10c-P** for analysis is a sensor wherein the molecular imprint recessed part **21c** molds plasma protein (especially albumin) as a mold and the interaction group **25** is designed as a group that specifically interacts with the surface of said plasma protein (especially albumin). Such sensing reagent used for the *in vivo* imaging can be injected before surgery and be used as a navigation drug that carries out *in vivo* imaging of a tumor during the surgery when a detection subject is configured as a tumor marker.

<3. Manufacturing method of a base material for making a sensor for analyzing a detection subject>

The manufacturing method of the base material for making a sensor for analyzing a detection subject of the present disclosure can use a method of synthesizing a molecularly imprinted polymer and introducing a predetermined functional group within a molecular imprint recessed part without a particular limitation. Several examples of the manufacturing method of the base material for making a sensor for analyzing a detection subject of the present disclosure include the following.

<3-1. Manufacturing method of a base material for making a sensor for analyzing a detection subject that is base plate-type>

For example, the manufacturing method of the base material **10-C'** for making a sensor for analyzing a detection subject that is base plate-type which is shown in Figure **5(A)** comprises the following step **(1-1)** to step **(1-4):**

*the monolayer formation step **(1-1)** of forming a monolayer having a group for binding a specific binding moelcule for a target of a detection subject and a polymerization initiating group on a surface at the base plate;
*the step **(1-2)** of introducing a mold to said group for binding a specific binding molecule via a first reversible linking group;
*the step **(1-3)** of forming a polymer film on said base plate surface by adding a polymerizable monomer, using said polymerizable monomer as a substrate, using said polymerization initiating group as a polymerizable initiating agent, and synthesizing a molecularly imprinted polymer for a part of said surface of said mold; and
*the step **(1-4)** of cleaving said first reversible linking group for conversion to said group for binding a specific binding molecule as well as removal of said mold.

Provided that said group for binding a specific binding molecule is a group adapted to a small substance.
The following limitations are further added to the manufacturing method of the base material **10a-C'** for making a sensor for analyzing a detection subject that is base plate-type which is shown in Figure **8(a)**, further comprising the group **23'** for binding a signal substance in the base material **10-C'** for making a sensor for analyzing a detection subject that is base plate-type which is shown in Figure **5(A)**.

*Comprising the modification step of modifying a surface of said mold with a polymerizable functional group via a second reversible linking group before said step **(1-3);**
*using said polymerizable monomer and said polymerizable functional group as a substrate, using said polymerization initiating group as a polymerizable initiating agent, and synthesizing a molecularly imprinted polymer for a part of said surface of said mold in said step **(1-3);**
*cleaving said first reversible linking group and said second reversible linking group for conversion to said group for binding a specific binding molecule and a group for binding a signal substance, respectively, as well as removal of said mold in said step **(1-4).**

Figure **12-1** to Figure **12-5** schematically show the manufacturing method of the base material **10a-C'** for making a sensor for analyzing a detection subject that is base plate-type which is shown in Figure **8(A).** Specifically, Figure **12-1** (Figure **12-1a** and Figure **12-2b**), Figure **12-2,** Figure **12-3,** Figure **12-4** and Figure **12-5** schematically show the step of preparing a mold, step **(1-1),** step **(1-2),** step **(1-3)** and step **(1-4),** respectively. In other words, the following steps are comprised in a method of manufacturing the base material **10a-C'** for making a sensor for analyzing a detection subject in the depicted embodiment.

(I)

*The monolayer formation step **(1-1)** of forming a monolayer SAM having the group **22'** for binding a specific binding molecule to a target of a detection subject and a polymerization initiating group **INI** on a surface at the base plate **30**;
*the step **(1-2)** of introducing a mold **60** to the group **22'** for binding a specific binding molecule via the first reversible linking group **22;**
*the step **(1-3)** of forming a polymer film on the base plate **30** surface by adding a polymerizable monomer **M28,** using the polymerizable monomer **M28** as a substrate, using the polymerization initiating group **INI** as a polymerizable initiating agent, and synthesizing the molecularly imprinted polymer **20a** for a part of a surface of the mold **60;**
*the step **(1-4)** of cleaving the first reversible linking group **22** for conversion to the group **22'** for binding a specific binding molecule as well as removal of the mold **60;** provided,

(II)

*comprising the modification step of modifying a surface of the mold **60** with the polymerizable functional group **27** via the second reversible linking group **23;**
*using the polymerizable monomer **M28** and polymerizable functional group **27** as a substrate, using the polymerization initiating group **INI** as a polymerizable initiating agent, and synthesizing a molecularly imprinted

polymer **20a** for a part of said surface of the mold **60** in said step **(1-3);**
*cleaving the first reversible linking group **22** and said second reversible linking group **23** for conversion to group **22'** for binding a specific binding molecule and the group **23'** for binding a signal substance, respectively, as well as removal of the mold **60** in said step **(1-4).**

Each step explained in detail below while referring to the drawings. The manufacturing method of the base material **10-C'** for making a sensor for analyzing a detection subject that is base plate-type which is shown in Figure **5(A)** would be formed when the above-mentioned limitation (II) is removed from the manufacturing method of the base material **10a-C'** for making a sensor for analyzing a detection subject that is base plate-type discussed in detail below.

<3-1-1. The step of preparing a mold>

The mold used in the manufacturing method of the base material **10a-C'** for making a sensor for analyzing a detection subject that is base plate-type which is shown in Figure **12-2** to Figure **12-5** below is prepared as a mold wherein appropriate surface modification has been made as needed.

(Mold)

The mold **60** may be the same substance as a detection subject or a target of the detection subject, or may be a substance that is different from a detection subject or a target of the detection subject. In addition, the mold **60** in the case of being a substance different from a detection subject or a target of the detection subject may be a biomolecule, or may be a particle core (also referred to as artificial particle).

A biomolecule preferably includes a plasma protein (albumin, γ-globulin, fibrinogen, and the like). In addition, the molecular weight of the biomolecule includes, for example, 1.8 to 15-fold, preferably 3 to 14-fold, more preferably 4 to 13.5-fold, 7 to 13-fold, 8 to 12.5-fold, or 10 to 12-fold of the molecular weight of the specific binding group **42.** A more preferable biomolecule includes albumin.

A particle core is not particularly limited to the extent of being able to be used as a mold in a molecular imprint, including artificially manufactured inorganic particle and organic particle. An inorganic particle includes metal, oxide of metal, nitride, fluoride, sulfide, boride and a composite compound thereof, as well as hydroxyapatite, and the like, preferably silicon dioxide (silica). In addition, an organic particle includes latex cured product, dextran, chitosan, polylactic acid, poly(meth)acrylate, polystyrene, polyethyleneimine, and the like.

A particle core is preferably used as the mold **60** in the embodiment shown in Figure **12-2** to Figure **12-5.** Since a particle core is an industrial product that is particle-size controlled, it is easy to control and homogenize the size of the recess part formed into the base material for making a sensor for analysis, which is preferable in terms of being able to make a sensor for analysis with even more excellent analysis property from the obtained base material for making a sensor for analysis. In addition, it is possible to use a particle core of which surface has been modified so as to have a desired binding property at the surface.

Since the size of the molecular imprint recessed part **21** depends on the size of the mold **60,** the size of the mold **60** can be appropriately determined in accordance with the size of a detection subject or a target of the detection subject. It is enough to use the mold **60** having about the same or greater size as said detection subject or a target of the detection subject in order to form a recessed part **21** for receiving a detection subject or a target of the detection subject of interest. For example, the average particle size of the mold **60** includes, for example, 1 nm to 10 um, preferably 50 to 1 μm, more preferably 100 to 500 nm, further preferably 150 to 250 nm, even more preferably 180 to 220 nm. Alternatively, when the average particle size of the mold 60 is R (nm) and the molecular weight of the specific binding group **42** is M, the average particle size of the mold **60** includes a particle size wherein the parameter R/M satisfies, for example, 0.005 to 0.05, preferably 0.01 to 0.04, more preferably 0.012 to 0.035, 0.013 to 0.035, 0.02 to 0.035, or 0.03 to 0.034. Average particle size refers to the Z average particle size measured by the dynamic light scattering method.

(Surface modification of a mold)

The mold **60** can be prepared in the form of which surface is modified by the binding group **22"** as shown as the mold **$60_1$** and the mold **$60_2$** in Figure **12-1a** and Figure **12-1b**.

A method of preparing such a mold includes an embodiment wherein a surface of the mold **$60_1$** is modified with the binding group **23"** in addition to the binding group **22"** as shown in Figure **12-1a,** a method of modifying a surface of the mold **$60_2$** with a polymerizable functional group via a reversible linking group **23** in addition to the binding group **22"** as shown in Figure **12-1b,** and the like.

For example, in the example of Figure **12-1a,** the mold **$60_1$** having the binding group **BG22"** and the binding group **BG23"** on the surface is used to appropriately react a molecule comprising the binding group **BG22'** corresponding

to the binding group **BG22"** and the binding group **22"** and a molecule comprising the binding group **BG23'** corresponding to the binding group **BG23"** and the binding group **23"**, whereby obtaining the mold **$60_1$** modified with the binding group **23"** in addition to the binding group **22"** on the surface.

In Figure **12-1a,** the binding group **BG22"** is not particularly limited, but includes, for example, the groups shown in column **2-1** of Table **2** above, preferably includes a carboxyl group. The binding group **BG22'** is not particularly limited, which includes, for example, the groups shown in column **2-2** of Table **2** above, preferably includes an amino group as the binding group **BG22'.** The binding group **22"** is not particularly limited (provided the group is different from the binding group **BG22'),** which includes the groups shown in column **1-2** of Table **1** above, preferably includes a polyhistidine tag. In addition, the binding group BG **23"** is not particularly limited, but includes, for example, the groups shown in column **2-1** of Table **2** above, preferably includes a carboxyl group. The binding group **BG23'** is not particularly limited, which includes the groups shown in column **2-2** of Table **2** above, preferably includes an amino group as the binding group **BG23'.** The binding group **23"** is not particularly limited (provided the group is different from the binding group **22'**), which includes the groups shown in column **1-2** of Table **1** above, preferably includes thiol group.

In addition, in the example of Figure **12-1b,** the mold **$60_2$** having the binding group **BG22"** and the binding group **BG23"** on the surface is used to appropriately react a molecule comprising the binding group **BG22'** corresponding to the binding group **BG22"** and the binding group **22"** and the polymerizable monomer **$M23_2$** comprising the binding group **BG23'** corresponding to the binding group **BG23",** the second reversible linking group **23** and the polymerizable functional group **27,** and obtain a mold **$60_2$** having the polymerizable functional group **27** and a second reversible linking group **23** as well as the binding group **22"** that can form the first reversible linking group **22.** In the example of Figure **12-1b,** the modification step of modifying the surface of the mold **60** with the polymerizable functional group **27** via the second reversible linking group **23** is comprised in the step of preparing a mold.

In Figure **12-1b,** the binding group **BG22"** is not particularly limited, but includes, for example, the groups shown in column **2-1** of Table **2** above, preferably includes a carboxyl group. The binding group **BG22'** is not particularly limited, which includes, for example, the groups shown in column **2-2** of Table **2** above, preferably includes an amino group as the binding group **BG22'.** In addition, the binding group **22"** is not particularly limited (provided the group is different from the binding group **BG22'**), which includes the groups shown in column **1-2** of Table **1** above, preferably includes a polyhistidine tag. The binding group **BG23"** is not particularly limited, which includes, for example, the groups shown in column **2-1** of Table **1** above, preferably includes an amino group. The binding group **BG23'** in the polymerizable monomer **$M23_2$** is not particularly limited, which includes, for example, the groups shown in column **2-2** of Table **1** above, preferably includes a carboxylic acid-active ester group. The second reversible linking group **23** is not particularly limited, which includes, for example, the groups shown in column **1-3** of Table **1** above, preferably includes a disulfide bond.

In Figure **12-1b,** the polymerizable functional group **27** only needs to contain a vinyl group, which preferably includes a (meth)acryloyl group.

As shown in Figure **12-1b,** the second reversible linking group **23** can be delivered to the surface of the mold **60** by using the mold **$60_2$** having the binding group **BG23"** on the surface beforehand.

Accordingly, it is possible to obtain the molds **$60_1$** and **$60_2$** (hereinafter, may also be collectively referred to as the "mold **60**") wherein the surface is modified with the binding group **22"** (and the binding group **23**" or the polymerizable functional group **27** via the second reversible linking group as needed).

Furthermore, depending on the substance employed as the mold **60,** there is a substance that naturally has the binding group **22**" (e.g., there is a case of selecting a sugar protein as a mold, the case of being able to use a sugar group that a sugar protein naturally has as the binding group **22**", and the like). In such a case, the mold **60** does not require the above-mentioned step.

<3-1-2. Step **(1-1)**>

In step **(1-1),** as shown in Figure **12-2,** a monolayer **SAM** having the group **22'** for binding a specific binding molecule to a target of a detection subject and the polymerization initiating group **INI** on the surface is formed at the base plate **30.**

In the depicted example, specifically, a monolayer **SAM** having the polymerization initiating group **INI** and the binding group **BG1'** on the surface is first formed on the base material **30** surface. This monolayer can be formed as a mixed self-assembled monolayer (mixed SAMs) by mixed self-assembling using a molecule having the polymerization initiating group **INI** at the terminal and a molecule having the binding group **BG1'** that is different from the polymerization initiating group **INI** at the terminal.

The polymerization initiating group **INI** is not particularly limited as long as it has a structure that may function as a polymerization initiating agent, which can be appropriately determined by those skilled in the art in accordance with the polymerization reaction used in the polymerization step discussed below. For example, the polymerization initiating group **INI** includes a group having a structure that generates radical upon polymerization reaction, specifically, a carbon-halogen binding group derived from organic halogen (-CX group; X shows halogen atom), an azo group derived from

an azo compound (-R-N = N-R'- group), a group for RAFT polymerization derived from a RAFT agent (e.g., a thiocarbonylthio group, and the like), and a group derived from TEMPO. The depicted embodiment exemplifies the case in which the polymerization initiating group **INI** is a -CBr group.

A group that can extend the group **22'** for binding a specific binding molecule to a target of a detection subject is appropriately selected as the binding group **BG1'.** For example, the groups shown in column **2-1** of Table **2** above are included, preferably an amino group is included. The group **22'** for binding a specific binding molecule is further introduced to the surface of the monolayer **SAM** by binding a substance for extending the group **22'** for binding a specific binding molecule to the binding group **BG1'.** For example, a substance for extending the group **22'** for binding a specific binding molecule includes a molecule having a group that can form the group **22'** for binding a specific binding molecule and the binding group **BG1"** that corresponds to the binding group **BG1',** or a molecule having the group **22'** for binding a specific binding molecule and the binding group **BG1"** that corresponds to the binding group **BG1'.** The binding group **BG1"** that corresponds to the binding group **BG1'** includes, for example, the groups shown in column **2-2** of Table **2** above. In the depicted embodiment, as an example of a molecule having a group that can form the group **22'** for binding a specific binding molecule and the binding group **BG1"** that corresponds to the binding group **BG1',** nitrilotriacetic acid (NTA) which is an aminopolycarboxylic acid-based chelating agent is used to bind NTA to introduce a group derived from NTA and nickel ion is further coordinated to a group derived from NTA to form a group derived from nickel-nitrilotriacetic acid which is an unlimited example of the group **22'** for binding a specific binding molecule.

Furthermore, the example of Figure **12-2** shows an embodiment of gradually forming a monolayer **SAM** having the group **22'** for binding a specific binding molecule and the polymerization initiating group **INI** on the surface by first making a mixed self-assembled monolayer having the binding group **BG1'** on the surface and then extending the binding group **BG22'** to the binding group **BG1',** but the present disclosure is not limited to this embodiment. For example, it is possible to gradually form a monolayer **SAM** having the group **22'** for binding a specific binding molecule and the polymerization initiating group **INI** on the surface by forming a mixed self-assembled monolayer (mixed SAMs) by mixed self-assembling using a molecule having the polymerizable initiating group **INI** at a terminal and a molecule having the binding group **BG22'** at a terminal on the base material **30** surface.

Accordingly, a monolayer **SAM** having the group **22'** for binding a specific binding molecule and the polymerization initiating group **INI** on the surface is formed at the base plate **30.**

<3-1-3. Step **(1-2)**>

In step **(1-2),** as shown in Figure **12-3a** and Figure **12-3b,** a mold **60** is introduced to the group **22'** for binding a specific binding molecule on the base plate **30** via the first reversible linking group **22.**

The first reversible linking group **22** is formed by binding between the group **22'** for binding a specific binding molecule on the base plate **30** and the binding group **22'** on the mold **60,** which includes, for example, the groups shown in column **1-3** of Table **1** above, preferably includes a coordinate bond.

The example shown in Figure **12-3a** shows the case of using the mold $\mathbf{60_1}$ obtained in Figure **12-1a,** and the example shown in Figure **12-3b** shows the case of using the mold $\mathbf{60_2}$ obtained in Figure **12-1b**.

In the example shown in Figure **12-3a,** the surface of the mold $\mathbf{60_1}$ immobilized on the base plate **30** can be modified by introducing the mold **60** in the state in which the polymerizable functional group **27** is still not introduced to the group **22'** for binding a specific binding molecule on the base plate **30** via the first reversible linking group **22,** and then using the polymerizable monomer $\mathbf{M23_1}$ having the group **23'** for binding a signal substance and the polymerizable functional group **27** in a state in which the mold **60** is immobilized on the base plate **30.** In the example of Figure **12-3a,** the modification step of modifying the surface of the mold **60** with the polymerizable functional group **27** via the second reversible linking group **23** is comprised after the step of introducing a mold.

The group **23'** for binding a signal substance in the polymerizable monomer $\mathbf{M23_1}$ is as discussed in "1-3-3. A group for binding a signal substance", which preferably includes a pyridyl disulfide group. The polymerizable function group **27** should only contain a vinyl group, which preferably includes a (meth)acryloyl group.

<3-1-4. Step **(1-3)**>

In step **(1-3),** as shown in Figure **12-4,** a polymer film is formed on the base plate **30** surface by adding the polymerizable monomer **M28,** using the polymerizable monomer **M28** and the polymerizable functional group **27** as a substrate, using the polymerization initiating group **INI** as a polymerizable initiating agent, and synthesizing the molecularly imprinted polymer **20a** for a part of the surface of the mold **60.**

In the depicted example, only one type of polymerizable monomer is shown, but a plurality of types may be combined as the polymerizable monomer. In addition, the polymerizable monomer is as discussed in the "1-3-6. A polymer configuring a molecularly imprinted polymer" above. The polymerizable monomer **M28** used in the depicted example is preferably a biocompatible monomer, wherein the biocompatible monomer is as discussed in "(A unit derived

from a biocompatible monomer)" of "1-3-6. A polymer configuring a molecularly imprinted polymer" above.

Surface-initiated atom transfer radical polymerization (SI-ATRP) progresses by constructing on the base material **30** surface a polymerization reaction system where the polymerizable functional group **27,** polymerizable monomer **M28,** polymerizable starting group **INI** and mold **60** coexist. Said polymerization reaction system further preferably comprises a transition metal complex formed of a transition metal or a transition metal compound and a ligand as a polymerization catalyst, and preferably a reducing agent is further used.

A transition metal or transition metal compound includes metal copper or copper compound, and the copper compound includes chloride, bromide, iodide, cyanide, oxide, hydroxide, acetate, sulfate, nitrified product, preferably bromide. A ligand is preferably multidentate amine, specifically including bidentate to sexadentate ligands. Among the above, a bidentate ligand is preferably included, more preferably 2,2-bipyridyl, 4,4'-di-(5-nonyl)-2,2'-bipyridyl, N-(n-propyl)pyridylmethanimine, N-(n-octyl)pyridylmethanimine, and the like are included, more preferably 2,2-bipyridyl is included.

A reducing agent includes alcohol, aldehyde, phenols, and organic compound, and the like, preferably includes an organic acid compound. An organic acid compound includes citric acid, oxalic acid, ascorbic acid, ascorbate, ascorbic acid ester, and the like, preferably includes ascorbic acid, ascorbate, ascorbic acid ester, and the like, more preferably includes ascorbic acid.

A water-based solvent such as a buffering liquid is used as a solvent, which preferably includes phosphate buffer liquid. In addition, a dissolving agent preferably comprises NaCl, wherein the concentration of NaCl includes, for example, 100 to 200 mM, preferably 120 to 160 mM. In addition, the pH of the dissolving agent includes, for example, 6 to 8, preferably 6.8 to 7.8, more preferably 7.2 to 7.6.

When a polymer chain elongates from a polymerization initiating group **INI** which is a radical generation source with the polymerizable monomer **M28** as a substrate, the thickness of the polymer film is increased and the elongated polymer chain reaches the mold **60** surface, the modified polymerizable functional group **27** is also taken in as a substrate at said surface to synthesize the molecularly imprinted polymer **20a** so as to form the recessed part **21** in the shape along the surface shape of the mold **60.** The polymer film of the molecularly imprinted polymer **20a** can grow to a thickness corresponding to about 1/3 to 1/2 of the upper and lower diameters (when the upper side of the drawing is considered up) of the mold **60** introduced to the base material **30.** This causes obtainment of a polymer film of the molecularly imprinted polymer **20a.**

<3-1-5. Step **(1-4)**>

In step **(1-4),** as shown in Figure **12-5,** the mold **60** is removed by cleaving the first reversible linking group **22** for conversion to the group **22'** for binding a specific binding molecule to a target, and cleaving the second reversible linking group **23** for conversion to the group **23'** for binding a signal substance. Since the second reversible linking group **23** is delivered only to the surface of the mold **60** in the above-mentioned surface modification step, the group **22'** for binding a specific binding molecule remains in the molecular imprint recessed part **21** which is a trace of removal of the mold **60,** and the group **23'** for binding a signal substance occurred from the second reversible linking group **23** is disposed only within the recessed part **21.** This causes obtainment of the base material **10a-C'** for making a sensor for analysis.

<3-2. Manufacturing method of a base material for making a sensor for analyzing a detection subject that is particle-like-1>

For example, the manufacturing method of the base material **10-P'** for making a sensor for analyzing a detection subject that is base plate-type which is shown in Figure **6(A)** comprises the following step **(1-11)** to step **(1-13):**

*the step **(1-11)** of preparing a mold of a molecular imprint wherein the surface is modified with a polymerizable functional group via a first reversible linking group;
*the step **(1-12)** of using said polymerizable functional group as a substrate and synthesizing a molecularly imprinted polymer for a part of said surface of said mold; and
*the step **(1-13)** of cleaving said first reversible linking group and removing said mold from said molecularly imprinted polymer to generate said group for binding a specific binding molecule.

Provided that said group for binding a specific binding molecule is a group adapted to a small substance.

Figure **13-1** to Figure **13-3** schematically show a manufacturing method of the base material **10-P'** for making a sensor for analyzing a detection subject that is base plate-type which is shown in Figure **6(A).** Specifically, Figure **13-1,** Figure **13-2,** and Figure **13-3** schematically show step **(1-11),** step **(1-12),** and step **(1-13),** respectively. In other words, in the depicted embodiment, the method of manufacturing the base material **10-P'** for making a sensor for analyzing a detection subject comprises the following steps:

*the step **(1-11)** of preparing the mold **60** of a molecular imprint wherein the surface is modified with the polymerizable functional group **27** via the first reversible linking group **22**;
*the step **(1-12)** of using the polymerizable functional group **27** as a substrate to synthesize the molecularly imprinted polymer **20** for a part of the surface of the mold **60;** and
*the step **(1-13)** of cleaving the first reversible linking group **22** to remove the mold **60** from the molecularly imprinted polymer **20** and cause the group **22'** for binding a specific binding molecule.

Each step is discussed in detail below while referring to the drawings.

<3-2-1. Step **(1-11)**>

In step **(1-11),** as shown in Figure **13-1** (Figure **13-1a** and Figure **13-1b**), a mold **60** of a molecular imprint, wherein the surface is modified with a polymerizable functional group **27** via the first reversible linking group **22,** is prepared.
In Figure **13-1a,** the surface of the mold $\mathbf{60_1}$ of the molecular imprint, wherein the surface is modified with the binding group **22"**, causes the monomer $\mathbf{M22_1}$ comprising the group **22'** for binding a specific binding molecule to a target of a detection subject and the polymerizable functional group **27** to act, to carry out modification with the polymerizable functional group **27** via the first reversible linking group **22** formed by the reaction between the binding group **22"** and the group **22'** for binding a specific binding molecule.
In Figure **13-1a,** the binding group **22"** is not particularly limited, but includes, for example, the groups shown in column **1-2** of Table **1** above, preferably includes a thiol group. The group **22'** for binding in the polymerizable monomer $\mathbf{M22_1}$ is not particularly limited, and includes, for example, a group shown in column **1-1** of Table **1** above, preferably includes a pyridyl disulfide group. The first reversible linking group **22** is not particularly limited, which includes, for example, the groups shown in column **1-3** of Table **1** above, preferably includes a disulfide bond.
In addition, in the example of Figure **13-1b**, the mold $\mathbf{60_2}$ having the binding group **BG22"** at the surface is used to react the polymerizable monomer $\mathbf{M22_2}$ comprising the binding group **BG22'** that corresponds to the binding group **BG22"**, the first reversible linking group **22,** and the polymerizable functional group **27** to obtain the mold $\mathbf{60_2}$ modified with the binding group **22"** via the first reversible linking group **22.**
In Figure **13-1b,** the binding group **BG22"** is not particularly limited, but includes, for example, the groups shown in column **2-1** of Table **2** above, preferably includes an amino group. The binding group **BG22'** in the polymerizable monomer $\mathbf{M22_2}$ is not particularly limited, which includes, for example, the groups shown in column **2-2** of Table **2** above, preferably includes a carboxylic acid-active ester group as the binding group **BG22'.** In addition, the first reversible linking group **22** is not particularly limited, which includes, for example, the groups shown in column **1-3** of Table **1** above, preferably includes a disulfide bond.
In Figure **13-1a** and Figure **13-1b,** the polymerizable functional group **27** should only contain a vinyl group, preferably includes a (meth)acryloyl group.
In Figure **13-1a** and Figure **13-1b,** the mold $\mathbf{60_1}$ and the mold $\mathbf{60_2}$ (hereinafter, collectively described as the "mold **60**") are as discussed in "(Mold)" of "3-1-2. Mold and modification step" above.
As such, the mold **60** modified with the polymerizable functional group **27** via the first reversible linking group **22** is obtained at the surface.

<3-2-2. Step **(1-12)**>

In step **(1-12),** as shown in Figure **13-2,** the polymerizable functional group **27** is used as a substrate to synthesize the molecularly imprinted polymer **20** for a part of the surface of the mold **60.**
In the depicted embodiment, an example using **M28** and **M29** as a polymerizable monomer is shown, but three or more types may be combined as the polymerizable monomer. In addition, the polymerizable monomer is as discussed in "1-3-6. A polymer configuring a molecularly imprinted polymer" above. The polymerizable monomer **M28** used in the depicted example is a biocompatible monomer, wherein the biocompatible monomer is as discussed in "(A unit derived from a biocompatible monomer)" of "1-3-6. A polymer configuring a molecularly imprinted polymer" above. The polymerizable monomer **M29** used in the depicted example is N-substituted or unsubstituted (meth)acrylamide, wherein the N-substituted or unsubstituted (meth)acrylamide is as discussed in the "(A unit derived from N-substituted or unsubstituted (meth)acrylamide)" of "1-3-6. A polymer configuring a molecularly imprinted polymer" above.
The concentration of the mold **60** in a polymerization reaction liquid is not particularly limited, but includes, for example, 1 to 10 μmol/L, preferably 3 to 8 μmol/L, more preferably 4.5 to 7 μmol/L, further preferably 5.5 to 6.5 pmol/L.
The concentration of the polymerizable monomer **M28** in a polymerization reaction liquid is not particularly limited, but includes, for example, 1 to 10 mmol/L, preferably 3 to 8 mmol/L, more preferably, 4.5 to 7 mmol/L, further preferably 5.5 to 6.5 mmol/L. In addition, the amount of use of the polymerizable monomer **M28** with respect to the mold

**60** includes, as the amount of use of the polymerizable monomer **M28** per 1 μmol of the mold **60,** for example, 0.1 to 2 mmol, preferably 0.3 to 1.8 mmol, more preferably 0.5 to 1.5 mmol, further preferably 0.8 to 1.2 mmol.

The concentration of the polymerizable monomer **M29** in a polymerization reaction liquid is not particularly limited, but includes, for example, 50 to 200 mmol/L, preferably 80 to 130 mmol/L, more preferably 100 to 115 mmol/L. In addition, the amount of use of the polymerizable monomer **M29** with respect to the mold **60** includes, as the amount of use of the polymerizable monomer **M29** per 1 pmol of the mold **60,** preferably, for example, 8 to 35 mmol/L, preferably 13 to 22 mmol/L, more preferably 17 to 20 mmol/L. Furthermore, the amount of use of the polymerizable monomer **M29** with respect to the polymerizable monomer **M28** includes, as the amount of use of the polymerizable monomer **M29** with respect to 1 mol of polymerizable monomer **M28,** 8 to 35 mol, preferably 13 to 22 mol, more preferably 17 to 20 mol.

The polymerization reaction liquid appropriately comprises a crosslinking agent, initiating agent, and solvent.

A crosslinking agent can include a compound wherein two or more ethylenically unsaturated groups are bound by a linker group. An example of a specific crosslinking agent includes the matter shown in Formula **(6)** below.

[Chemical 15] W-Z-W (6)

In Formula **(6),** the W represents ethylenically unsaturated groups that may the same or different with respect to one another, and Z represents a linker group. An ethylenically unsaturated group includes an acrylic group and a methacrylic group. A linker group includes, for example, with the carbon number of 1 to 6, preferably 2 to 6, an alkylene group, amino group (-NH-), ether group, carbonyl group, ester bond (-COO- or -OCO-), amide bond (-CONH- or - NHCO-), sulfoxide group (-SO-), sulphonyl group ($-SO_2-$), and a group wherein two or more of the above are bound. When two or more of the above-mentioned groups are bound to configure the above-mentioned linker, said bond number is preferably 5 or less or 4 or less, more preferably 3 or less or 2. A more specific example of a crosslinking agent includes a low molecule crosslinking agent such as N,N'-methylene bisacrylamide, and ethylene glycol dimethacrylate, preferably N,N'-methylene bisacrylamide.

The concentration of the crosslinking agent in the polymerization reaction liquid is not particularly limited, but includes, for example, 1 to 10 mmol/L, preferably 3 to 8 mmol/L, more preferably 4.5 to 7 mmol/L, further preferably 5.5 to 6.5 mmol/L.

An initiating agent includes peroxide such as ammonium persulfate and potassium persulfate, and azoic polymerization initiating agent such as azobisisobutyronitrile and 2,2'-azobis(2-methylpropionamidine) dihydrochloride, preferably includes an azoic polymerization initiating agent, more preferably 2,2'-azobis(2-methylpropionamidine) dihydrochloride.

The concentration of the initiating agent in the polymerization reaction liquid is not particularly limited, but includes, for example, 4 to 40 mmol/L, preferably 12 to 32 mmol/L, more preferably 18 to 28 mmol/L, further preferably 22 to 26 mmol/L.

A water-based solvent such as a buffering liquid is used as a solvent, which preferably includes a phosphate buffer liquid. In addition, a dissolving agent preferably comprises NaCl, wherein the concentration of NaCl includes, for example, 100 to 200 mM, preferably 120 to 160 mM. In addition, the pH of the dissolving agent includes, for example, 6 to 8, preferably 6.8 to 7.8, more preferably 7.2 to 7.6.

A specific polymerization method includes a deemulsifying agent precipitation polymerization method, dispersion polymerization method, emulsion polymerization method, seed emulsion polymerization method, and the like, preferably includes deemulsifying agent precipitation polymerization method.

Progression of polymerization reaction in such a polymerization reaction system causes synthesis of the molecularly imprinted polymer **20** and formation of the molecular imprint recessed part **21** complementary to the surface shape of the mold **60.** In addition, the molecular imprint recessed part **21** is efficiently formed due to the fact that the mold **60** can easily exist near the polymer boundary surface.

<3-2-3. Step **(1-13)**>

In step **(1-13),** as shown in Figure **13-3,** the first reversible linking group **22** is cleaved to remove the mold **60** from the molecularly imprinted polymer **20** and cause the group **22'** for binding a specific binding molecule. This causes obtainment of the base material **10' (10-P')** for making a sensor for analysis.

<3-3. Manufacturing method of a base material for making a sensor for analyzing a detection subject that is particle-like-2>

For example, the manufacturing method of the base material **10c-P'** for making a sensor for analyzing a detection subject that is base plate-type which is shown in Figure **10(A)** comprises the following step **(1-21)** to step **(1-24):**

*the step **(1-21)** of preparing a surface of a mold of a molecular imprint wherein the surface is modified with a

polymerizable functional group via a third reversible linking group;

*the step **(1-22)** of using a monomer comprising said polymerizable functional group and an interaction group for said mold as a substrate and synthesizing a molecularly imprinted polymer for a part of said surface of said mold;

*the step **(1-23)** of cleaving said third reversible linking group and removing said mold from said molecularly imprinted polymer; and

*the step **(1-24)** introducing a molecule having a functional group comprising a group for binding a specific binding molecule to a target of a detection subject and a group for binding a signal substance via the third reversible linking group.

Provided that said group for binding a specific binding molecule is a group adapted to a small substance.

In addition, when the interaction group **25** for a mold is not comprised as in **10b-PC'** of Figure **11,** only a monomer comprising an interaction group for a mold should be excluded from the substrate in the above-mentioned step **(1-22).**

Figure **14-1** to Figure **14-4** schematically show a manufacturing method of the base material **10c-P'** for making a sensor for analyzing a detection subject that is base plate-type which is shown in Figure **10(A).** Specifically, Figure **14-1,** Figure **14-2,** Figure **14-3,** and Figure **14-4** schematically show step **(1-21),** step **(1-22),** step **(1-13),** and step **(1-14),** respectively. In other words, in the depicted embodiment, the method of manufacturing a base material **10c-P'** for making a sensor for analyzing a detection subject comprises the following steps:

*the step **(1-21)** of preparing the mold **60c** of a molecular imprint wherein the surface is modified with a polymerizable functional group **27** via the third reversible linking group **26b;**

*the step **(1-22)** of using the monomer **M25** comprising the polymerizable functional group **27** and the interaction group **25** for the mold **60c** as a substrate and synthesizing the molecularly imprinted polymer **20c** for a part of the surface of the mold **60;**

*the step **(1-23)** of cleaving the third reversible linking group **26b** and removing the mold **60c** from the molecularly imprinted polymer **20c;** and

*the step **(1-24)** of introducing the functional molecule **R24** having the functional group **24b** comprising the group **22'** for binding a specific binding group for a target of a detection subject and the group **23'** for binding a signal substance via the third reversible linking group **26b.**

Each step is discussed in detail while referring to the drawings.

<3-3-1. Step (1-21)>

In step **(1-21),** as shown in Figure **14-1,** the mold 60c of a molecular imprint, wherein the surface is modified with a polymerizable functional group **27** via the third reversible linking group **26b,** is prepared.

The mold **60c** includes those discussed in "(Mold)" of "3-1-2. Mold and modification step", but preferably includes a biomolecule, more preferably includes a plasma protein (albumin, γ-globulin, fibrinogen, and the like), more preferably includes albumin.

In the depicted embodiment, the mold **60c** itself comprises an interaction site **65c** and a binding group **BG6"** on the surface. The interaction site **65c** is not particularly limited, but includes, for example, the groups shown in column **3-2** of Table **3** above, preferably includes an acidic amino acid residue, more preferably includes a carboxyl group. The binding group **BG6"** is not limited, and includes the groups shown in column **2-1** of Table **2** above, preferably includes an amino group.

The monomer **M27** comprising the binding group **BG6'** corresponding to the binding group **BG6**", the third reversible linking group **26b,** and the polymerizable functional group **27** is acted on the mold **60c** to obtain the mold **60c** of a molecular imprint wherein the surface is modified with the polymerizable functional group **27** via the third reversible linking group **26b.**

The binding group **BG6'** is not particularly limited, but includes, for example, the groups shown in column **2-2** of Table **2** above, preferably includes a carboxylic acid-active ester group. The reversible linking group **26b** is not particularly limited, which includes, for example, the groups shown in column **1-3** of Table **1** above, preferably includes a disulfide group. The polymerizable functional group **27** should only contain a vinyl group, which preferably includes a (meth)acryloyl group.

<3-3-2. Step **(1-22)**>

In step **(1-22),** as shown in Figure **14-2,** the polymerizable functional group **27** is used as a substrate to synthesize the molecularly imprinted polymer **20c** for a part of the surface of the mold **60.**

The depicted embodiment shows an example of using the monomer **M25** comprising the interaction group **25** for the mold **60c** as well as the polymerization monomers **M28** and **M29** as a polymerization monomer, but three or more types may be used in combination as a polymerization monomer other than monomer **M25.** In addition, a polymerizable monomer other than the monomer **M25** is as discussed in "1-3-6. A polymer configuring a molecularly imprinted polymer" above. The polymerizable monomer **M28** used in the depicted example is a biocompatible monomer, wherein the biocompatible monomer is as discussed in "(A unit derived from a biocompatible monomer)" of "1-3-6. A polymer configuring a molecularly imprinted polymer" above. The polymerizable monomer **M29** used in the depicted example is N-substituted or unsubstituted (meth)acrylamide, wherein the N-substituted or unsubstituted (meth)acrylamide is as discussed in the "(A unit derived from N-substituted or unsubstituted (meth)acrylamide)" of "1-3-6. A polymer configuring a molecularly imprinted polymer" above.

In the monomer **M25** comprising the interaction group **25** for the mold **60c,** the interaction group **25** is as discussed in "1-3-5. Interaction group" above, preferably includes a pyrrolidyl group.

The concentration of the mold **60c** in a polymerization reaction liquid; the concentration of the polymerizable monomer **M28** and the amount of use of the polymerizable monomer **M28** with respect to the mold **60c;** and the polymeric monomer **M29,** the amount of use of the polymeric monomer **M29** with respect to the mold **60c** and the amount of use of the polymeric monomer **M29** with respect to the polymerizable monomer **M28** are the same as the concentration of the mold **60** in a polymerization reaction liquid; the concentration of the polymerizable monomer **M28** and the amount of use of the polymerizable monomer **M28** with respect to the mold **60;** and the polymeric monomer **M29,** the amount of use of the polymeric monomer **M29** with respect to the mold **60** and the amount of use of the polymeric monomer **M29** with respect to the polymerizable monomer **M28** discussed in "3-2-2. Step **(1-12)"** above.

The concentration of the monomer **M25** comprising the interaction group **25** is not particularly limited, but includes, for example, 1.5 to 15 mmol/L, preferably 4.5 to 12 mmol/L, more preferably 6 to 10 mmol/L, further preferably 8.5 to 9.5 mmol/L. In addition, the amount of use of the monomer **M25** comprising the interaction group **25** for the mold **60** includes, as the mount of use of the monomer **M25** per 1 μmol of the mold **60,** for example, 0.15 to 3 mmol, preferably 0.45 to 2.7 mmol, more preferably 0.75 to 2.2 mmol, further preferably 1.2 to 2.2 mmol. In addition, the amount of use of the monomer **M25** with respect to the polymerizable monomer **M28** includes, as the amount of use of the monomer **M25** with respect to 1 mol of the polymerizable monomer **M28,** for example, 0.3 to 3 mol, preferably 0.5 to 2.5 mol, more preferably 1 to 2 mol, further preferably 1.3 to 1.7 mol. Furthermore, the amount of use of the monomer **M25** with respect to the polymerizable monomer **M29** includes, as the amount of use of the monomer **M25** with respect to 100 mol of the polymerizable monomer **M29,** for example, 2 to 20 mol, preferably 5 to 13 mol, more preferably 8 to 9 mol.

The polymerization reaction liquid appropriately comprises a crosslinking agent, initiating agent, and solvent. The details of the crosslinking agent, initiating agent, and solvent and the amount of use, polymerization reaction type thereof are as discussed in "3-2-2. Step **(1-12)"** above.

In the polymerization reaction system of step **(1-22),** as shown in Figure **14-2,** the monomer **M25** comprising the interaction group **25** for the mold **60c** among the monomer components specifically interacts with the interaction site **65c** of the mold **60c** via the interaction group **25** to form a composite of a monomer **M25-**mold **60c.** Progression of polymerization reaction in a state in which such a composite is formed enables formation of the molecular imprint recessed part **21c** complementary to the surface shape of the mold **60c** as well as presence of the third reversible linking group **26b** on the molecular imprint recessed part **21c** surface. In addition, the molecular imprint recessed part **21c** is even more efficiently formed due to the fact that the mold **60c** can even more easily exist near the polymer boundary surface due to hydrophilicity.

In step **(1-22),** the polymerization reaction liquid may be exposed to a low temperature condition of 1 to 15°C, preferably 1 to 10°C, more preferably 2 to 6°C, in order to sufficiently form a composite of the monomer **M25**-mold **60c** before being exposed to the temperature condition that progresses polymerization reaction. The time of exposure to the low temperature condition includes, for example, 20 to 30 hours. The temperature condition that progresses polymerization reaction includes, for example, 40 to 76°C, preferably 50 to 74°C, more preferably 60 to 72°C. The time of exposure to the temperature condition that progresses polymerization reaction includes, for example, 10 to 20 hours. Furthermore, even when polymerization reaction is carried out under the heating condition, the shape of the mold **60c** is retained to the extent of not causing a denaturation that causes loss of the specificity of a molecular imprint space, and thus it is possible to effectively acquire specificity of the molecular imprint recessed part **21c.**

As such, the molecularly imprinted polymer **20c** is synthesized, and a composite of the molecularly imprinted polymer **20c** and the mold **60c** is obtained. The composite of the molecularly imprinted polymer **20c** and the mold **60c** is formed by a non-covalent bond between the interaction group **25** and the interaction site **65c** of the mold **60c.**

<3-3-3. Step **(1-23)**>

In step **(1-23),** as shown in Figure **14-3,** the third reversible linking group **26b** is cleaved to remove the mold **60c** from the molecularly imprinted polymer **20c.** This causes the interaction group **25** and the binding group **26b'** to be

provided on the recessed part **21c** surface of the molecularly imprinted polymer **20c.** The binding group **26b'** is a group that occurs by the cleavage of the third reversible linking group **26b,** which specifically includes the groups shown in column **1-1** of Table **1** above, preferably includes a thiol group.

<3-3-4. Step **(1-24)**>

In step **(1-24),** as shown in Figure **14-4,** the functional molecule **R24** having the functional group **24b** comprising the group **22'** for binding a specific binding molecule to a target of a detection subject and the group **23'** for binding a signal substance is introduced the recessed part **21c** of the polymer **20c** via the third reversible linking group **26b.**

The functional group **24b** comprising the group **22'** for binding a specific binding molecule to a target of a detection subject and the group **23'** for binding a signal substance is as discussed in "1-3-4. Functional group" above. The functional molecule **R24** having the functional group **24b** is a molecule having the binding group **26b''** for the binding group **26b',** which is represented by Formula **(7)** below.

[Chemical 16] $R^7-L^7-R^2-L^1-R^1$ (7)

In Formula **(7),** $R^1$ represents a group for binding a specific binding molecule, $L^1$ represents a direct binding or linking group, and $R^2$ represents a linking group comprising a group for binding a signal substance, wherein the details of these groups are as discussed in "1-3-4. Functional group" above.

In Formula **(7),** $R^7$ is the binding group **26b'',** and $L^7$ represents a direct binding or linking group. The binding group **26b''** is not particularly limited as long as the group has binding property for the binding group **26b',** which includes, for example, the groups shown in column **1-2** of Table **1** above. When the direct binding or linking group $L^7$ is a linking group, a specific example of said linking group includes an alkylene group that may comprise a polyoxyalkylene group (e.g., C1 to 8 alkylene group, or polyoxy C1 to 3 alkylene C1 to 8 alkylene group), carbonyl group, ester bond (-COO- or -OCO-), amide bond (-CONH- or - NHCO-), and a group consisting of any combination thereof. From the viewpoint of molecular design and the like, said linking group includes preferably a combination of an alkylene group and an ester bond and/or amide bond, more preferably a combination of an alkylene group and an ester bond or an amide bond, further preferably a combination of one or two or more alkylene group (the total of the carbon number of all alkylene groups comprised in said linking group is, for example, 1 to 8, preferably 2 to 6, more preferably 3 to 5, especially preferably 4) and an ester bond or an amide bond.

The specific unlimited example of the functional molecule **R24** having the functional group **24b** includes the compound shown in Formulas **(7a), (7b),** and **(7c)** below.

[Chemical 17]

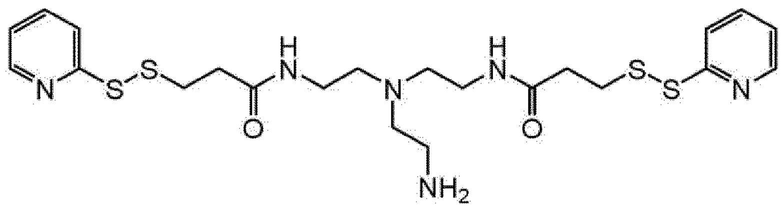

(7a)

[Chemical 18]

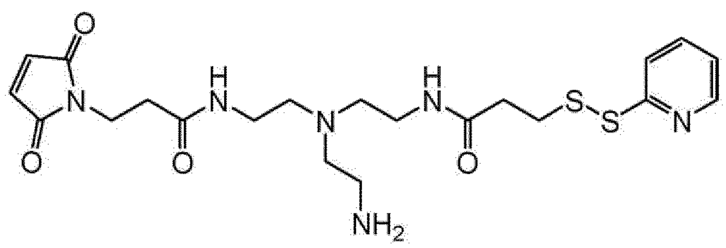

(7b)

[Chemical 19]

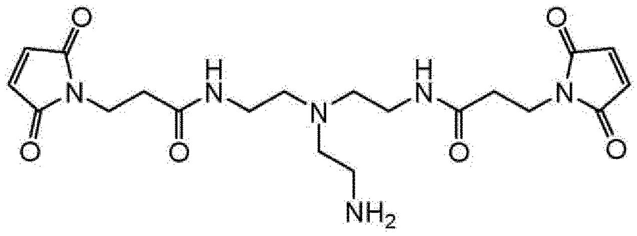

(7c)

As such, the functional molecule **R24** having the functional group **24b** is introduced to the recessed part **21c** of the polymer **20c** via a third reversible linking group **26b,** whereby the base material **10c'** (**10c-P'**) for making a sensor for analysis is obtained.

<3-4. Manufacturing method of a base material for making a sensor for analyzing a detection subject wherein a particle is immobilized on a base plate>

As shown in Figure **7(A)** and Figure **11(A),** the manufacturing method of the base material for making a sensor for analyzing a detection subject wherein a particle is immobilized on a base plate should only be an appropriate combination of the manufacturing method discussed in "3-2. Manufacturing method of a base material for making a sensor for analyzing a detection subject that is particle-like-1" and "3-3. Manufacturing method of a base material for making a sensor for analyzing a detection subject that is particle-like-2" above and the method of fixing a polymer particle on the base plate **30.**

The step of fixing a polymer particle on the base plate **30** can be carried out any time after forming a particle of a molecularly imprinted polymer (e.g., the molecularly imprinted polymers **10** and **10c**) where a mold (e.g., the molds **60** and **60c**) is removed in the manufacturing method discussed in "3-2. Manufacturing method of a base material for making a sensor for analyzing a detection subject that is particle-like-1" and "3-3. Manufacturing method of a base material for making a sensor for analyzing a detection subject is particle-like-2" above. For example, it is possible to fix the particle of the obtained molecularly imprinted polymers **10** and **10c** on the base plate **30** after the "3-2-3. Step **(1-13)"** above, after the "3-3-3. Step **(1-23)"** above (preferably between the "3-3-3. Step **(1-23)"** above and the "3-3-4. Step **(1-24)"** above), or the like. The method of fixing a particle of a molecularly imprinted polymer on the base plate **30** is not particularly limited, wherein those skilled in the art can appropriately determine a common method of fixing a polymer nanoparticle to a base plate surface.

Figure **15** schematically shows the step of fixing a particle of the molecularly imprinted polymer **20c** on the base plate **30** between the "3-3-3. Step **(1-23)"** above and the "3-3-4. Step **(1-24)"** above. In the example shown in Figure **15,** a molecular film having the binding group **BG9"** on the surface is formed on the base plate **30** surface and the binding group **BG9'** existing on the surface of the molecularly imprinted polymer **20c** is bound to the binding group **BG9"** to fix the molecularly imprinted polymer **20c** on the base plate **30.**

The functional group **BG9"** of the molecular film surface formed at the base plate **30** is not particularly limited, but includes, for example, the groups shown in column **2-2** of Table **2** above, preferably including a carboxylic acid-active ester group. The binding group **BG9'** that exists on the surface of the molecularly imprinted polymer **20c** is not particularly limited, which includes, for example, the groups shown in column **2-1** of Table **2** above. The binding group **BG9'** that exists on the surface of the molecularly imprinted polymer **20c** includes a group derived from a monomer used for synthesis of the molecularly imprinted polymer **20c** and/or a group derived from an initiating agent that remains at the terminal of a polymer configuring the molecularly imprinted polymer **20c,** and the like.

<4. Manufacturing method of a sensor for analyzing a detection subject>

The manufacturing method of a sensor for analyzing a detection subject of the present disclosure comprises the following steps:

the step **(1)** of carrying out the manufacturing method of a base material for making a sensor for analyzing a detection subject; and
the step **(2)** of binding said specific binding molecule to said group for binding a specific binding molecule.

Figure **13-4** schematically shows the manufacturing method of the sensor **10 (10-P)** for analyzing a detection subject of the present disclosure. In other words, in the embodiment shown in Figure **13-4,** the manufacturing method of the sensor **10 (10-P)** for analyzing a detection subject comprises the following steps:

the step **(1)** of carrying out the manufacturing method of the base material **10' (10-P')** for making a sensor for analyzing a detection subject; and
the step **(2)** of binding a specific binding molecule **R42** to the group **22'** for binding a specific binding molecule.

In addition, the manufacturing method of a sensor for analyzing a detection subject of the present disclosure of the case of comprising a group for binding signal substance in the base material for making a sensor for analyzing a detection subject comprises the following steps:

the step **(1)** of carrying out the manufacturing method of a base material for making a sensor for analyzing a detection subject;
the step **(2)** of binding said specific binding molecule to said group for binding a specific binding molecule; and
the step **(3)** of binding a signal substance to said group for binding a signal substance.

Figure **12-6** and Figure **14-5** schematically show the manufacturing method of the sensors **10a-C** and **10c (10c-P)** for analyzing a detection subject of the present disclosure. In other words, in the embodiment shown in Figure **12-6** and Figure **14-5,** the manufacturing method of the sensors **10a-C** and **10c (10c-P)** for analyzing a detection subject comprises the following steps:

the step **(1)** of carrying out the manufacturing method of the base materials **10a-C'** and **10c' (10c-P')** for making a sensor for analyzing a detection subject;
the step **(2)** of binding the specific binding molecule **R42** to a group for binding the specific binding molecule **22';** and
the step **(3)** of binding the signal substance **R43** to the group **23'** for binding a signal substance.

Step **(1)** is as discussed in the "3. Manufacturing method of a base material for making a sensor for analyzing a detection subject" above. When the manufacturing method of a sensor for analyzing a detection subject of the present disclosure carries out step **(3),** as for the order of step **(2)** and step **(3),** either can be carried out first, or they can be carried out simultaneously.

The specific binding molecule **R42** comprises the specific binding group **42** for target of a detection subject and binding groups **22"** and **22b'** '. The specific binding group **42** for a target of a detection subject is as discussed in the "2-2. A specific binding group for a target of a detection subject" above. Furthermore, although not depicted, the manufacturing method of the sensor **$10a_1$-C** for analysis (Figure **8(B2)**) should only carry out the same procedure as Figure **12-6** excluding the use of a molecule comprising the specific binding group **42,** (see Figure **2B-2**) having a different signal, wherein a different signal **sg** is bound to the specific binding group **42,** and the binding group **22"** as the specific binding molecule **R42.**

In addition, the binding group **22"** is not particularly limited as long as the group has binding property to the group **22'** for binding a specific binding molecule, which includes, for example, the groups shown in column **1-2** of Table **1** above. The binding group **22b''** is not particularly limited as long as the group has binding property to the group **22'** for binding a specific binding molecule, which includes, for example, the groups shown in column **2-2** of Table **2** above.

A signal substance **R43** comprises the signal group **43** and the binding groups **23"** and **23b".** The signal group **43** is as discussed in "2-3. Signal group" above. In addition, the binding group **23"** is not particularly limited as long as the group has binding property to the group **23'** for binding a signal substance, which includes, for example, the groups shown in column **1-2** of Table **1** above. The binding group **23b''** is not particularly limited as long as the group as binding property to the group **23'** for binding a signal substance, which includes, for example, the group of column **2-2** of Table **2** above.

In a manufacturing method of a sensor for analyzing a detection subject of the present disclosure (e.g., the sensor **10a-C** for analyzing a detection subject discussed above, or the like) which is base plate-type and has the signal group **43,** a group for binding a signal substance is only provided at a molecular imprint recessed part that forms a sensor field on the base plate **30,** which enables the signal group **42** to be disposed only at a molecular imprint recessed part due to the reaction property of the binding group of the signal substance **R42.**

In addition, regarding the sensor for analyzing a detection subject of the present disclosure that is base plate-type and has the signal group **43,** it is possible to introduce one type of specific binding group **42** and one type of signal group **43** to all molecular imprint recessed parts on one base plate **30,** and it is also possible to introduce one type of specific binding group **42** to one molecular imprint recessed part, introduce another type of specific binding group **42** to another molecular imprint recessed part, and introduce different types of signal groups **43** corresponding to the types of the specific binding group **42,** respectively.

<5. Analysis method of a subject substance>

The analysis method of a detection subject of the present disclosure comprises: the step of contacting a sample comprising a detection subject to a sensor for analyzing a detection subject and binding said detection subject to said specific binding group; and the step of detecting the bond between said specific binding group and said detection subject.

<5-1. Sensor for analyzing a detection subject>

The sensor for analyzing a detection subject is as discussed in "2. Sensor for analyzing a detection subject" above.

<5-2. Detection subject>

The detection subject is not particularly limited as long as the target of the detection subject is a subject that specifically binds to the specific binding group **42,** which includes those discussed in "2-1. Detection subject" above.

The form of the analysis sample liquid comprising a detection subject is not particularly limited, but from the viewpoint of rapidity of analysis, those that did not go through the treatment of separating the detection subject is included. The treatment of separating a detection subject includes ultracentrifugation, ultrafiltration, successive flow electrophoresis, filtration using a size filter, gel filtration chromatography, and the like.

The analysis sample liquid comprising a detection subject may be a sample obtained from an environment where a detection subject exists (when the detection subject is a cell or an extracellular vesicle), or a sample obtained from an environment where a detection subject may be generated (when the detection subject is an extracellular vesicle and is a product or discharge from a cell). Specifically, the analysis sample liquid may be a biological sample comprising a cell. When the detection subject is an extracellular vesicle such as exosome, a cell that produces or discharges the detection subject includes a cancer cell, mast cell, dendritic cell, reticulocyte, epithelial cell, B cell, nerve cell, mucosal cell, and the like. More specifically, an analysis test liquid comprising a detection subject includes body fluids such as blood, milk, urine, saliva, lymph, cerebrospinal fluid, amniotic fluid, tear fluid, sweat, nasal discharge, throat swab, nasopharyngeal liquid, and the like, and further includes a treatment liquid wherein these body fluids underwent pretreatment such as removal of unnecessary components, and culture liquid obtained by culturing cells comprised in these body fluids. Among these analysis test liquids, body fluids such as urine, saliva, tear fluid, sweat, and nasal discharge are particularly preferable in terms of non-invasiveness and easiness of collection.

When a sample comprising a detection subject is contacted to a sensor for analyzing a detection subject, the detection subject or a target of the detection subject is specifically captured by the specific binding group **42** within a molecular print recessed part.

<5-3. Targeting and sensing>

When a detection subject or a target of the detection subject is specifically captured by the specific binding group **42** within a molecular print recessed part, the bond between the specific binding group and said detection subject is detected.

For example, when a sensor for analyzing a detection subject does not have the signal group **43** within a molecular print recessed part like the sensor **10** for analyzing a detection subject shown in Figure **1B-1,** said bond can be detected by surface plasmon resonance (SPR), reflectometric interference spectroscopy, or the like.

In addition, when a sensor for analyzing a detection subject has the signal group **43** within a molecular print recessed part, since the signal group **43** would be affected by the environmental change by the detection subject or a target of the detection subject at the moment of binding, before and after the specific capturing, a signal would change. In other words, in this case, the sensor for analysis can read binding information of a detection subject or a target of the detection subject that would be a sensing subject with a signal change, which signal change causes detection of the detection subject or the target of the detection subject. Since the capturing of the detection subject or the target of the detection subject and the signal change occur almost simultaneously, there is no need to add a reagent for detection of binding, and detection can be promptly carried out.

In addition, in the case wherein a sensor for analysis is configured so that the signal group **43** would be one of the fluorescent dye pair that causes fluorescence resonance energy transfer (FRET), and one of said fluorescent dye pair is bound to a detection subject or a target of the detection subject beforehand, when the detection subject or the target of the detection subject is specifically captured by the specific binding group **42** within a molecular imprint recessed part, at the moment thereof, the fluorescent dye in the signal group **43** and the fluorescent dye in the detection subject would get close, and thus fluorescence is radiated by FRET. The detection subject is detected by this fluorescence radiation by this FRET. Since the capturing of a detection subject or a target of the detection subject and fluorescence

radiation by FRET occur almost simultaneously, there is no need to add a reagent for detection of the detection subject, and detection can be promptly carried out. The fluorescent dye pair that causes FRET is not particularly limited, and it is not limited which of a donor dye and acceptor dye is selected as the signal group **43.** Preferably, it is possible to select a donor dye as the signal group **43.** A specific example of the donor dye/acceptor dye configuring a pair of fluorescent dyes that causes FRET includes fluorescein isothiocyanate (FITC)/tetramethylrhodamine isothiocyanate (TRITC), Alexa Fluor647/Cy5.5, HiLyte Fluor647/Cy5.5, R-phycoerythrin (R-PE)/allophycocyanin (APC), and the like.

<5-4. Double targeting and sensing>

In addition, Figure **16-1** and Figure **16-2** schematically show the targeting trend of the case wherein the sensor **10c-P** for analyzing a detection subject shown in Figure **10(B)** has a molecular imprint recessed part **21c** which uses albumin (may be other plasma protein) as a mold **60c** and a specific interaction group **25** for albumin and is used for the purpose shown in "2-4. Usage" above. The depicted example shows the case wherein the detection subject **70** of the sensor **10c-P** for analysis is a microparticle having a film structure like exosome and the target **72** which is a direct target is a tumor marker protein expressed on the surface of said microparticle (specific example of the case of being "Embodiment **2"** of "2-1. Detection subject" above). In addition, Figure **16-1** and Figure **16-2** both schematically show both the enlarged view **(i)** of the molecular imprint recessed part **21c** and the total view **(ii)** of the sensor **10c-P** for analysis of a detection subject corresponding to **(i).**

As shown in Figure **16-1,** when the sensor **10c-P** for analysis is administered in blood, first, albumin which is the many plasma proteins that exist in blood (same substance as the mold **60c**) is specifically captured by the molecular imprint recessed part **21c** which uses the albumin in the sensor **10c-P** for analysis as a mold and a specific interaction group **25** for albumin. This causes the sensor **10c-P** for analysis to capture the stealth property *in vivo.* In this case, the specific binding group **42** is shielded by the captured albumin (same substance as the mold **60c**).

Meanwhile, when the sensor **10c-P** for analysis that captured the stealth property reaches a tumor tissue, the albumin concentration would decrease in the tumor tissue, and thus, as shown in Figure **16-2,** the captured albumin would be taken out, exposing the specific binding group **42,** and a tumor marker protein (target **72**) specifically binds to the exposed specific binding group **42.** This binding information is detected as a signal change based on the signal group. Furthermore, when the detection subject **70** is a microparticle having a film structure and the target **72** thereof is set as a direct capturing subject as shown in Figure **16-2(ii),** the molecular imprint recessed part **21c** does not have to be in the size that can receive the entirety of the detection subject **70,** but should only be in the size that can receive the target **72** near the specific binding site.

As such, in in the embodiment shown in Figure **16-1** and Figure **16-2,** the sensor **10c-P** for analysis enables double targeting by the difference in the environment (albumin concentration) where the sensor is exposed. When the object is such double targeting, the combination of the mold **60c** of the molecular imprint recessed part **21c** and the interaction group **25** and the specific binding group **42** is not limited to the embodiment shown in Figure **16-1** and Figure **16-2,** and can be optionally selected.

In the discussion above, explanation has been provided while referring to the case wherein the sensor **10c-P** for analyzing a detection subject has the molecular imprint recessed part **21c** which uses albumin as the mold **60c** and the specific interaction group **25** for albumin and is administered in blood to be delivered near a tumor, but the double targeting is not limited to this example. As long as the sensor **10c-P** for analyzing a detection subject has the molecular imprint recessed part **21c** and the interaction group **25** for the mold thereof, regardless of what kind of mold is used as the mold **60c,** the embodiment shown in Figure **16-1** would be achieved in an environment wherein the concentration of the mold **60c** of the molecular imprint recessed part **21c** is high, and the embodiment shown in Figure **16-2** would be achieved in an environment wherein the concentration of the mold **60c** of the molecular imprint recessed part **21c** is low.

In one embodiment, a method of diagnosing and examining a subject using the sensor for analysis of the present disclosure, comprises:

A) the step of collecting a sample (comprising, for example, urine, cerebrospinal fluid, sweat, nasal mucus, saliva, blood, or tear) of a subject;
B) the step of contacting the sample with the sensor for analysis;
C) the step of detecting a signal from the sensor for analysis; and
D) the step of analyzing the signal and then diagnosing the subject (e.g., determining presence/absence or progression of a disease).

In one embodiment, a program that causes a computer to execute a method of diagnosing and examining a subject using a diagnosis or examination system comprising the sensor for analysis of the present disclosure, comprises:

A) the step of providing a sample (comprising, for example, urine, cerebrospinal fluid, sweat, nasal mucus, saliva,

blood, or tear) obtained from the subject to the diagnosis or examination system;
B) the step of activating the diagnosis or examination system to contact the sample with the sensor for analysis;
C) the step of the diagnosis or examination system analyzing a signal from the sensor for analysis; and
D) the step of the diagnosis or examination system analyzing the signal and then diagnosing or detecting the subject (e.g., determining presence/absence or progression of a disease from a detection result).

In one embodiment, a record medium storing a program that causes a computer to execute a method of diagnosing or examining a subject using the sensor for analysis of the present disclosure, comprises:

A) the step of providing a sample (comprising, for example, urine, cerebrospinal fluid, sweat, nasal mucus, saliva, blood, or tear) obtained from the subject to the diagnosis or examination system;
B) the step of activating the diagnosis or examination system to contact the sample with the sensor for analysis;
C) the step of the diagnosis or examination system analyzing a signal from the sensor for analysis; and
D) the step of the diagnosis or examination system analyzing the signal and then diagnosing or detecting the subject (e.g., determining presence/absence or progression of a disease from a detection result).

In one embodiment, a diagnosis or examination system, comprises:

A) a providing unit that provides a sample (comprising, for example, urine, cerebrospinal fluid, sweat, nasal mucus, saliva, blood, or tear) obtained from the subject;
B) an activation unit that activates so as to contact the sample to the sensor for analysis;
C) a detection unit that detects a signal from the sensor for analysis; and
D) a diagnosis unit that analyzes the signal and then provides diagnosis to or examines the subject (e.g., determines presence/absence or progression of disease from a detection result).

Said step B) to step D) can all be fully automatically carried out, wherein when they are fully automatically carried out, the steps can be carried out using machine learning and/or artificial intelligence, and the method, program, record medium which is a record thereof, and system which realize these steps are also within the scope of the present disclosure.

Generally, the various exemplified embodiments of the present disclosure may be implemented with a hardware or dedicated circuit, software, logic, or any combination thereof. Some embodiments may be implemented with a hardware, and other embodiments may be implemented with a firmware or software that can be executed by a controller, microprocessor, or other computing devices. While the exemplary embodiment of the present disclosure can be shown as a block diagram, flowchart, or using other several graphical expressions, it is understood that the block, apparatus, system, technique, or method described herein may be implemented with, as a non-limiting example, hardware, software, firmware, dedicated circuit or logic, general hardware or controller, or other computing devices, or some sort of combination thereof.

In one embodiment, the present disclosure also provides at least one computer program product tangibly stored on a non-transitory computer readable medium, which realizes the technique of the present disclosure. The computer program product comprises a computer executable order such as those comprised in a program module, which is executed by a device on an actual processor or on a virtual processor that is subjected in order to execute the method/process described in herein. Generally, a program module comprises a routine, program, library, object, class, component, data structure, and the like, which execute a specific task or implement a specific abstract data type. The function of the program module may be combined or divided between program modules as desired in various embodiments. The computer executable order for a program module may be executed within a local device or a dispersion device. In the dispersion device, the program module may be disposed in both local and remote storage media.

The storage medium of the present disclosure may be a computer readable medium, and the computer readable medium may be a computer readable signal medium or computer readable storage medium. The computer readable medium includes, but not limited to, an electronic, magnetic, optical, electromagnetic, infrared, or semiconductor system, apparatus, or device, or any appropriate combination of the above. A more specific example of a computer readable storage medium includes one or more wired electrical connection, portable computer diskette, hard disk, random access memory (RAM), read-only memory (ROM), erasable programmable read-only memory (EPROM or flash memory), optical fiber, portable compact disk read-only memory (CD-ROM), optical storage device, magnetic storage device, or any appropriate combination of the above.

The computer program code for executing the method disclosed herein may be written in any combination of one or more programming language. When a program code is executed by a processor or a controller, the program code may be provided to a general computer dedicated computer, or processor or controller of other programmable data treatment apparatuses so as to execute the function/operation designated in the flowchart and/or block diagram.

A program code may be executed completely on a computer, executed partially on a computer as a stand-alone software package, executed partially on a computer and partially on a remote computer, or executed completely on a remote computer or a server. A program code may be distributed to a specially programmed device that may be generally called a "module" herein. The software component portion of the module may be written in any computer language, may be a portion of a monolithic code base, or may be developed at a more discrete code portion as it is typical in an object-oriented computer language. In addition, the module may be dispersed throughout a plurality of computer platforms, servers, terminals, mobile devices, or the like. Furthermore, a predetermined module may be implemented so that an explained function would be executed by a separate processor and/or computing hardware platform.

While the operation is shown in a specific order, in order to obtain a desired result, it should not be understood that such operation should be executed in the shown specific order or sequence, or all of the depicted operations are requested to be executed. In certain situations, multitask and parallel treatment may be advantageous. In the same manner, several specific implementation details are comprised in the explanation above, but they should not be interpreted as restriction to the scope of the present disclosure. Rather, they should be interpreted as explanations of a feature that may be unique to the specific embodiment. Certain features explained in the context of a different embodiment may be combined and implemented in a single embodiment. On the other hand, various features explained in the context of a single embodiment may be implemented separately or in any partial combination in a plurality of embodiments.

In the case of using the technique of the present disclosure, the technique is not intended to be used only to a human, but can also be used for other animals other than a human (cat, dog, cow, horse, bat, fox, mongoose, raccoon, and the like).

(Application Example)

1. Examination of a plague such as cancer of animals that are pets (cat, dog, bird, and the like)
2. Detection of infection-causing virus, infectious protein (prion, and the like), and other pathogenic/infectious factors held by domestic animals (cow, horse, pig, chicken, and the like)
3. Detection of infection-causing virus, infectious protein (prion, and the like), and other pathogenic/infectious factors held by harmful animal (bird, bat, fox, mongoose, raccoon, and the like)
4. Quality and freshness check of aquatic products
5. Quality and freshness check of farm products
6. Pathogenic bacteria/virus check of plants, trees, and the like

The present disclosure has been explained above while showing a preferable embodiment for easy understanding. The present disclosure is explained below based on the Examples, but the explanation above and the Examples below are provided only for the purpose of exemplification, and are not provided for the purpose of limiting the present disclosure. Therefore, the scope of the present disclosure is not limited by the embodiments or the Examples specifically described herein, but is limited only by the Claims.

[Examples]

The present disclosure is explained in detail below based on the Examples, but the present disclosure is not limited thereto. The used reagent can be obtained as shown by various descriptions, other reagents of Merck, Sigma-Aldrich, Funakoshi, and CosmoBio may be used.

CKX31 (OLYMPUS, Tokyo, Japan) was used for the microscopy of cells, KUBOTA2800 (KUBOTA, Tokyo, Japan) was used as a centrifugal separator, CO2 WATER JACKETED INCUBATOR (Thermo Fisher Scientific Inc, Massachusetts, USA) was used as an incubator, KS-243 (TOMY SEIKO Co,Ltd., Tokyo, Japan) was used as an autoclave, and a biological clean bench (ORIENTAL GIKEN INC, Tokyo, Japan) was used as a clean bench. qNano Gold (Izon Science Ltd., Christchurch, New Zealand) was used for exosome concentration measurement. In addition, UV Ozone Cleaner (BioForce Nanosciences, Inc.) was used for the UV ozone treatment of a gold base plate, fluorescence microscope (Olympus Corporation, Tokyo, Japan) with a SIC automatic dispensing apparatus (SYSTEM INSTRUMENTS Co.,Ltd., Tokyo, Japan) was used for fluorescence measurement, and Andor SOLIS (Andor Technology Ltd, Belfast, Northern Ireland) was used as a spectroscopic software.

In addition, MALDI-TOF/MS (MALDI-7090, SHIMADZU CORPORATION) was used as MALDI-TOF-MS, MALDI-TOF-MS analysis software (MALDI Solutions, SHIMADZU CORPORATION) was used, protein calibration standard I (BRUKER) was used for carburation. Sinapic acid was used for a matrix. J-725 Circular Dichroism spectrometer (JASCO Corporation, Tokyo, Japan) was used for CD spectrum. The pH was measured by a table pH meter F-52(HORIBA, Kyoto, JAPAN) for buffer preparation. Fluorescence spectrum measurement was carried out using the Fluorescence

spectrophotometer F-2500 (Tokyo, Japan) made by Hitachi High-Tech. Amicon Ultra-4 (10kDa) was used for the ultrafiltration film used for ultrafiltration. Thermo Scientific™ Nano dropTM One ultramicro-ultraviolet-visible spectrophotometer (Thermo Fisher) was used for absorption spectrum measurement. The average particle size and polydispersion index (PDI) of the particle that was made were measured by Dynamic Light Scattering (DLS) with ZETASIZER NANO-ZS MAL500735 (Malvern, UK), and Zetasizer software was used as an analysis software. A transmission electron microscope (JEM-1230, made by JEOL) was used for TEM analysis.

<Example 1: Analysis sensor **$10a_1$-C** of a detection subject that is base plate-type (introducing an anti-HER2 affibody as a specific binding substance and Alexa Fluor 647 as a signal substance)>

In the present Example, an analysis sensor **$10a_1$-C** of a detection subject that is base plate-type (introducing AlexaFluor 647 as a signal substance) was made. This analysis sensor of a detection subject that is base plate-type set exosome secreted by an SK-BR-3 cell which is an HER2 overexpress-type breast cancer cell line as the detection subject, set HER2 as the target of the detection subject, and selected and introduced anti-HER2 affibody as a specific binding molecule. In addition, Alexa Fluor 647 was introduced as a signal substance. Furthermore, the detection subject was analyzed using the obtained analysis sensor of a detection subject that is base plate-type.

Regarding the method of making an analysis sensor of a detection subject that is base plate-type, a base plate **10a-C'** for making an analysis sensor of a detection subject that is base plate-type was made in accordance with Figure **12-1,** Figure **12-2,** Figure **12-3a,** Figure **12-4,** and Figure **12-5,** and an analysis sensor **$10a_1$-C** of the detection subject was made in accordance with Figure **12-6** (provided that a molecule comprising a specific binding group **42,** (see Figure **2B-2**) having a different signal group and binding group **22",** wherein a different signal group **sg** is bound to the specific binding group **42,** was used as the specific binding molecule **R42**) .

<1> Making of a silica particle modified with a polyhistidine tag and a thiol group

In accordance with Figure **12-1a,** a mold silica particle modified with a polyhistidine tag and a thiol group was made.

<1-1> Reagent

*Sicastar(R)-red F (micromod; average particle size of 200nm; solid component concentration of 25 mg/mL; the functional group of the particle surface is a carboxyl group (1 μmol/g) ; the fluorescence property is 569 nm excitation, 585 nm fluorescence)

**Mold **60:** silica particle
**Binding group **BG22":** carboxyl group

*2-aminoethanethiol hydrochloride (Tokyo Chemical Industry)

**Binding group **BG12':** amino group
**Binding group **23":** thiol group

*Polyhistidine tag (Thermo Fisher Scientific; His-tag;Ac-KHHHHHH-$NH_2$; Ac represents acetyl group, K represents lysine residue, and H represents histidine residue)

**Binding group **BG22':** amino group
**Binding group **22":** polyhistidine

*DMT-MM(4-(4,6-dimethoxy-1,3,5-triazine-2-yl)-4-methylmorpholiniumchloride; triazine-based condensing agent)

<1-2> Experimental procedure

A silica particle modified with a polyhistidine tag and a thiol group was made by adding 20 equivalent of 2-aminoethanethiol hydrochloride, polyhistidine tag and DMT-MM to the carboxyl group of the Sicastar(R)-red F surface and shaking (25°C, overnight). Then, a dispersion medium was substituted with water by centrifugal separation (10,000 g, 15 minutes). Furthermore, the operation of substituting a dispersion medium with water by centrifugal separation

(10,000 g, 10 minutes) was carried out two times. 100 μL of the made silica particle was collected, and the dry weight was measured. The solid component concentration of the obtained silica particle was 7.41 mg/mL.

<2> Formation of a monolayer (step **(1-1)**)

In accordance with Figure **12-2,** a monolayer having a NTA-Ni complex and a polymerization initiating group on the surface was formed at the base plate.

<2-1> Reagent

*Amino-$EG_6$-undecanethiol hydrochloride (DOJINDO LABORATORIES)
**Binding group **BG1':** amino group
*2-(2-bromoisobutyryloxy) undecyl thiol (SURF MODS)
**Polymerizable initiating group **INI:** -CBr group
*Isothiocyanobenzyl-NTA (DOJINDO LABORATORIES), anhydrous $NiCl_2$ (NACALAI TESQUE)
**Group **22'** for binding a specific binding molecule: nickel-nitrilotriacetic acid-driven group

<2-2> Experimental procedure

After a gold sputtered or coated glass base plate was washed with ethanol, UV ozone treatment (20 minutes) was carried out to remove the organic matter on the gold base plate surface. Then, the base plate was immersed in an ethanol solution comprising 0.5 mM of amino-$EG_6$-undecanthiol hydrochloride and 0.5 mM of 2-(2-bromoisobutyryloxy) undecyl thiol and settled (25°C, overnight) to form a mixed self-assembled monolayer (mixed SAMs).
After washing the base plate with pure water, 35 μL of the base plate was dropped in a 5 mM of an isothiocyanobenzyl-NTA solution (prepared by dissolving in 50 μL of DMSO to be prepared to 46 mM, and then diluting with a carbonate buffer of 10 mM, pH9.2) and settled (room temperature, 2 hours). After washing the base plate with pure water, the base plate was immersed in 4 mM of $NiCl_2$ aqueous solution and settled (room temperature, 15 minutes) to form a NTA-Ni complex (nickel-nitrilotriacetic acid-driven group) on the mixed SAM of the base plate surface, and then the base plate was washed with pure water.

<3> Introduction of a mold (step **(1-2)**)

In accordance with Figure **12-3a,** a mold was introduced to the base plate, and the surface of the mold was modified with a methacryloyl group.

<3-1> Reagent or the like

*$M23_1$: 2-(2-pyridyl)dithioethyl methacrylamide

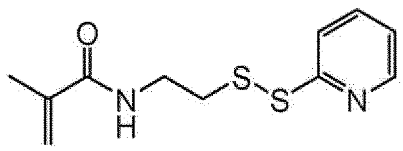

2-(2-pyridyl)dithioethyl methacrylamide

**Group **23'** for binding a signal substance: pyridyl disulfide group (converted to thiol group)
**Polymerizable functional group **27:** methacryloyl group
**First reversible linking group **22:** coordinate bond
**Second reversible linking group **23:** disulphide group

<3-2> Experimental procedure

A silica particle modified with a polyhistidine tag and a thiol group made in <1> above was dropped on the base substrate and settled (room temperature, 1 hour) to immobilize the silica particle.

After washing the base plate with pure water, 2-(2-pyridyl)dithioethyl methacrylamide was dissolved in 50 μL of DMSO and diluted with PBS (pH7.4) to prepare a PBS solution comprising 100 μM of 2-(2-pyridyl)dithioethyl methacrylamide. The base plate was immersed in this solution and settled (25°C, overnight) to introduce the methacryloyl group to the silica particle surface by disulfide exchange reaction.

<4> Synthesis of a molecularly imprinted polymer (step **(1-3)** )

In accordance with Figure **12-4,** a polymer film of a molecularly imprinted polymer was synthesized.

<4-1> Reagent and polymerization recipe

*M28: 2-methacryloyloxyethyl phosphorylcholine (MPC)

[Table 4]

| Reagent | Concentration |
|---|---|
| 2-Methacryloyloxyethyl Phosphorylcholine (MPC) | 50 mM |
| 2,2'-Bipyridyl | 2 mM |
| $CuBr_2$ | 1 mM |
| L-Ascorbic acid | 0.5 mM |
| PBS (10 mM phosphate, 140 mM NaCl, pH7.4) | 10 mL |

<4-2> Experimental procedure

In accordance with the polymerization recipe of Table **4,** a surface-initiated atom transfer radical polymerization (SI-ATRP) was carried out on the base plate obtained in <3> above to synthesize a polymer thin film of a molecularly imprinted polymer **20a.** Specifically, a solution of the above-mentioned polymerization recipe excluding L-ascorbic acid was prepared in a Schlenk flask, and the base plate obtained in <3> above was put in to carry out nitrogen substitution, and then L-ascorbic acid was injected with a syringe to initiate polymerization. Polymerization was carried out for 3 hours at 40°C, and then the base plate was washed with pure water.

<5> Removal of the mold (step **(1-4)**)

In accordance with Figure 12-5, the mold was cut out from the molecularly imprinted polymer to obtain an analysis sensor **10a-C'** of a detection subject that is base plate-type.

100mM of EDTA-4Na aqueous solution was dropped to the base plate obtained in <4> above and settled (room temperature, 15 minutes) to remove $Cu^{2+}$. Next, the base plate was washed with pure water and 50 mM of TCEP aqueous solution was dropped and settled (room temperature, 3 hours) to reduce the disulfide bond and achieve conversion to a thiol group (group **23'** for binding a signal substance). Then, the base plate was washed with pure water, immersed in 50 mM of acetate buffer (pH 4.0) containing 0.5 wt% of SDS, and shaken (25°C, overnight) to cut the chelate (coordinate bond) between the NTA-Ni complex on the base plate and the polyhistidine tag on the silica particle, achieve conversion to a Ni-NTA group (group **22'** for binding a specific binding molecule to a target), and remove the silica particle. This causes obtainment of the base material **10a-C'** for making an analysis sensor of a detection subject that is base plate-type.

<6> Introduction of an anti-HER2 affibody and Alexa Fluor 647 (step **(2)** and step **(3)**)

In accordance with Figure **12-6,** Alexa Fluor 647 and an anti-HER2 affibody were introduced to the analysis sensor **10a-C'** of a detection subject that is base plate-type.

<6-1> Reagent

*R43: Alexa Fluor 647 C2-maleimide (Thermo Fisher Scientific)

**Signal group **43:** Alexa Fluor 647
**Binding group **23":** maleimide group

*R42: Z342-EGFP-Histag

**Specific binding group 42: anti-HER2 affibody
**Binding group **22":** polyhistidine tag
**Different signal group **sg:** EGFR

The natural Kd for HER2 of the anti-HER2 affibody in Z342-EGFP-Histag is greater than the natural Kd for HER2 of the anti-HER2 affibody where EGFR is not fused due to the effect of the EGFR being fused (i.e., the binding capacity is inferior to the anti-HER2 affibody where EGFR is not fused) . In fact, the natural Kd for HER2 of the anti-HER2 affibody in Z342-EGFP-Histag was $1.06 \times 10^{-10}$M.

<6-2> Experimental procedure

The base material **10a-C'** for making an analysis sensor of a detection subject that is base plate-type obtained in <5> above was washed with pure water, and 100μM of Alexa Fluor 647 $C_2$ Maleimide (in 20% DMSO aqueous solution) was dropped and settled (room temperature, 1 hour) to utilize the thiol group (group **23'** for binding a signal substance) within the molecular imprint recessed part **21** to introduce a fluorescence group Alexa Fluor 647 (signal group **43**).

After washing the base plate with pure water, PBS (10mM phosphate,140mM NaCl,pH7.4) solution comprising 0.3 μM of Z342-EGFP-Histag was dropped and settled (room temperature, 1 hour) to utilize the nickel-nitrilotriacetic acid-driven group (group **22'** for binding a specific binding molecule) within the molecular imprint recessed part **21** to introduce an anti-HER2 affibody (specific binding group **42**). This causes obtainment of the analysis sensor **$10a_1$-C** of a detection subject that is base plate-type.

<7> Analysis of a detection subject with fluorescence measurement

The analysis sensor **$10a_1$-C** of a detection subject that is base plate-type obtained in <6> above was used to carry out exosome capturing test with a fluorescence microscope with a SIC automatic dispensing apparatus.

<7-1> Experimental procedure

Exosome (exosome secreted by a SK-BR-3 cell) was purified from a SK-BR-3 cell line (high Her2 expression) culture supernatant in the following manner.

A supernatant wherein an SK-BR3 cell was cultured for 3 days at a DMEM culture medium (containing 10% FBS (exosome free), 1% penicillin) was centrifugally separated for 10 minutes at 1500g and for 10 minutes at 3000G, which supernatant underwent ultrafiltration (10 minutes at 500G × 2 times). The exosome solution obtained by the ultrafiltration was purified using an exosome extraction kit qEV (Meiwafosis). The particle size and concentration of exosome were measured using q-Nano.

Exosome secreted by a SK-BR-3 cell was dissolved at the concentration of $3.33 \times 10^{-17}$M, $8.33 \times 10^{-17}$M, $1.67 \times 10^{-16}$M, $3.33 \times 10^{-16}$M, $8.33 \times 10^{-16}$M, $1.67 \times 10^{-15}$M, $3.33 \times 10^{-15}$M, and $1.67 \times 10^{-14}$M in PBS (10mM phosphate, 140mM NaCl, pH7.4) to prepare an exosome solution. The prepared exosome solution was added to the analysis sensor **10a-C** of a detection subject that is base plate-type obtained in <6> above.

Regarding the measurement conditions of the fluorescence microscope, 9 points on the base plate were set as measurement locations, the filter is Cy5, the objective lens is 5-fold, the exposure time is 0.1 second, the power of light is 100%, and the light source is a mercury lamp.

<7-2> Relationship between relative fluorescence intensity and exosome concentration

The relative fluorescence intensity was calculated in accordance with the following formula to study the relationship between relative fluorescence intensity and exosome concentration. The result is shown in Figure **17A.**

[Numeral 1]

$$\Delta I = \frac{I - I_0}{I_0}$$

$\Delta I$ : Relative
$I_0$ : Fluorescence intensity of
$I$ : Fluorescence intensity fluorescence intensity exosome concentration 0 of each concentration

As shown in Figure **17A,** in the analysis sensor **10a$_1$-C** of a detection subject that is base plate-type introduced with Alexa Fluor 647 and anti-HER2 affibody, quenching of about a little less than 2% to a little less than 3% was confirmed at the exosome concentration of 3.33 × $10^{-17}$M to 1.67 × $10^{-14}$M. Meanwhile, as discussed in Comparative Example **1** below, in an analysis sensor of a detection subject for comparison where an anti-HER2 affibody is not introduced, there was no change in the fluorescence intensity in all exosome concentrations. In view of the above, it was shown that the analysis sensor **10a-C** of a detection subject that is base plate-type where Alexa Fluor 647 and anti-HER2 affibody are introduced detected exosome secreted by a SK-BR-3 cell.

<7-3> Derivation of dissociation constant

Dissociation constant $K_d$ was calculated with curve fitting using DeltaGraph based on the fluorescence intensity change. The calculation formula is shown below.

[Numeral 2]

$$Y = \left\{(1 + K \times G + K \times H) - ((1 + K \times G + K \times H)^2 - 4 \times K \times K \times H \times G)^{1/2}\right\} \times \frac{D}{2KG}$$

Fluorescence

Y : intensity change
H : Exosome concentration
G : First concentration of host molecule
**D** : Maximum fluorescence
**K :** Binding constant (provided that it is assumed that G = 0.01) intensity change

As a result, the dissociation constant Kd by the analysis sensor **10a$_1$-C** of a detection subject that is base plate-type introduced with Alexa Fluor 647 of the present Example is 6.1 × $10^{-18}$M, achieving exceptional improvement effect of 8.5 orders with respect to the natural Kd (1.06×$10^{-10}$) of the ant-HER2 affibody used in the present Example, wherein astonishing binding capacity improvement effect compared to Comparison Example 2 discussed below was recognized. In addition, the binding capacity improvement effect by the present Example was significantly excellent compared to Examples 3 and 5 discussed below.

<Comparative Example 1: Analysis sensor of a detection subject that is base plate-type not comprising an affibody>

An analysis sensor of a detection subject for comparison was made in the same manner as Example 1 excluding the point of not introducing an affibody. The relationship between the relative fluorescence intensity and exosome concentration was studied in the same manner as Example 1 regarding this analysis sensor of a detection subject for comparison. The result is shown in Figure **17B.**

As shown in Figure **17B,** there was no change in fluorescence intensity in all exosome concentrations in an analysis sensor of a detection subject for comparison where anti-HER2 affibody is not introduced.

<Example 2: Analysis sensor **10a$_1$-C** of a detection subject that is base plate-type (introducing an anti-HER2 affibody as a specific binding substance and rhodamine as a signal substance)>

In the present Example, an analysis sensor **10a$_1$-C** of a detection subject that is base plate-type (introducing rhodamine as a signal substance) was prepared. In the same manner as Example 1, this analysis sensor of a detection subject that is base plate-type set exosome secreted by an SK-BR-3 cell which is an HER2 overexpress-type breast cancer cell line as the detection subject, set HER2 as the target of the detection subject, and selected and introduced anti-HER2 affibody as a specific binding molecule. In addition, rhodamine was introduced as a signal substance. Furthermore, the obtained analysis sensor of a detection subject that is base plate-type was used to analyze the detection subject.

<1> Experimental procedure

The making was carried out with the same method as Example 1, excluding the point of using Tetramethylrhodamine (TAMRA)-5 C2 maleimide instead of Alexa Fluor 647 C2-maleimide as a signal substance **R43.** In the same manner, a total of three (N = 3) analysis sensors **10a$_1$-C** (introducing rhodamine as a signal substance) of a detection subject that are base plate-type were prepared.

<2> Analysis of a detection subject using fluorescence measurement

Analysis of exosome secreted by a SK-BR-3 cell was carried out with the same method as Example 1 regarding each of the analysis sensors **10a$_1$-C** (introducing rhodamine as a signal substance, N = 3) of a detection subject that are base plate-type obtained in <1> above. Furthermore, the concentrations of the exosome were set as $2.0 \times 10^{-17}$M, $5.0 \times 10^{-17}$M, $1.0 \times 10^{-16}$M, $2.0 \times 10^{-16}$M, $5.0 \times 10^{-16}$M, $1.0 \times 10^{-15}$M, $2.0 \times 10^{-15}$M, and $1.0 \times 10^{-14}$M. Regarding the measurement conditions of the fluorescence microscope, 7 points on the base plate were set as measurement locations, the filter is GW (excitation) - Cy3 (fluorescence), the objective lens is 5-fold, the exposure time is 0.1 second, the power of light is 100%, and the light source is a mercury lamp. The result is shown in Figure **18.**

As a result, in the analysis sensor **10a$_1$-C** of a detection subject that is base plate-type introduced with rhodamine and anti-HER2 affibody, 10-fold or greater quenching was confirmed in all exosome concentrations compared to the analysis sensor **10a$_1$-C** of a detection subject that is base plate-type introduced with Alexa Fluor 647 and anti-HER2 affibody of Example 1, which is recognized as significantly high sensitivity. For example, a little over 30% quenching was observed in the exosome concentration of $1.0 \times 10^{-14}$M. Such sensitivity of the present Example was also excellent compared to Example 3 discussed below. Furthermore, such highly sensitive detection is possible regarding any of N = 3, and it was also understood to be excellent in terms of reproducibility. This excellent reproducibility was also significant compared to Example 1 discussed above and Example 3 discussed below.

<3> Derivation of dissociation constant

The dissociation constant $K_d$ was calculated based on the fluorescence intensity change in the same manner as <7> of Example 1. As a result, the dissociation constant $K_d$ by the analysis sensor **10a$_1$-C** of a detection subject that is base plate-type introduced with rhodamine and anti-HER2 affibody of the present Example is $8.41 \times 10^{-18}$, achieving exceptional improvement effect of a little less than 9 orders of magnitude with respect to the natural Kd ($1.06 \times 10^{-10}$) of the anti-HER2 affibody used in the present Example, wherein astonishing binding capacity improvement effect compared to the Comparison Example 2 discussed below was recognized. In addition, the binding capacity improvement effect by the present Example was also significantly excellent compared to Examples 3 and 5 discussed below.

<4> Derivation of detection limit

The detection limit (DL) was calculated in accordance with the following formula.

[Numeral 3]

$$DL = 3.3s/a$$

s : Standard deviation of fluorescence intensity of exosome concentration 0

a : Inclination by the average fluorescence intensity of concentration 0. $2.0\times10^{-17}$. $5.0\times10^{-17}$ [M]

As a result, the detection limit (DL) by the analysis sensor **$10a_1$-C** of a detection subject that is base plate-type introduced with rhodamine and anti-HER2 affibody of the present Example was $5.19 \times 10^{-17}$M, which was also significantly excellent compared to Example 3 discussed below.

<Comparative Example 2: Analysis sensor of a detection subject that is base plate-type introduced with an antibody>

A sensor for analysis was prepared in the same manner as Example 1 excluding the point of introducing an anti-Her2 antibody (the original dissociation constant with respect to Her2 of this anti-Her2 antibody is $5.8 \times 10^{-10}$M) instead of an anti-HER2 affibody. Specifically, the following procedure was carried out instead of binding Z342-EGFP-Histag to the analysis sensor **10a-C'** of a detection subject that is base plate-type in <6> of Example 1.

100 μM of polyhistidine protein G dissolved in PBS was added to immobilize protein G that can bind an antibody via a polyhistidine tag. 0.3μM of anti-Her2 antibody dissolved in PBS was added to the base plate to immobilize the anti-Her2 antibody via protein G.

Excluding the point above, a sensor for analysis was made in the same manner as Example 1, and the dissociation constant was derived in the same manner as <3> of Example 2. As a result, the dissociation constant $K_d$ by the sensor for analysis of the present Comparative Example is $6.34 \times 10^{-16}$M, which remained an improvement effect of 6 orders with respect to the original Kd ($5.8 \times 10^{-10}$) of the anti-HER2 antibody used in the present Comparative Example.

<Example 3: Analysis sensor of a detection subject that is base plate-type (provided that the sensor is a type of analysis sensor **$10^1$** of a detection subject that is base plate-type that utilizes a signal substance fused to a specific binding group)>

In the present Example, an analysis sensor of a detection subject that is base plate-type (provided that the sensor is a type of analysis sensor **$10^1$** of a detection subject that is base plate-type that utilizes a signal substance fused to a specific binding group) was made. In the same manner as Example 1, this analysis sensor of a detection subject that is base plate-type set exosome secreted by an SK-BR-3 cell which is an HER2 overexpress-type breast cancer cell line as the detection subject, set HER2 as the target of the detection subject, and selected and introduced anti-HER2 affibody as a specific binding molecule. In addition, a signal substance fused to a specific binding group without introducing a signal substance was utilized. Furthermore, analysis of the detection subject was carried out using the obtained analysis sensor of a detection subject that is base plate-type.

<1> Experimental procedure

An analysis sensor of a detection subject that is base plate-type was made in the same manner as Example 3 (N = 2) excluding the step of introducing Tetramethylrhodamine-5 C2 maleimide. In other words, the analysis sensor of the detection subject that is base plate-type of the present Example is not introduced with a signal substance, unlike the analysis sensor **10a-C** of a detection subject that is base plate-type of Example 2. In this regard, since Z342-EGFP-Histag (a molecule having a polyhistidine tag and an anti-HER2 affibody to which a fluorescence group EGFP is fused) is used as the specific binding molecule **R42** that provides the specific binding group **42** as discussed above (the column of Example 1) in this sensor, strictly speaking, the specific binding group **42** is an anti-HER2 affibody to which a fluorescence group EGFP is fused, i.e., the specific binding group **44** wherein a signal group **43** is conjugated with the specific binding group **42.** Therefore, the analysis sensor of a detection subject that is base plate-type of the present Example is a type of analysis sensor **$10^1$** of a detection subject that is base plate-type utilizing a signal substance fused to a specific binding group.

<2> Analysis of a detection subject using fluorescence measurement

Regarding each of the analysis sensors (utilizing EGFP fused to an anti-HER2 affibody) of a detection subject that are base plate-type obtained in <1> above, analysis of exosome secreted by an SK-BR-3 cell was carried out with the same method and condition as Example 2 (provided that the filter was changed to BW (excitation)-Cy3 (fluorescence) to measure EGFP, N=2). The result is shown in Figure **19.**

As a result, in a sensor that is a type of sensor **$10^1$** of a detection subject that is base plate-type utilizing EGFP fused to an anti-HER2 affibody, while the fluctuation range was about 5% to 15% with the quenching at the exosome concentration of $1.0 \times 10^{-14}$M, exosome was detected with high sensitivity.

<3> Derivation of dissociation constant

The dissociation constant $K_d$ was calculated in the same manner as <7> of Example 1 based on the fluorescence intensity change. As a result, the dissociation constant $K_d$ is $2.29 \times 10^{-17}$, which was an exceptional improvement with

respect to the original Kd ($1.06 \times 10^{-10}$) of the anti-HER2 affibody to which a fluorescence group EGFP is fused.

<4> Derivation of detection limit

The detection limit (DL) was calculated in the same manner as Example 2. As a result, the detection limit (DL) was $3.70 \times 10^{-14}$M.

<Example 4: Analysis sensor **10-PC** of a detection subject that is particle-like and immobilized on a base plate (introducing an anti-HER2 affibody as a specific binding substance)>

In the present Example, an analysis sensor **10-PC** of a detection subject that is particle-like and immobilized on a base plate was prepared. This analysis sensor of a detection subject that is particle-like and immobilized on a base plate set exosome secreted by an SK-BR-3 cell which is an HER2 overexpress-type breast cancer cell line as the detection subject, set HER2 as the target of the detection subject, and selected and introduced an anti-HER2 affibody as a specific binding molecule. Furthermore, analysis of the detection subject was carried out using the obtained analysis sensor **10-PC** of a detection subject that is particle-like and immobilized on a base plate.

Regarding a method of making an analysis sensor of a detection subject that is particle-like and immobilized on a base plate, a base plate **10-PC'** for making an analysis sensor of a detection subject that is base plate-type was made in accordance with Figure **13-1b,** Figure **13-2,** Figure **13-3,** and Figure **15,** and the analysis sensor **10-PC** of a detection subject was made in accordance with Figure **13-4.**

<1> Synthesis of a disulfide bond-type methacryloyl modification reagent

In accordance with the following scheme, a disulfide bond-type methacryloyl modification reagent (Compound **(5)** of the following scheme) used in <2> discussed below was synthesized. In the present Example, this reagent was used as a monomer **M22$_2$** comprising a binding group **BG22'** and a first reversible linking group **22** polymerizable functional group **27.**

[Chemical 20]

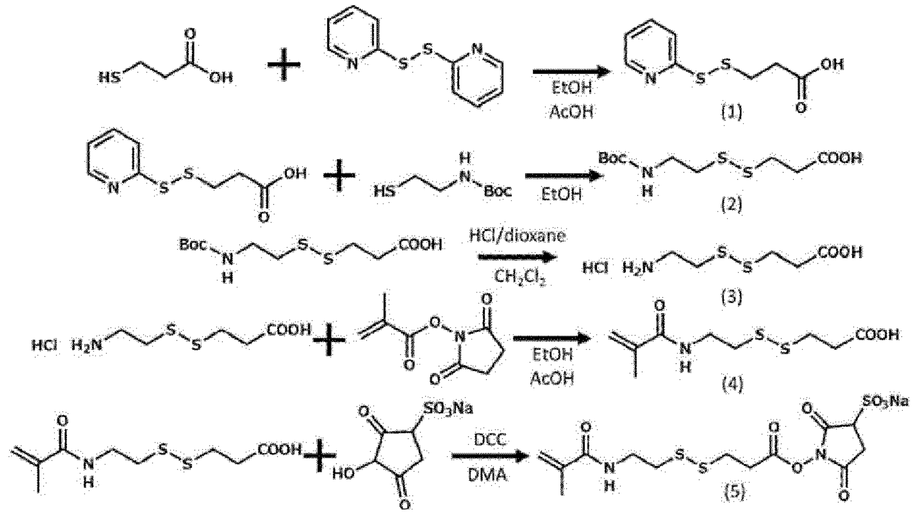


3-mercaptopropionic acid (0.900g ,8.50 mmol) was dissolved in 20 mL of ethanol to add 1.6 mL of acetate. 2,2-dipyridyl disulfide (3.75g ,17 mmol) dissolved in 30ml of ethanol was dropped thereto while stirring at room temperature. Reaction was carried out for 3 hours at 25°C to obtain Compound **(1).**

The purified Compound **(1)** (1.43 g,3.97 mmol) was dissolved in ethanol 15 mL, and then 2-(Boc-amino) ethanethiol (1.69 mL,9.99mmol, 2eq) dissolved in 20 mL of ethanol was added. Reaction was carried out for 3 hours at room temperature to obtain Compound **(2).**

4N HCl dioxane (6.60mL, 25.5 mmol, 5 eq) dissolved in 10 mL of $CH_2Cl_2$ was dropped to the purified compound **(2)** (1.43g, 5.10mmol) dissolved in 5 mL of $CH_2Cl_2$ while being stirred under ice cooling. Then, reaction was carried out overnight at room temperature to obtain Compound **(3).**

The purified Compound **(3)** (531.6mg, 2.45mmol) was dispersed in $CH_2Cl_2$ 5 mL to add DIEA (1.28mL, 7.35mmol, 3 eq) . 3.5mL of $CH_2Cl_2$ that dissolved N-succinimidyl methacrylate (670 mg, 3.68mmol, 1.5eq) was dropped

thereto in a nitrogen atmosphere to be reacted overnight at room temperature to obtain Compound **(4).**

The purified Compound **(4)** (290mg,1.16mmol, 1.1eq), sulfo-NHS (230mg, 1.1mmol), and DCC (304.5mg, 1.54mmol, 1.4 eq) were dissolved in DMA 5 mL to be reacted for 24 hours at room temperature. After the reaction, the solution was cooled at 4°C and precipitate was removed by filtration. Then, vacuum distillation of the filtrate was carried out to remove DMA. The obtained white-colored individual was dissolved in AcOEt, to which Hexane was added to cause another precipitation to obtain Compound **(5).** ($^1$H-NMR chart 7 (500MHz,$CD_3OD$):δ=8.1 (t,1H, -CO-NH-C-), 5.7-5.3 (s, 2H, CH3-C=$CH_2$), 4.0-3.8 (d,1H ,-CO-CH-$SO_3$Na) , 3.4-3.3 (m, 2H,$NaSO_3$-CH-$CH_2$-), 3.3-2.7 (m, 8H, -NH-$CH_2$-$CH_2$-S-S-$CH_2$-$CH_2$-) , 1.85 (s, 3H, $CH_3$-C=$CH_2$)

<2> Modification of a mold (step **(1-11)**)

In accordance with Figure **13-1b,** a mold HSA modified with a methacryloyl group via a disulfide bond was made.

<2-1> Reagent

*Albumin, from Human Serum (HSA; human serum albumin) (WAKO JUNYAKU KOGYO)

**Mold **60:** HSA
**Binding group **BG22":** amino group

*M22$_2$: Compound **(5)** synthesized in <1> above

**Binding group **BG22':** carboxylic acid-active ester group
**First reversible linking group **22:** disulfide group
**Polymerizable functional group **27:** methacryloyl group

<2-2> Experimental procedure

2 mL of 10 mM phosphate buffer solution (pH 7.4) that dissolved HSA (100mg, 2.23 μmol) and 1 mL of 10 mM phosphate buffer solution (pH 7.4) that dissolved Compound **(5)** synthesized in <1> above (20 mg, 44.6 pmol, 20eq) were mixed to be reacted overnight at 4°C. Then, washing was carried out 3 times with Amicon Ultra-4 10 kDa (4°C, 7500 G, 20 min) to obtain a mold HSA modified with a methacryloyl group via a disulfide bond.

<3> Synthesis of a molecularly imprinted polymer (step **(1-12)**)

In accordance with Figure **13-2,** a particle of a molecularly imprinted polymer was synthesized.

<3-1> Reagent and polymerization recipe

*M28: 2-methacryloyloxyethyl phosphorylcholine (MPC)
*M29: N-isopropylacrylamide (NIPAm)

[Table 5]

| Reagent | Amount of use |
|---|---|
| Methacryloyl group modification HSA | 18.3 mg (0.30 μmol) |
| N-Isopropylacrylamide (NIPAm) | 610 mg (5.40 mmol) |
| 2-Methacryloyloxyethyl Phosphorylcholine (MPC) | 88.5 mg (0.30 mmol) |
| Pyrrolidyl acrylate (PyA) | 63.6 mg (0.45 mmol) |
| N,N'-Methylenebisacrylamide(MBAA) | 46.2 mg (0.30 mmol) |

(continued)

| Reagent | Amount of use |
|---|---|
| 2-2'-Azobis(2-methyl propionamide) dihydrochloride (V-50) | 316 mg (1.20 mmol) |
| PBS (10 mM phosphate, 140 mM NaCl, pH7.4) | 50 mL |

<3-2> Experimental procedure

In accordance with the polymerization recipe of Table **5,** emulsifier-free precipitation polymerization was carried out for 16 hours at 70°C in a nitrogen atmosphere in a 50 mL Schlenk flask to synthesize particle MIP-NGs of a molecularly imprinted polymer **20.**

<4> Removal of mold (step **(1-13)**)

In accordance with Figure **13-3,** the mold was removed from the molecularly imprinted polymer.

Regarding a PBS liquid comprising a composite of the particle MIP-NGs of the molecularly imprinted polymer 20 and the mold HSA, pure water substitution of a solvent was carried out by ultrafiltration (25°C, 7500g, 20 minutes, 3 times) using a dialysis tube of Amicon Ultra-4 10 kDa. Tris(2-carboxyethyl)phosphine Hydrochloride (TCEP) was added to this composite so as to be 20 mM to be reacted overnight at 25°C. This causes the disulfide bond between the mold HSA and MIP-NGs to be reduced and cleaved, exposing the thiol group (group **22'** for binding a specific binding molecule to a target).

Furthermore, screening was carried out using a chromatography filling agent Sephadex G-100 for size exclusion chromatography, removal of TCEP was carried out by ultrafiltration (25°C, 7500 g, 20 min, 3 times) using a dialysis tube of Amicon Ultra-4 10 kDa, and solvent substitution to 140 mM NaCl 10 mM tris-hydrochloric acid buffer pH 7.4 was carried out. Furthermore, the mold HSA was removed by anion exchange chromatography. This caused obtainment of the particle MIP-NGs of the molecularly imprinted polymer **20.**

<5> Particle analysis

Regarding the obtained MIP-NGs, dynamic light scattering (DLS) measurement and transmission electron microscope (TEM) measurement were carried out. In the TEM measurement, the average value of the long axis and the short axis of the particle was set as a particle size in the transmission electron microscopic image, and said particle sizes of 100 randomly selected particles were measured to use the average value thereof as the number for average particle size. The result is shown in the table below. In addition, the TEM image is shown in Figure **20.** In Figure **20,** the length of the scale bar is 50 nm.

[Table 6]

| DLS | | | | TEM |
|---|---|---|---|---|
| Z average particle size | Volume average particle size | PDI | Solid component concentration | Numher average particle size |
| **128.6 nm** | **156.4 nm** | **0.293** | **0.04** | **50-80nm** |

<6> Immobilization of a particle to a base plate

In accordance with Figure **15** (provided that the molecularly imprinted polymer **20** is replaced with the molecularly imprinted polymer **20**), the particle MIP-NGs of the molecularly imprinted polymer **20** obtained in <4> above was immobilized on the base plate.

After washing the SPR gold base plate with pure water and ethanol, the SPR gold base plate was immersed in an ethanol solution prepared so that 11-Mercaptoundecanoic acid would be 1 mM to be incubated overnight at 25°C. After washing the base plate after the reaction with ethanol and water and drying the base plate with nitrogen gas, Biacore3000 was used to carry out injection of a PBS solution of 0.1 M N-Hydroxysuccinimide(NHS), 0.4 M 1-(3-dimethylaminopropyl)-3-ethylcarbodiimide hydrochloride (EDC) for incubation for 10 minutes at 25°C. The obtained base plate (wherein the base plate surface is modified with a carboxylic acid-active ester group as the binding group **BG9''**) was injected with 400 ug/mL of MIP-NGs solution to be incubated for 10 minutes at 25°C to obtain a base plate wherein MIP-NGs is fixed. This causes obtainment of the base material **10-PC'** for making a sensor for analyzing a detection subject.

<7> Introduction of affibody (step **(2)**)

In accordance with Figure **13-4,** an anti-Her2 affibody was introduced to the base material **10-PC'** for making a sensor for analyzing a detection subject obtained in <6> above.

<7-1> Reagent

*Anti-Her2 Affibody(R) Molecule (anti-Her2 affibody molecule, Abcam; the natural Kd of this molecule is $3.2 \times 10^{-11}$M, and the molecular weight is about 6,000)
*Dithiothreitol

**Specific binding group **42:** anti-Her2 affibody
**Binding group **22":** thiol group

<7-2> Experimental procedure

10 μL of a 1 mg/mL solution of anti-Her2 affibody molecule (10 mM PB, 137 mM NaCl, 0.05vol% Tween20 (pH 7.4); PBST) was added to 40 μL of 20 mM of DTT solution (50 mM PB, 137 mM NaCl (pH7.5)), reacted for 2 hours at 25°C, then Amicon Ultra-0.5 (MWCO: 10 kDa) was used to carry out ultrafiltration (4°C, 14000 × g, 15 minutes) 3 times to carry out purification at PBST. This causes obtainment of a molecule **R42** that provides anti-Her2 affibody.

The base material **10-PC'** for making a sensor for analyzing a detection subject obtained in <6> above was immersed in 3.1 mM of 2,2-dipyridyl disulphide solution (ethanol:water = 1:1 (volume ratio)) for incubation for 3 hours at 25°C to activate the thiol group (group **22'** for binding a specific binding molecule to a target) within the molecular imprint recessed part **21** into a pyridyl disulfide group (group **22'** for binding a specific binding molecule to a target). Then, the pyridyl disulfide group was injected with 10 μg/mL of solution of the molecule **R42** that provides an anti-Her2 affibody for 7 minutes at the flowrate of 20 μL/min to bind the anti-Her2 affibody group (specific binding group **42**). Then, 1 mg/mL skim milk aqueous solution and 1 M aminoethanol (pH8.4) were reacted in order, to carry out blocking treatment of the base plate surface (binding group **BG9"** that was not used to fix a particle). This caused obtainment of the sensor **10-PC** for analyzing a detection subject.

<8> Analysis of a detection subject by SPR measurement

SPR measurement was carried out using the sensor **10-PC** for analyzing a detection subject that is particle-like and immobilized at a base plate obtained in <7> above.

<8-1> Experimental procedure

Exosome secreted by an SK-BR-3 cell was dissolved in PBS (10 mM phosphate, 140mM NaCl, pH7.4) at the concentration of $0 \times 10^{-16}$M, $0.032 \times 10^{-16}$M, $0.064 \times 10^{-16}$M, $0.125 \times 10^{-16}$M, $0.25 \times 10^{-16}$M, $0.5 \times 10^{-16}$M, $1.0 \times 10^{-16}$M, $2.5 \times 10^{-16}$M, $5.0 \times 10^{-16}$M, $10 \times 10^{-16}$M, $50 \times 10^{-16}$M, $100 \times 10^{-16}$m, $500 \times 10^{-16}$M, $1000 \times 10^{-16}$M to prepare an exosome solution. The prepared exosome solution was added to the sensor **10-PC** for analyzing a detection subject that is particle-like and immobilized on a base plate obtained in <7> above.

The measurement conditions of SPR are as shown below.

Flowrate: 20 μL/min
Injection volume: 100 μL
Temperature: 25°C
Data point: the point of 6 minutes after injection
Running buffer: PBS

<8-2> Result

The difference in the resonance unit (RU) value between right before the injection of the exosome solution and at the point of 6 minutes after the injection was used as the amount of change in response to create an adsorption isotherm. The result thereof is shown in Figure **21.** Furthermore, the dissociation constant $K_d$ was calculated by curve fitting in the same manner as <7> of Example 1. As a result, the dissociation constant $K_d$ is $2.0 \times 10^{-18}$(M), which was

an exceptional improvement with respect to the natural Kd ($3.2 \times 10^{-11}$M) of the anti-HER2 affibody used in the present Example.

<Example 5: Sensor **IOb-PC** of a detection subject that is particle-like and immobilized on a base plate (introducing anti-HER2 affibody as a specific binding substance and ATTO647N as a signal substance)>

In the present Example, a sensor **10b-PC** of a detection subject that is particle-like and immobilized on a base plate was made. This analysis sensor of a detection subject that is particle-like and immobilized on a base plate set exosome secreted by an SK-BR-3 cell which is an HER2 overexpress-type breast cancer cell line as the detection subject, set HER2 as the target of the detection subject, and selected and introduced anti-HER2 affibody as a specific binding molecule. In addition, ATTO647N was introduced as a signal substance. Furthermore, analysis of the detection subject was carried out using the obtained analysis sensor **IOb-PC** of a detection subject that is particle-like and immobilized on a base plate.

Regarding the analysis sensor **IOb-PC** of a detection subject that is particle-like and immobilized on a base plate, since the analysis sensor **10c-P** of a detection subject that is particle-like is immobilized on the base plate, the sensor is an embodiment wherein the interaction group **25** is excluded. Therefore, regarding a method of making the analysis sensor **IOb-PC** of a detection subject that is particle-like and immobilized on a base plate, a base plate **10b-PC'** for making an analysis sensor of a detection subject that is particle-like was made in accordance with Figure **14-1,** Figure **14-2** (provided that a monomer **M25** having the interaction group 25 is not used), Figure **14-3,** Figure **15,** and Figure **14-4,** and the analysis sensor **IOb-PC** of a detection subject was made in accordance with Figure **14-5.**

<1> Modification of mold (step **(1-21)**)

In accordance with Figure **14-1,** a mold HSA modified with a methacryloyl group via a disulfide bond was made.

<1-1> Reagent

*Albumin, from Human Serum (HSA; human serum albumin) (WAKO JUNYAKU KOGYO)

**Mold **60c:** HSA
**Binding group **BG6'':** amino group

*M27: Compound **(5)** synthesized in <1> of Example 4

**Binding group **BG6':** carboxylic acid-active ester group
**Third reversible linking group **26b:** disulfide group
**Polymerizable functional group **27:** methacryloyl group

<1-2> Experimental procedure

2 mL of 10 mM phosphate buffer solution (pH 7.4) wherein HSA (100mg, 2.23 μmol) is dissolved and 1 mL of phosphate buffer solution (pH 7.4) wherein Compound **(5)** synthesized in <1> of Example 4 (20 mg, 44.6 μmol, 20eq) is dissolved were mixed to be reacted overnight at 4°C. Then, washing was carried out with Amicon Ultra-4 10 kDa (4°C, 7500 G, 20 min) 3 times to obtain a mold HSA modified with a methacryloyl group via a disulfide bond.

<2> Synthesis of a molecularly imprinted polymer (step **(1-22)**)

In accordance with Figure **14-2** (provided that a monomer **M25** having the interaction group **25** was not used), a particle of a molecularly imprinted polymer was synthesized.

Specifically, emulsifier-free precipitation polymerization was carried out for 16 hours at 70°C in a nitrogen atmosphere in a 50mL Schlenk flask in accordance with the same polymerization recipe as Table **5** of Example 4 to synthesize particle MIP-NGs of a molecularly imprinted polymer **20b** (in the form of not having the interaction group **25** of the molecularly imprinted polymer **20c**).

<3> Removal of mold (step **(1-23)**)

In accordance with Figure **14-3** (provided that the interaction group **25** is absent), a mold was removed from a molecularly imprinted polymer.

Regarding a PBS liquid comprising a composite of a particle MIP-NGs of the molecularly imprinted polymer **20b** (in the form of not having the interaction group **25** of the molecularly imprinted polymer **20c**) and the mold HSA, pure water substitution of a solvent was carried out by ultrafiltration (25°C, 7500g, 20 minutes, 3 times) using a dialysis tube of Amicon Ultra-4 (10 kDa). Tris(2-carboxyethyl)phosphine Hydrochloride (TCEP) was added to this composite so as to become 20 mM to be reacted overnight at 25°C. This caused the disulfide bond between the mold HSA and MIP-NGs to be reduced and cut to expose the thiol group (binding group **26b'**).

Furthermore, screening was carried out using a chromatography filling agent Sephadex G-100 for size exclusion chromatography, removal of TCEP was carried out by ultrafiltration (25°C, 7500 g, 20 min, 3 times) using a dialysis tube of Amicon Ultra-4 10 kDa, and solvent substitution to 140 mM NaCl, 10 mM tris-hydrochloric acid buffer pH 7.4 was carried out. Furthermore, the mold HSA was removed by anion exchange chromatography. This caused obtainment of the particle MIP-NGs of the molecularly imprinted polymer **20** (in the form of not having the interaction group **25** of the molecularly imprinted polymer **20c**) .

<4> Fixing of a particle to a base plate

In accordance with Figure **15** (provided that the particle-like molecularly imprinted polymer **20c** is replaced with the particle-like molecularly imprinted polymer **20b**), the particle MIP-NGs of the molecularly imprinted polymer **20b** (in the form of not having the interaction group **25** of the molecularly imprinted polymer **20c**) obtained in <3> above was immobilized on the base plate. The specific Experimental procedure is the same as <6> of Example 4.

<5> Synthesis of a functional molecule **R24**

The following scheme was used to synthesize a functional molecule **R24** having a functional group **24b** and a group 22b' for binding a specific binding molecule to a target, which is used in <6> discussed below. The functional molecule **R24** is a compound represented by Formula **(7a)** above.

[Chemical 21]

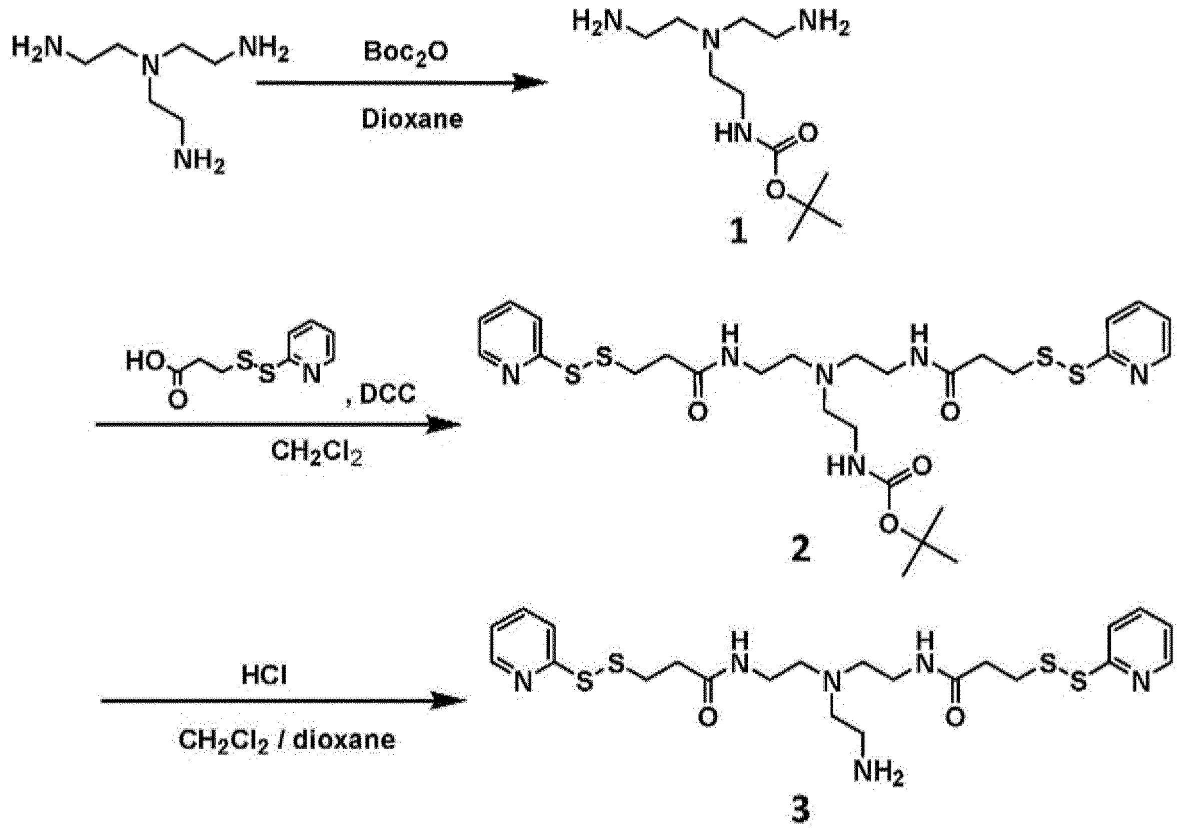


Tris(2-aminoethyl)amine 1800 μL (12 mmol) was dissolved in Dioxane (4 mL) to be stirred under ice cooling. dioxane (10 mL), where $Boc_2O$ 440 mg (2. 0 mmol) was dissolved, was slowly dropped thereto to be further stirred overnight (0°C → room temperature). Vacuum distillation of the solvent after the reaction was carried out then, the residue was dissolve with Milli-Q water to carry out extraction with $CH_2Cl_2$ (15 mL) (×4). The organic layer was dried with $Na_2SO_4$ , then the solvent was removed by using a rotary evaporator, and vacuum drying was carried out to obtain a clear oil-like targeted object **(1).** (yield: 364 mg, 74 %) $^1$H-NMR (300 MHz, $CDCl_3$) δ = 5.29 (br, 1H, carbamide), 3.18 (t, 2H, $-CH_2$-NH-Boc), 2.75 (t, 4H, $H_2N-CH_2$-), 2.53 (m, 6H, $-CH_2$-), 1.45 (s, 9H, Boc)

(3-Pyridyldithio)propionic acid 323 mg (1.5 mmol) and DCC 330 mg (1.6 mmol) were dissolved in $CH_2Cl_2$ (10 mL) to be stirred under ice cooling. Compound (1) 130 mg (0.53 mmol) was added to be further stirred for 1 hour. After the reaction, the precipitate was removed by filtration, and the solvent underwent vacuum distillation with a rotary evaporator. The residue was purified with silica gel chromatography (EA/Hx=45/55 , 100/0, EA/MeOH=99/1 → 80/20) to obtain Compound **(2).** (yield: 230 mg , 68 %)$^1$H-NMR (300 MHz, $CDCl_3$) δ= 8.44 (d, 2H, pyridyl), 7.70-7.60 (m, 4H, pyridyl), 7.18 (br, 2H, amide), 7.12-7.07 (m, 2H, pyridyl), 5.01 (br, 1H, carbamide), 3.34-3.29 (m, 4H, $H_2N-CH_2$-), 3.14-3.07 (m, 6H, $-CH_2$-NH-Boc, $-CH_2$-), 2.68 (t, 4H, $-CH_2$-), 2.61-2.51 (m, 6H, $-CH_2$-), 1.43 (s, 9H, Boc)

Compound **(2)** 230 mg (0.36 mmol) was dissolved in $CH_2Cl_2$ (5 mL), to which 4 N HCl in dioxane 0.5 mL was added to be stirred under ice cooling. After being stirred overnight (0°C → room temperature), an excessive amount of diethyl ether is added to separate precipitate with decantation. The obtained solid was washed with diethyl ether, which underwent vacuum drying to obtain a targeted object (Compound **(3)**). (yield: 255 mg, quant.) $^1$H-NMR (300 MHz, $D_2O$) δ= 8.51 (d, 2H, pyridyl), 8.14(t, 2H, pyridyl), 8.01 (d, 2H, pyridyl), 7.56 (m, 2H, pyridyl), 3.67-3.3.56 (m, 6H, HN-$CH_2$-), 3.43 (t, 6H, $-CH_2$-N), 3.09 (t, 4H, $-CH_2$-), 2.74 (t, 4H, $-CH_2$-)

<6> Introduction of a function group **24b** (step **(1-24)**)

In accordance with Figure **14-4** (provided that the interaction group **25** is absent), the functional molecule **R24** was introduced to the binding group **26b''** of the molecular imprint recessed part **21c** (provided that the interaction group **25** is absent).

<6-1> Reagent

*Functional molecule **R24:** the compound represented by Formula **(7a)** above obtained in <5> above.

**Group **22b'** for binding a specific binding molecule: pyridyl disulfide group
**Group **23b'** for binding a signal substance: amino group
**Binding group **26b'':** pyridyl disulfide group

<6-2> Experimental procedure

The base plate wherein particle MIP-NGs of the molecularly imprinted polymer 20b (in the form of not having the interaction group **25** of the molecularly imprinted polymer **20c**) is immobilized that is obtained in <4> above was immersed in a 900 ug/mL PBS solution of the functional molecule **R24** to react the pyridyl disulfide group (binding group **26b''**) of the binding molecule **R24** and the thiol group (binding group **26b'**) of the molecularly imprinted polymer **20b** for 2 hours at room temperature °C. This caused obtainment of the base material **10b-PC'** for making a sensor for analyzing a detection subject.

<7> Introduction of an affibody and a fluorescence substance (step **(2)** and step **(3)**)

In accordance with Figure **14-5** (provided that the interaction group **25** is absent), an anti-Her2 affibody and a fluorescence substance were introduced to the base material **10b-PC'** for making a sensor for analyzing a detection subject.

<7-1> Reagent

*Molecule **R42** that provides an anti-Her2 affibody: the compound synthesized in <7-2> of Example 4 using Anti-Her2 Affibody(R) Molecule (anti-Her2 affibody, Abcam) and dithiothreitol

**Specific binding group **42:** anti-Her2 affibody (the natural Kd of this molecule is $3.2 \times 10^{-11}$M, and the molecular weight is about 6,000)

**Binding group **22b"**: thiol group

*Signal substance **R43:** Atto647N-NHS (ATTO-TEC)

**Signal group **43:** Atto647N
**Binding group **23b"**: carboxylic acid-active ester group

<7-2> Experimental procedure

The 10 μg/mL PBS solution of the molecule **R42** that provides the anti-Her2 affibody was reacted with the base material **10b-PC'** for making a sensor for analyzing a detection subject obtained in <7> above (room temperature, 2 hours) to introduce the anti-Her2 affibody (specific binding group **42**) to the pyridyl disulfide group (group **22b'** for binding a specific binding molecule). Then, 100 μM of 2-mercaptoethanol solution was reacted to carry out capping of the remaining pyridyl disulfide group (remaining group of the group **22b'** for binding a specific binding molecule). Next, the base plate was reacted with an Atto647N-NHS solution (room temperature, 2 hours) to introduce Atto647N (signal group **43**) on the amino group (group **23b'** for binding a signal substance). This caused obtainment of the sensor **10b-PC** for analyzing a detection subject (wherein the sensor **10c-P** for analyzing a detection subject with no interaction group **25** is immobilized on a base plate).

<8> Analysis of a detection subject by fluorescence measurement

Exosome capturing test using a fluorescence microscope with a SIC automatic dispensing apparatus was carried out using the sensor **10b-PC** for analyzing a detection subject obtained in <7> above (wherein the sensor **10c-**

**P** for analyzing a detection subject with no interaction group **25** is immobilized on a base plate).

<8-1> Experimental procedure

Exosome secreted by an SK-BR-3 cell was dissolved in PBS (10mM phosphate, 140mM NaCl, pH7.4) at the concentration of 0 to 1.0 $\times$ $10^{-13}$M to prepare an exosome solution. The prepared exosome solution was dropped to the sensor for analyzing a detection subject obtained in <7> above.

Regarding the measurement conditions of the fluorescence microscope, the measurement location is point 3 on the base plate, the filter is Cy5, the objective lens is 5-fold, the exposure time is 0.1 second, the power of light is 100%, and the light source is a mercury lamp.

<8-2> Result

In the same manner as Example 1, the relative fluorescence intensity was calculated to study the relationship between the relative fluorescence intensity and exosome concentration. The result is shown in Figure **22.**

As shown in Figure **22,** in the sensor **10b-PC** for analyzing a detection subject obtained in <7> above (wherein the sensor **10c-P** for analyzing a detection subject with no interaction group **25** is immobilized on a base plate), it has been confirmed that the degree of quenching increases in an exosome concentration-dependent manner. In other words, the recognition capacity with respect to an exosome secreted by an SK-BR-3 cell was shown.

The dissociation constant $K_d$ was calculated by curve fitting in the same manner as <7> of Example 1. As a result, the dissociation constant $K_d$ was 1.0 $\times$ $10^{-17}$ (M), which was exceptional improvement with respect to the natural Kd (3.2 $\times$ $10^{-11}$M) of the anti-HER2 affibody molecule used in the present Example. In addition, detection limit was derived in the same manner as <4> of Example 2. As a result, the detection limit was 5.93 $\times$ $10^{-18}$(M) .

Another sensor **10b-PC** for analyzing a detection subject (wherein the sensor **10c-P** for analyzing a detection subject with no interaction group **25** is immobilized on a base plate) was obtained in the same manner as described above excluding the point of using an anti-HSA affibody instead of the anti-HER2 affibody. Figure **23** shows the relative fluorescence intensity change of when exosome (5 $\times$ $10^{-17}$M) secreted by an SK-BR-3 cell which is an HER2 overexpress-type breast cancer cell line was added to the sensor for analysis of the present Example, wherein the anti-HER2 affibody was introduced and a different sensor for analysis where the anti-HSA affibody was introduced.

As shown in Figure **23,** the exosome secreted by an SK-BR-3 cell was specifically detected by the sensor for analysis of the present Example where the anti-HER2 affibody was introduced.

<Example 6: Analysis sensor **10a-C** of a detection subject that is base plate-type (introducing anti-HER2 nanobody as a specific binding substance and Alexa Fluor 647 as a signal substance)>

An analysis sensor **10a-C** of a detection subject that is base plate-type (introducing Alexa Fluor 647 as a signal substance) was made in the same manner as Example 1 excluding the point of introducing an anti-Her2 nanobody instead of the anti-HER2 affibody. The original Kd of the anti-Her2 nanobody molecule is 1 $\times$ $10^{-9}$M.

The dissociation constant $K_d$ was calculated by curve fitting in the same manner as <7> of Example 1. As a result, the dissociation constant $K_d$ is 5.81 $\times$ $10^{-19}$(M), which was exceptional improvement with respect to the natural Kd (1 $\times$ $10^{-9}$M) of the anti-HER2 nanobody molecule used in the present Example.

<Example 7: Analysis sensor **10c-P** of a detection subject that is particle-like and immobilized on a base plate (introducing anti-HER2 affibody as a specific binding substance and ATTO647N as a signal substance)>

In the present Example, an analysis sensor **10c-P** of a detection subject that is particle-like in the form of being immobilized on a base plate was made. This analysis sensor of a detection subject that is particle-like and immobilized on a base plate set exosome secreted in an SK-BR-3 cell which is an HER2 overexpress-type breast cancer cell line as a detection subject, set HER2 as a target of the detection subject, and selected and introduced an anti-HER2 affibody as a specific binding molecule. In addition, ATTO647N was introduced as a signal substance. In addition, this analysis sensor has an interaction group **25** that interacts with HSA which is a mold. Furthermore, analysis of the detection subject was carried out using the obtained analysis sensor **10c-P** of a detection subject that is particle-like and immobilized on a base plate.

Regarding a method of making an analysis sensor **10c-P** of a detection subject that is particle-like and immobilized on a base plate, a base plate **10c-P'** for making an analysis sensor of a detection subject that is particle-like in the form of being immobilized on a base plate was made in accordance with Figure **14-1,** Figure **14-2,** Figure **14-3,** Figure **15** and Figure **14-4,** and furthermore, an analysis sensor **10c-P** of a detection subject that is particle-like and immobilized on a base plate was made in accordance with Figure **14-5.**

<1> Modification of mold (step **(1-21)**)

In accordance with Figure **14-1,** a mold HSA modified with a methacryloyl group via a disulfide bond was made.

<1-1> Reagent

*Albumin, from Human Serum (HSA; human serum albumin) (WAKO JUNYAKU KOGYO)

**Mold **60c:** HSA
**Binding group **BG6":** amino group
**Interaction site **65c:** carboxyl group

*M27: Compound **(5)** synthesized in <1> of Example 4

**Binding group **BG6':** carboxylic acid-active ester group
**Third reversible linking group **26b:** disulfide group
**Polymerizable functional group **27:** methacryloyl group

<1-2> Experimental procedure

The same procedure as <1-2> of Example 5 was carried out.

<2> Synthesis of a molecularly imprinted polymer (step **(1-22)**)

In accordance with Figure **14-2,** a particle of a molecularly imprinted polymer was synthesized.

<2-1> Reagent and polymerization recipe

*M25: pyrrolidyl acrylate (PyA)
**Interaction group **25:** pyrrolidine group
*M28: 2-methacryloyloxyethyl phosphorylcholine (MPC)
*M29: N-isopropylacrylamide (NIPAm)

[Table 7]

| Reagent | Amount of use |
|---|---|
| Methacryloyl group modification HSA | 18.3 mg (0.30 μmol) |
| N-Isopropylacrylamide (NIPAm) | 610 mg (5.40 mmol) |
| 2-Methacryloyloxyethyl Phosphorylcholine (MPC) | 88.5 mg (0.30 mmol) |
| Pyrrolidyl acrylate (PyA) | 63.6 mg (0.45 mmol) |
| N,N'-Methylenebisacrylamide (MBAA) | 46.2 mg (0.30 mmol) |
| 2-2'-Azobis(2-methyl propionamide) dihydrochloride (V-50) | 316 mg (1.20 mmol) |
| PBS (10 mM phosphate,140 mM NaCl, pH7.4) | 50 mL |

<2-2> Experimental procedure

Emulsifier-freer precipitation polymerization was carried out for 16 hours at 70°C in a nitrogen atmosphere in a 50mL Schlenk flask in accordance with the polymerization recipe of Table **7** to synthesize particle MIP-NGs of a molecularly imprinted polymer **20c.**

<3> Removal of mold (step **(1-23)**)

In accordance with Figure **14-3,** the mold was removed from the molecularly imprinted polymer **20c.** The specific procedure is the same as <3> of Example 5.

<4> Particle analysis

Regarding the obtained particle MIP-NGs of the molecularly imprinted polymer **20c,** dynamic light scattering (DLS) measurement and transmission electron microscope (TEM) measurement were carried out in the same manner as <5> of Example 4. The measurement results are shown in the table below. In addition, the TEM image is shown in Figure **24.** In Figure **24,** the length of the scale bar is 50 nm.

[Table 8]

| DLS | | | | TEM |
|---|---|---|---|---|
| Z average particle size | Volume average particle size | PDI | Solid component concentration | Number average particle size |
| 152.5 nm | 27.96 nm | 0.31 | 0.04 | 20-30nm |

<5> Fixing of a particle to a base plate

In accordance with Figure **15,** the particle MIP-NGs of the molecularly imprinted polymer **20c** obtained in <3> above was immobilized on the base plate. The specific Experimental procedure is the same as <6> of Example 4 and <4> of Example 5. This caused obtainment of a base plate **10c-P'** for making an analysis sensor of a detection subject that is particle-like and immobilized on the base plate.

<6> Introduction of the functional group 24b (step **(1-24)**)

In accordance with Figure **14-4,** the functional molecule **R24** was introduced to the binding group **26b"** of the molecular imprint recessed part **21c.** The specific Experimental procedure is the same as <6> of Example 5.

<7> Introduction of an affibody and a fluorescence substance (step **(2)** and step **(3)**)

In accordance with Figure **14-5,** an anti-Her2 affibody and a fluorescence substance (Atto647N-NHS) were introduced to the base material **10c-P'** for making a sensor for analyzing a detection subject that is immobilized on the base plate. The specific Experimental procedure is the same as <7> of Example 5. This caused obtainment of an analysis sensor **10c-P** of a detection subject that is particle-like and immobilized on the base plate.

<8> Analysis of a detection substance by fluorescence measurement

Exosome capturing test using a fluorescence microscope with a SIC automatic dispensing apparatus was carried out using the sensor **10c-P** for analyzing a detection subject that is immobilized on a base plate obtained in <7> above.

<8-1> Experimental procedure

Sample liquids (a) to (d) comprising exosome secreted by an SK-BR-3 cell and HSA at the concentrations shown in the Table below were prepared.

[Table 9]

| | SK-BR-3 cell-driven exosome final concentration (M) | HSA final concentration (M) |
|---|---|---|
| (a) | $1.93 \times 10^{-16}$ | 0 |
| (b) | $1.93 \times 10^{-16}$ | $0.72 \times 10^{-4}$ |
| (c) | $1.93 \times 10^{-16}$ | $1.50 \times 10^{-4}$ |

(continued)

| | SK-BR-3 cell-driven exosome final concentration (M) | HSA final concentration (M) |
|---|---|---|
| (d) | $1.93 \times 10^{-16}$ | $7.52 \times 10^{-4}$ |

The prepared sample liquids were added to the analysis sensor **10c-P** of a detection subject that is particle-like and immobilized on a base plate obtained in <7> above.

Regarding the measurement conditions of the fluorescence microscope, per each sample, the measurement location is point 3 on the base plate, the filter is Cy5, the objective lens is 5-fold, the exposure time is 0.1 second, the power of light is 100%, and the light source is a mercury lamp.

<8-2> Result

The relative fluorescence intensity was calculated in the same manner as Example 1. The result is shown in Figure **25.** As shown in Figure **25,** regardless of the point that sample liquids (a) to (d) all comprise the same concentration of detection subject (exosome secreted by an SK-BR-3 cell), while no quenching was confirmed when there is a certain degree of concentration of HSA like sample liquid (d), quenching was confirmed when there is zero or low HSA concentration like sample liquids (a) to (c). The result is recognized as achieving the double targeting that while HSA is captured by the interaction group **25** and the molecular imprint recessed part **21c** which uses HSA as a mold, whereby an anti-Her2 affibody (specific binding group **42**) is shielded, as shown in Figure **16-1** in sample liquid (d) wherein there is a certain degree of concentration of HSA, HSA is not captured and anti-Her2 affibody (specific binding group **42**) is exposed to capture the exosome secreted by an SK-BR-3 cell which is a detection subject as shown in Figure **16-2** in sample liquids (a) to (c) with zero or low HSA concentration.

Table **10** shows the summary of the sensors for analysis of a detection subject made in Examples 1 to 7 and Comparative Example 2 above.

[Table 10]

| | Example 1 | Example 2 | Comparative Example 2 | Example 3 | Example 4 | Example 5 | Example 6 | Example 7 |
|---|---|---|---|---|---|---|---|---|
| Sensor type | $10a_1$-C | $10a_1$-C | Antibody substitution type of Examples 1 and 2 | $10_1$ | 10-PC | 10b-PC | 10a-C | 10c-P |
| MIP polymer shape | Film | Film | Film | Film | Particle | Particle | Film | Particle |
| MIP polymer particle size (nm) | - | - | - | - | Volume average :156.4<br>Z average :128.6<br>Number average :50-80 | | - | Volume average :27.96<br>Z average :152.5<br>Number average :20-30 |
| Sensor shape | Base plate | Base plate | Base plate | Base plate | Base plate | Base plate | Base plate | Particle |
| Mold | Silica particle | Silica particle | Silica particle | Silica particle | HSA | HSA | Silica particle | HSA |
| Mold size (nm) | 200 | 200 | 200 | 200 | - | - | 200 | - |
| Mold molecular weight | - | - | - | - | 66500 | 66500 | - | 66500 |
| Signal group 43 | Alexa Flour 647 | Rhodamine | Alexa Flour 647 | Fusion EGFP | - | ATTO647N | Alexa Flour 647 | ATTO647N |
| Specific binding group 42 | Anti-Her 2 affibody | Anti-Her 2 affibody | Anti-Her 2 affibody | Anti-Her 2 affibody | Anti-Her 2 affibody | Anti-Her 2 affibody | Anti-Her2 nanobody | Anti-Her 2 affibody |
| Different signal group sg | Fusion EGFP | Fusion EGFP | - | - | - | - | - | - |
| Specific binding group 42 molecular weight | 6,000 | 6,000 | 150,000 | 6,000 | 6,000 | 6,000 | 15,000 | 6,000 |
| Specific binding group natural dissociation constant (M) | $1.06\times10^{-10}$ | $1.06\times10^{-10}$ | $5.8\times10^{-10}$ | $1.06\times10^{-10}$ | $3.2\times10^{-11}$ | $3.2\times10^{-11}$ | $1\times10^{-9}$ | - |
| Dissociation constant by sensor (M) | **$6.1\times10^{-18}$** | **$8.41\times10^{-18}$** | **$6.34\times10^{-16}$** | **$2.29\times10^{-17}$** | **$2.0\times10^{-18}$** | **$1.0\times10^{-17}$** | **$5.81\times10^{-19}$** | |

<Example 8: Immune response using a particle (nonimmobilized) for sensing for PSA>

In the present example, a particle of which sensing subject is set as porcine serum albumin (PSA) was used in a free state of not being immobilized on a base plate, wherein immune response to each of PSA and transferrin was tested.

The particle with PSA as a sensing subject (fluorescence group introduction MIP-NGs; Example 4) was made with the same method as the "Synthesis of a nanoparticle for sensing-3" and "synthesis of a nanoparticle for sensing-4" of Example 7.

80 ug/mL of fluorescence group introduced MIP-NGs was added with a protein solution (in 10mM phosphate buffer liquid (pH7.4) comprising 140mM NaCl) so that the final concentration of the protein (PSA or transferrin) would be 5, 10, 50, 100, 500, 1000 ug/mL (final solution volume: 500 μL), and incubated for 15 minutes at 25°C. Then, the fluorescence spectrum of a solution was measured with a fluorescence spectrophotometer (F-2500, Hitachi High-Tech) (excitation wavelength λex: 647 nm, λem: 658 to 690nm). The result thereof is shown in Figure **16.** In addition, the relative fluorescence intensity of the fluorescence wavelength 557 nm with respect to the protein concentration of each of PSA and transferrin (PSA: 5, 10, 50, 100, 500, 1000 μg/mL, transferrin: 10, 50, 100, 500, 1000 μg/mL) of PSA and transferrin was plotted. The result thereof is shown in Figure **17.**

As shown in Figure **16,** when a PSA solution is added to the fluorescence group introduction MIP-NGs of Example 4 in a free state, increase in fluorescence intensity was observed as the concentration increased. Since the same phenomenon is confirmed (Example 7) in the case of fixing the fluorescence group introduction MIP-NGs of Example 4 to a base plate, the increase in fluorescence intensity shown in Figure **16** was suggested to be fluorescence intensity change associated with the PSA binding to the fluorescence group introduction MIP-NGs of Example 4. In addition, as shown in Figure **17,** greater fluorescence intensity change was shown in the case of adding PSA compared to the fluorescence change of the case of adding transferrin which is a reference protein. This showed that the fluorescence group introduction MIP-NGs of Example 4 can selectively adsorb PSA and transmit the binding information as a fluorescence signal even in a free state of not being immobilized on a base plate.

<Example 9: Synthesis of a nanoparticle for sensing-5>

In the present Example, a nanoparticle for Fc domain sensing (and for IgG sensing via Fc domain capturing), which corresponds to the nanoparticle **10a** for sensing of Figure **2,** was synthesized.

9-1. Synthesis of a functional monomer

The functional monomer shown in Formula **51b** was designed and synthesized in accordance with the following scheme. Furthermore, the functional monomer shown in Formula **51b** has a phenylboronic acid group and a secondary amino group, wherein the bornyl aryl group interacts with a sugar chain of an Fc domain of IgG and the secondary amino group is used for introduction of a fluorescence molecule in post-imprinting modification in MIP-NGs.

[Chemical 22]

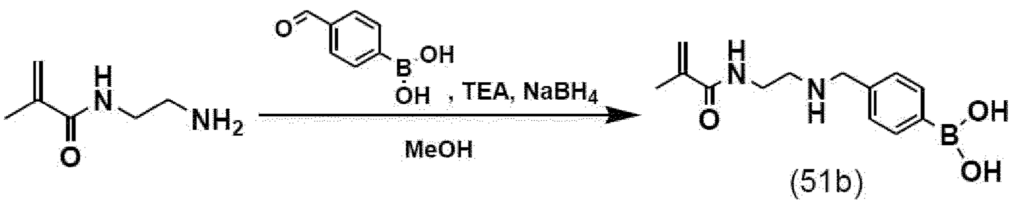


(51b)

Ethylenediaminemonomethacrylamide hydrochloride (ca.1.57 mmol) and 4-formylphenylboronic acid (215 mg, 1.5 mmol) and triethylamine (210 μL, 1.5 mmol) were dissolved in methanol (10 mL) to be stirred at room temperature (0.5 h). Reaction tracking was carried out at TLC (MeOH , UV@254 nm and ninhydrin), and appearance of a spot (Rf: 0.55, purple) dyed by ninhydrin and UV absorption was confirmed, from which it was judged that a Schiff base is formed, to which $NaBH_4$ (120 mg, 9.0 mmol) was added. After the addition, stirring was further carried out overnight (8h) at room temperature. Since the targeted object spot (Rf: 0.25, yellow) was confirmed at TLC (MeOH → UV@254 nm and ninhydrin), the reaction was terminated and the solvent underwent vacuum distillation with a rotary evaporator. The residue was purified using an automated column chromatography system (silica: universal premium, MeOH/DCM=70/30 , 100/0, GR) to obtain a targeted object.

Yield: 175 mg (0.668 mmol, 42.5 %)
$^1$H-NMR (500 MHz, $D_2O$): δ=7.62 (d, 2H, phenyl), 7.30 (d, 2H, phenyl), 5.69 (s, 1H, vinyl), 5.44 (s, 1H, vinyl), 3.97 (s, 2H, N-$CH_2$-), 3.47 (t, 2H, -$CH_2$-), 2.99 (br, 2H, -$CH_2$-), 1.89 (s, 3H, -Me)

9-2. Preparation of an Fc domain

10mg of IgG was dissolved in 10 mM phosphate buffer saline (pH7.4, 140 mM NaCl) (5 mL) to prepare an antibody solution.0.02M L-cysteine, 0.002M EDTA, and 0.1mg/mL papain were dissolved in 10 mM phosphate buffer saline (pH7.4, 140 mM NaCl) to prepare an enzyme solution. The same volume of antibody solution and enzyme solution are mixed to be reacted for 24 hours at 37°C. Then, 10 kDa of ultrafiltration film (7500 g, 20 minutes × 3) was used to the reaction liquid to substitute the solvent with 20 mM phosphate buffer (pH7.0). Next, 100 kDa of ultrafiltration film (Amicon Ultra) (7500 g, 20 min × 3) was used to separate an unreacted IgG. The obtained solution (1mL) was purified using a Protein A column (Hitrap™ Protein A HP, Cytiva) .20 mM phosphate buffer (pH7.0) was used as a binding buffer and citrate buffer (pH 3.0) was used as an elution buffer to carry out purification at a syringe (flow rate: about 1 mL/min). Regarding the solution after the purification, 10 kDa of ultrafiltration film (7500 g, 20 minutes ×3) was used to substituted the solvent with 10 mM carbonate buffer liquid (pH 9.2). The purification of the Fc domain was confirmed with SDS-PAGE.

9-3. Synthesis of a nanoparticle for sensing-5

The mixture (pre-polymerization solution) of the components shown in the table below was exposed to emulsifier-free precipitation polymerization for 12 hours at 50°C to synthesize a polymer nanogel (Step **1**). In order to render the particle purification operation easy, labeling monomer was also used as a monomer to be comprised in the pre-polymerization solution. The solution (2 mL) after the polymerization was substituted with 10 mM phosphate buffer liquid (pH7.4, 140 mM NaCl) at an ultrafiltration film (10 kDa, 7500 × g, 20 minutes × 3). The obtained solution (2 mL) went through size exclusion chromatography (Sephadex G-100) to remove an unreacted monomer. The solution (1 mL) obtained thereby and 40mg/mL SDS aqueous solution (1 mL) were mixed, incubated for 5 minutes, then added to an anion exchange resin (DEAE-Sephadex, 10 cm, 1.5 cm i.d.) to elute 10mM TrisHCl buffer (pH7.4,140 mM NaCl) as an eluate (Step **2**). Then, purification was carried out by going through a desalting column (eluate: 10 mM phosphate buffer (pH7.4, 140 mM NaCl)) in order to remove SDS.

[Table 11]

| | | Example 5 **MIP-NGs** | Comparative Example 2 **NIP-NGs** |
|---|---|---|---|
| Mold protein | Fc domain (IgG fragment) | 2.50 mg (0.050 μmol) | - |
| N-substituted or non-substituted (meth)acrylamide | *N*-Isopropylacrylamide (NIPAm) | 102 mg (0.90 mmol) | |
| Biocompatible monomer | 2-Metacryloyloxyethyl phosphorylcholine (MPC) | 3.69 mg (0.0125 mmol) | |
| Crosslinking agent | *N,N'*-Methylenbisacrylamide (MBAA) | 7.71 mg (0.050 mmol) | |
| Labeling monomer | Methacryloxyethyl Thiocarbamoyl Rhodamine B (MTRB) | 0.42 mg (0.625 μmol) | |
| Functional momoner | Functional monomer shown in Fomrula (51b) | 15.73 mg (0.060 mmol) | |
| Initiating agent | 2-2'-Azobis(2-methyl propion amide) dihydrochloride (V-50) | 54.2 mg (0.20 mmol) | |
| Solvent | 10 mM Carbonate buffer 2% DMSO (pH 9.2) | 25 mL | |

Regarding the obtained MIP-NGs (Example 5) and NIP-NGs (Comparative Example 4), when the particle size was measured in the same manner as Example 1, existence of a nanometerorder particle was confirmed. Specifically, with Z average particle size, the particle size was 21 nm in MIP-NGs (Example 5) and the particle size was 18 nm in

NIP-NGs (Comparative Example 4). The Z potential was shown to be positively charged at 19 mV in MIP-NGs (Example 5) and 2.9 mV in NIP-NGs (Comparative Example 4). These charges are considered to be derived from the secondary amino group (and polymerization initiating agent) of the functional monomer. In view of the above, it is suggested that a particle incorporated with a functional monomer has been synthesized. In addition, in view of the result of the fluorescence measurement ($\lambda_{ex}$ = 280nm) of a particle solution before and after the purification, since there was loss of the fluorescence derived from tryptophan comprised in the Fc region near 340 nm where it was recognized before the purification, it was confirmed that washing and removal of most Fc domain fragments were able to be carried out.

Furthermore, 5 μL of ATTO647N NHS (10 mg/mL) DMSO solution was added to MIP-NGs (0.5 mg/mL, 1 mL) after the purification to be incubated for 2 hours at 25°C (Step **3**). Ultrafiltration (10 kDa, 7500 × g, 20 minutes × 3) of the solution after the reaction removed the unreacted fluorescence molecule to obtain the fluorescence group introduction MIP-NGs (Example 5). Furthermore, fluorescence introduction was confirmed based on the point that there was observation of a fluorescence peak near 670 nm which was not found before the introduction based on the fluorescence measurement (excitation wavelength: 647 nm) before and after the fluorescence molecule (ATTO647N) introduction operation. The same operation was carried out to the NIP-NGs to obtain the fluorescence group introduction NIP-NGs (Comparative Example 4).

9-4. Base plate for sensing-4

The gold sputter glass base plate (4.3 × 9.8 mm) was washed with pure water and EtOH, and UV-O3 washing (20 minutes) was carried out. The base plate after the washing was immersed in an 1mM 11-mercaptoundecanoic acid EtOH solution to introduce carboxyl group to the surface utilizing self-assembled monolayer formation (25°C, 24 hour). After washing the base plate with EtOH, the base plate was immersed in an aqueous solution wherein 0.2M EDC and 0.05M NHS were dissolved to carry out activation of the surface carboxyl group (25°C, 1 hour). The solution (in PBS, 0.5 mg/mL) of 50pL of fluorescence group introduction MIP-NGs (Example 5) or fluorescence group introduction NIP-NGs (Comparative Example 4) was added onto the base plate after the reaction to fix the nanoparticle by amine linking (25°C, 1 hour). Then, 1 M aminoethanol aqueous solution (50 μL) was dropped to the unreacted active ester to carry out inactivation (25°C, 0.5 hour). Finally, Protein free blocking buffer(50μL) was dropped to carry out blocking of the surface (25°C, 0.5 hour). Furthermore, the florescence intensity at the surface before and after immobilization was measured, wherein since there had been observation of increase in the fluorescence intensity at the base plate after fixing, fixing of a particle was confirmed.

9-5. Fluorescence measurement

Regarding the fluorescence detection capacity of a protein of the base plate for sensing that had been made, examination was carried out using a fluorescence microscope with an automatic liquid handling robot. The measurement sequence conditions are as shown below.

Fluorescence microscope

Filter: Cy5
Object lens: ×5
Exposure time: 0.1 second
Power of light: 12%
Light source: mercury lamp
ROI: base plate central part 3 × 5 = 15 locations

Sequence of automatic dispensation apparatus

1. Chip attachment
2. Sample suction 150 μL
3. Reaction 5 minutes (25°C)
4. Measurement position keep Repeat 2 to 4

Figure **18** shows the relative fluorescence intensity change of when an Fc domain fragment (0-1600nM) which is a mold protein was added to the base plate for sensing that had been made. Since great fluorescence change was shown at the base plate wherein MIP-NGs (Example 5) is immobilized compared to the base plate wherein NIP-NGs (Comparative Example 4) is immobilized, it is suggested that a binding space of the Fc domain fragment was formed by molecular imprinting in MIP-NGs (Example 5), and a functional monomer was incorporated into said space and

fluorescence labeling was carried out by post-imprinting modification, which enabled fluorescence signal conversion of the binding of the Fc domain fragment. In addition, the apparent binding constant ($K_d$) was calculated as $9.8 \times 10^{-9}$M from the obtained fluorescence change rate.

The Fc domain fragment and the whole IgG and Lysozyme (Lyz) as reference proteins were used to test the protein selectivity of the base plate for sensing that had been made. Figure **19** shows the fluorescence response upon adding each reference protein (100 nM) with the relative fluorescence intensity change upon adding the Fc domain fragment (100 nM) as 1. MIP-NGs (Example 5) showed the greatest fluorescence change upon adding the Fc domain fragment. In addition, since about 70% of the whole IgG having the Fc domain fragment as the partial structure thereof is also responding, it is suggested that the Fc site of the IgG can be recognized. In other words, it is shown that the MIP-NGs (Example 5) that had been made while using the Fc domain fragment as a mold can also detect IgG via capturing of the Fc domain. Meanwhile, whole IgG and Lyz both showed greater fluorescence response than the Fc domain fragment in the NIP-NGs (Comparative Example 4). It is presumed that this is a non-specific bond with a functional monomer residue that is randomly present on the NIP-NGs (Comparative Example 4) surface and the fluorescence molecule introduced thereto. The pI of the whole IgG is up to 8.5, and Lyz is 11.2, which are positively charged in a neutral solution. In view of the above, it is considered that the electrostatic interaction with the phenylboronic acid group of the functional monomer, hydrogen binding with an amide binding site, and the like contribute to the binding.

While the preferable embodiments of the present disclosure are as discussed above, the present disclosure is not limited thereto, and other various embodiments that do not deviate from the gist of the present disclosure are carried out. The present application claims priority over Japanese Patent Application No. 2020-159008 filed on September 23, 2020 in Japan, and PCTJP2021/007726 filed on March 1, 2021. The entire content thereof is incorporated herein by reference.

[Reference Signs List]

Base material for making a sensor for analysis ... 10', 10a', 10b', and 10c'
Base material for king a sensor for analysis (base plate-type) ... 10-C', and 10a-C'
Base material for making a sensor for analysis (particle-like) ... 10-P', 10a-P', and 10c-P'
Base material for making a sensor for analysis (particlefixed base plate-type) ... 10-PC', and 10b-PC'
Sensor for analysis ... 10, $10_1$, 10a, 10b, and 10c
Sensor for analysis (base plate-type) ... 10-C, 10a-C, and $10a_1$-C
Sensor for analysis (particle-like) ... 10-P, 10a-P, and 10c-P
Sensor for analysis (particle-immobilized base plate-type) ... 10-PC, and lOb-PC
Molecularly imprinted polymer ... 20, 20a, 20b, and 20c
Molecular imprint recessed part ... 21, and 21c
Group for binding a specific binding molecule to a target ... 22', and 22b' (thiol, disulfide)
Binding group ... 22'' (disulfide)
Monomer comprising a group for binding a specific binding molecule and a polymerizable functional group ... M22
First reversible linking group ... 22
Group for binding a signal substance ... 23', and 23b'
Second reversible linking group ... 23
Polymerizable monomer (monomer providing a group for binding a signal monomer) ... $M23_1$, and $M23_2$
Functional group ... 24b
Functional molecule having a functional group and a group for binding a specific binding molecule to a target ... R24
Interaction group (for a mold of said molecular imprint recessed part of detection subject) ... 25
Monomer comprising an interaction group for a mold ... M25 Binding group ... 26b''
Third reversible linking group 26b
Polymerizable functional group ... 27
Polymerizable monomer ... $M22_1$
Polymerizable monomer (e.g., Compound (5)) ... $M22_2$
Polymerizable monomer (e.g., biocompatible monomer) ... M28
Polymerizable monomer (e.g., acrylamide monomer) ... M29
Base plate ... 30
Binding group (on a base plate) ... BG1'
Specific binding group (for a target) ... 42
Specific binding molecule (for a target) ... R42
Different signal group ... sg

Specific binding group having a different signal group ... $42_1$
Signal group ... 43
Signal substance ... R43
Specific binding group having a signal group ... 44
Mold ... 60, $60_1$, $60_2$, and 60c
Interaction site (on a mold) ... 65c
Binding group (on a mold) ... BG6", BG9"BG22", and BG23"
Binding group (corresponding to BG6", BG9", BG22", and BG23'') ... BG6', BG9'BG22', and BG23'
Detection subject ... 70
Target ... 72
INI ... Polymerizable initiating group
SAM ... Monolayer

## Claims

1. A base material for use in making a sensor for use in analyzing a detection subject, comprising a molecularly imprinted polymer having:

   a molecular imprint recessed part that receives a target of a detection subject; and
   a group for use in binding a binding molecule to the target provided within the molecular imprint recessed part,
   wherein the group for use in binding a binding molecule is a group adapted to a small substance.

2. A base material for use in making a sensor for use in analyzing a detection subject, comprising a molecularly imprinted polymer having:

   a molecular imprint recessed part that receives a target of a detection subject; and
   a group for use in binding a binding molecule to the target provided within the molecular imprint recessed part,
   wherein the molecular imprint recessed part has a shape or configuration adapted to a small substance.

3. The base material for use in making a sensor for use in analyzing a detection subject of claim 1 or 2, further having a group for use in binding a signal substance within the molecular imprint recessed part.

4. The base material for use in making a sensor for use in analyzing a detection subject of claim 3, wherein the group for use in binding a binding molecule and the group for use in binding a signal substance are independent from one another in the molecular imprint recessed part surface.

5. The base material for use in making a sensor for use in analyzing a detection subject of claim 3, wherein the group for use in binding a binding molecule and the group for use in binding a signal substance configure the functional group shown in Formula (1) below within the molecular imprint recessed part.

   [Chemical 23] $R^2$-$L^1$-$R^1$ (1)

   <$R^1$ represents the group for use in binding a binding molecule, $L^1$ represents a direct binding or linking group, and $R^2$ represents a linking group comprising the group for use in binding a signal substance.>

6. The base material for use in making a sensor for use in analyzing a detection subject of claim 5, wherein a linking group comprising the group for use in binding a signal substance is represented by Formulas (2) and (3) below.

   [Chemical 24] -$NR^3$- (2)

[Chemical 25]

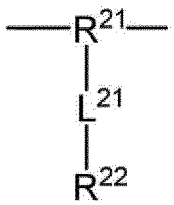

(3)

<$R^3$ represents an alkyl group with a carbon number of 1 to 8 that may comprise hydrogen or a polyalkylene glycol chain, $R^{21}$ represents a nitrogen atom or hydrocarbon, $L^{21}$ represents a direct binding or linking group, and $R^{22}$ represents the group for use in binding a signal substance.>

**7.** The base material for use in making a sensor for use in analyzing a detection subject of any of claims 1 to 6, wherein the group for use in binding a binding molecule is a group selected from the group consisting of an amino group, a carboxyl group, a boronic acid group, a cis-diol group, an aldehyde group, a ketone group, an oxyamino group, a biotin (biocytin, desthiobiotin) group, a nickel-nitrilotriacetic acid-driven group, a thiol group, and a pyridyl disulfide group.

**8.** The base material for use in making a sensor for use in analyzing a detection subject of any of claims 1 to 7, wherein the group for use in binding a binding molecule is a group selected from the group consisting of a nickel-nitrilotriacetic acid-driven group, a thiol group, and a pyridyl disulfide group.

**9.** The base material for use in making a sensor for use in analyzing a detection subject of any of claims 1 to 8, wherein the group for use in binding a binding molecule is adapted to a small substance via an Fc domain binding protein of an antibody, avidin, streptavidin, NeutrAvidin, a polymer comprising one or more of the binding group of claim 7, dendrimer, or oligoDNA.

**10.** The base material for use in making a sensor for use in analyzing a detection subject of any of claims 1 to 9, wherein the Fc domain binding protein of an antibody is protein A, protein G, protein L, or protein A/G.

**11.** The base material for use in making a sensor for use in analyzing a detection subject of any of claims 1 to 10, having an interaction group for a mold of the molecular imprint recessed part or the detection subject within the molecular imprint recessed part.

**12.** The base material for use in making a sensor for use in analyzing a detection subject of any of claims 1 to 11, wherein the binding molecule is a specific binding molecule.

**13.** The base material for use in making a sensor for use in analyzing a detection subject of any of claims 1 to 12, wherein the specific binding molecule is selected from the group consisting of an antibody mimetic, a phospholipid binding protein, an artificial (polymer) receptor, and lectin.

**14.** The base material for use in making a sensor for use in analyzing a detection subject of any of claims 1 to 13, wherein the detection subject is a microparticle having a film structure.

**15.** The base material for use in making a sensor for use in analyzing a detection subject of any of claims 1 to 14, wherein a mold of the molecular imprint recessed part is a particle core or a biomolecule.

**16.** The base material for use in making a sensor for use in analyzing a detection subject of any of claims 1 to 15, wherein a mold of the molecular imprint recessed part is a silica particle or serum protein.

**17.** The base material for use in making a sensor for use in analyzing a detection subject of any of claims 1 to 16, wherein a mold of the molecular imprint recessed part is a serum protein, the detection subject is a microparticle having a film structure, and the target is a molecule expressed on a surface of the microparticle.

**18.** The base material for use in making a sensor for use in analyzing a detection subject of any of claims 1 to 17, comprising a base plate and a polymer film provided on a surface of the base plate, wherein the imprint polymer

configures the polymer.

**19.** The base material for use in making a sensor for use in analyzing a detection subject of any of claims 1 to 17, wherein the imprint polymer is particle-like.

**20.** The base plate for use in making a sensor for use in analyzing a detection subject of claim 19, wherein the imprint polymer that is particle-like is immobilized on a base plate.

**21.** A sensor for use in analyzing a detection subject, comprising:

the base material for use in making a sensor for use in analyzing a detection subject of any one of claims 1 to 20; and
a specific binding group for a target of the detection subject bound to the group for use in binding a specific binding molecule,
wherein the specific binding molecule is a small substance group.

**22.** The sensor for use in analyzing a detection subject of claim 21, further comprising a signal group bound to the group for use in binding a signal substance.

**23.** An analysis method of a detection subject, comprising:

the step of contacting a sample comprising a detection subject to the sensor for use in analyzing a detection subject of claim 21 or 22 and binding the detection subject to the specific binding group; and
the step of detecting a bong between the specific binding group and the detection subject.

**24.** A method of manufacturing a base material for use in making a sensor for use in analyzing a detection subject, comprising:

the monolayer formation step **(1-1)** of forming a monolayer having a group for use in binding a binding molecule to a target of a detection subject and a polymerization initiating group on a surface at a base plate;
the step **(1-2)** of introducing a mold to the group for use in binding a binding molecule via a first reversible linking group;
the step **(1-3)** of adding a polymerizable monomer, using the polymerizable monomer as a substrate, using the polymerization initiating group as a polymerizable initiating agent, and synthesizing a molecularly imprinted polymer for a part of a surface of the mold to form a polymer film on the base plate surface; and
the step **(1-4)** of cleaving the first reversible linking group for conversion to the group for use in binding a binding molecule as well as removal of the mold,
wherein the group for use in binding a binding molecule is a group adapted to a small substance.

**25.** A method of manufacturing a base material for use in making a sensor for use in analyzing a detection subject, comprising:

the monolayer formation step **(1-1)** of forming a monolayer having a group for use in binding a binding molecule to a target of a detection subject and a polymerization initiating group on a surface at a base plate;
the step **(1-2)** of introducing a mold to the group for use in binding a binding molecule via a first reversible linking group;
the step **(1-3)** of adding a polymerizable monomer, using the polymerizable monomer as a substrate, using the polymerization initiating group as a polymerizable initiating agent, and synthesizing a molecularly imprinted polymer for a part of a surface of the mold to form a polymer film on the base plate surface; and
the step **(1-4)** of cleaving the first reversible linking group for conversion to the group for use in binding a binding molecule as well as removal of the mold,
wherein the molecular imprint recessed part has a shape or configuration adapted to a small substance.

**26.** A method of manufacturing the base material for use in making a sensor for use in analyzing a detection subject of claim 16, comprising:

the modification step of modifying the surface of the mold via a polymerizable functional group via a second reversible linking group,

wherein the polymerizable monomer and the polymerizable functional group are used as a substrate and the polymerization initiating group is used as a polymerizable initiating agent to synthesize a molecularly imprinted polymer for a part of the surface of the mold in the step **(1-3)**, and
wherein the first reversible linking group and the second reversible linking group are cleaved for conversion to the group for use in binding a binding molecule and a group for use in binding a signal substance, respectively, as well as removal of the mold in the step **(1-4)**.

**27.** A method of manufacturing a base material for use in making a sensor for use in analyzing a detection subject, comprising:

the step **(1-11)** of preparing a mold of a molecular imprint wherein a surface is modified with a polymerizable functional group via a first reversible linking group;
the step **(1-12)** of using the polymerizable functional group as a substrate and synthesizing a molecularly imprinted polymer for a part of the surface of the mold; and
the step **(1-13)** of cleaving the first reversible linking group and removing the mold from the molecularly imprinted polymer to generate the group for use in binding a binding molecule,
wherein the group for use in binding a binding molecule is adapted to a small substance.

**28.** A method of manufacturing a base material for use in making a sensor for use in analyzing a detection subject, comprising:

the step **(1-11)** of preparing a mold of a molecular imprint wherein a surface is modified with a polymerizable functional group via a first reversible linking group;
the step **(1-12)** of using the polymerizable functional group as a substrate and synthesizing a molecularly imprinted polymer for a part of the surface of the mold; and
the step **(1-13)** of cleaving the first reversible linking group and removing the mold from the molecularly imprinted polymer to generate the group for use in binding a binding molecule,
wherein the molecular imprint recessed part has a shape or configuration adapted to a small substance.

**29.** A method of manufacturing a base material for use in making a sensor for use in analyzing a detection subject, comprising:

the step **(1-21)** of preparing a mold of a molecular imprint wherein a surface is modified with a polymerizable functional group via a third reversible linking group;
the step **(1-22)** of using the polymerizable functional group as a substrate and synthesizing a molecularly imprinted polymer for a part of the surface of the mold;
the step **(1-23)** of cleaving the third reversible linking group and removing the mold from the molecularly imprinted polymer; and
the step **(1-24)** of introducing a molecule having functional group comprising a group for use in binding a binding molecule for a target of a detection subject and a group for use in binding a signal substance via third reversible linking group,
wherein the group for use in binding a binding molecule is a group adapted to a small substance.

**30.** A method of manufacturing a base material for use in making a sensor for use in analyzing a detection subject, comprising:

the step **(1-21)** of preparing a mold of a molecular imprint wherein a surface is modified with a polymerizable functional group via a third reversible linking group;
the step **(1-22)** of using the polymerizable functional group as a substrate and synthesizing a molecularly imprinted polymer for a part of the surface of the mold;
the step **(1-23)** of cleaving the third reversible linking group and removing the mold from the molecularly imprinted polymer; and
the step **(1-24)** of introducing a molecule having functional group comprising a group for use in binding a binding molecule for a target of a detection subject and a group for use in binding a signal substance via third reversible linking group,
wherein the molecular imprint recessed part has a shape or configuration adapted to a small substance.

**31.** The method of manufacturing a base material for use in making a sensor for use in analyzing a detection subject

of claim 29 or 30, further using a monomer comprising an interaction group for the mold as the substrate in the step **(1-22)**.

**32.** A method of manufacturing the base material for use in making a sensor for use in analyzing a detection subject of any of claims 17 to 21, wherein the binding molecule is a specific binding molecule.

**33.** A method of manufacturing a sensor for use in analyzing a detection subject, comprising:

the step **(1)** of carrying out the method of manufacturing a base material for use in making a sensor for use in analyzing a detection subject of any of claims 24 to 32; and
the step **(2)** of binding the binding molecule to the group for use in binding a binding molecule.

**34.** A method of manufacturing a sensor for use in analyzing a detection subject, comprising:

the step **(1)** of carrying out the method of manufacturing a base material for use in making a sensor for use in analyzing a detection subject of any of claims 26, 29 and 31;
the step **(2)** of binding the binding molecule to the group for use in binding a binding molecule; and
the step **(3)** of binding a signal substance to the group for use in binding a signal substance.

[Figure 1A]

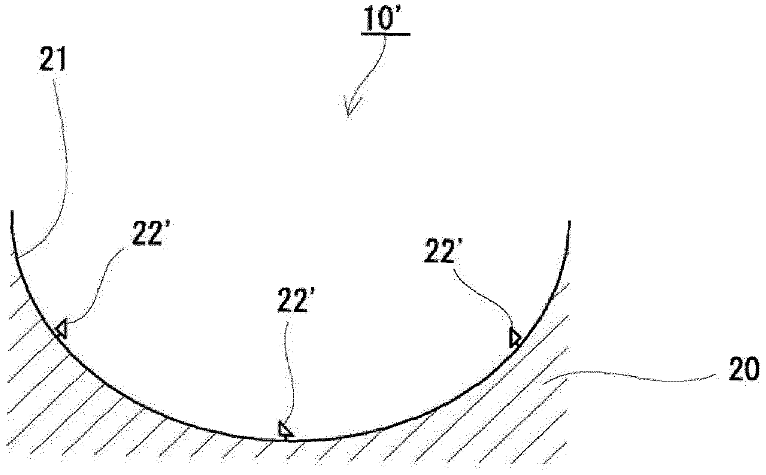


[Figure 1B-1]

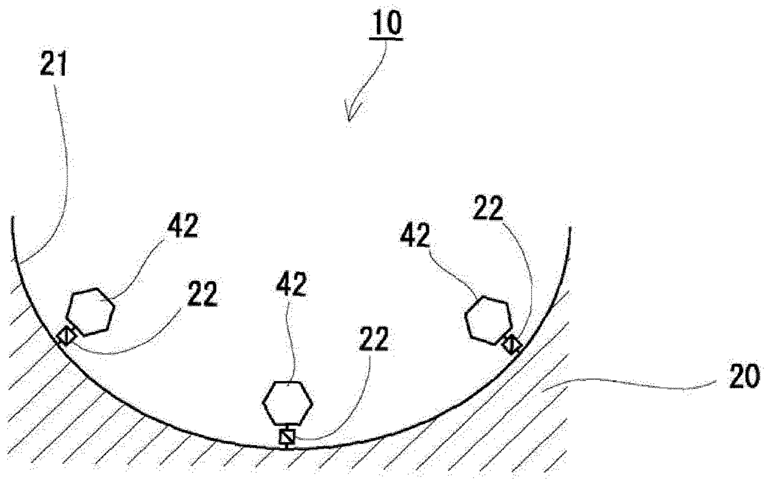


[Figure 1B-2]

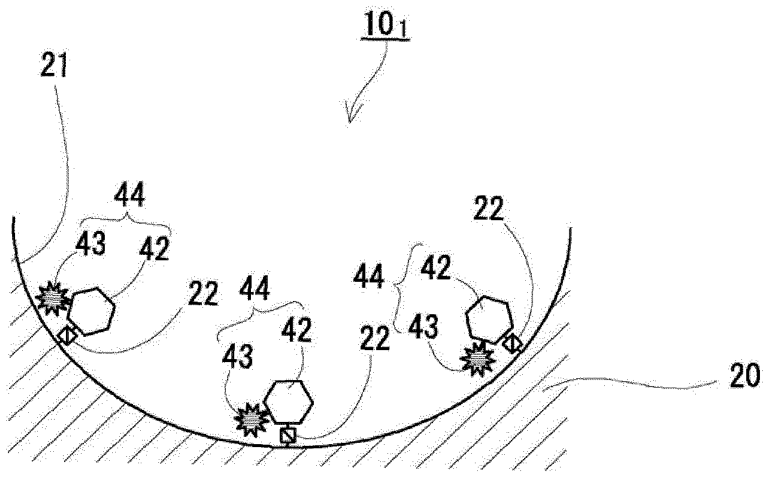

[Figure 2A]

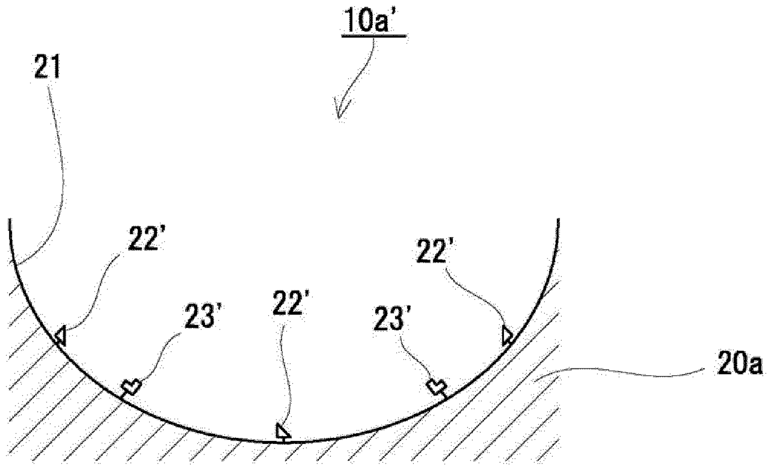


[Figure 2B-1]

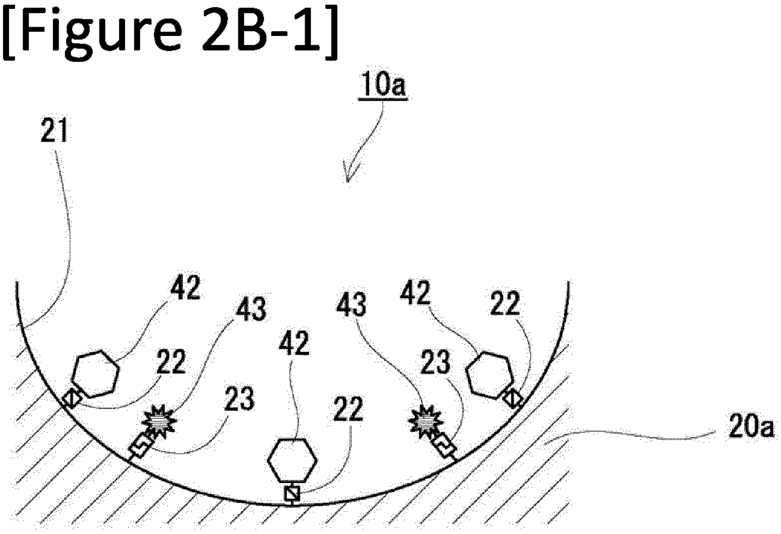


[Figure 2B-2]

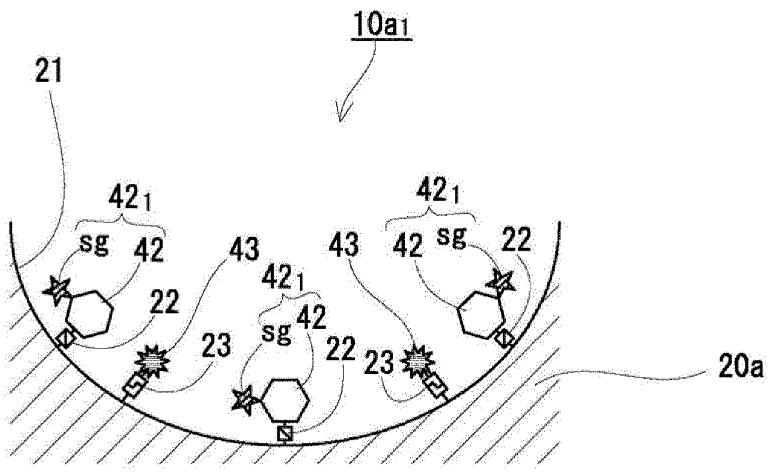

[Figure 3A]

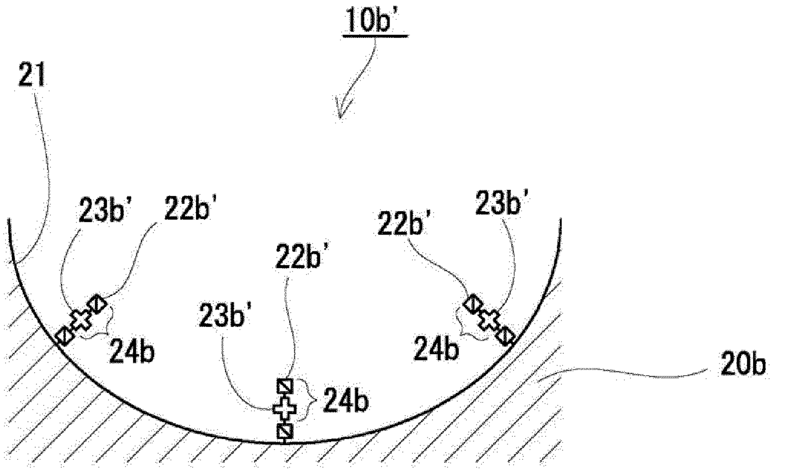


[Figure 3B]

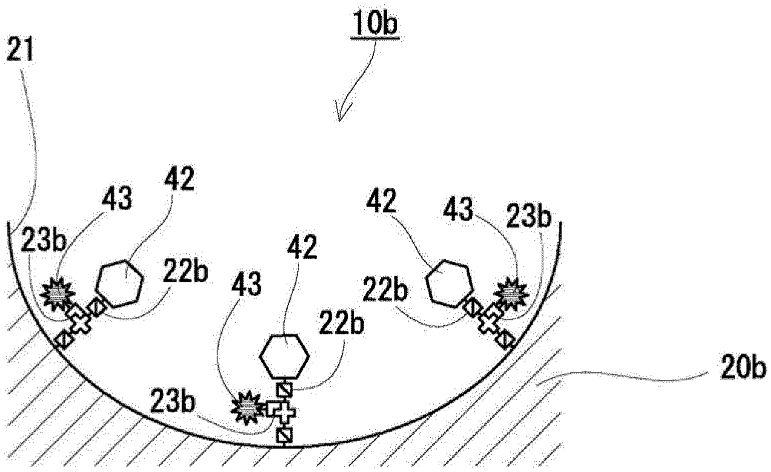


[Figure 4A]

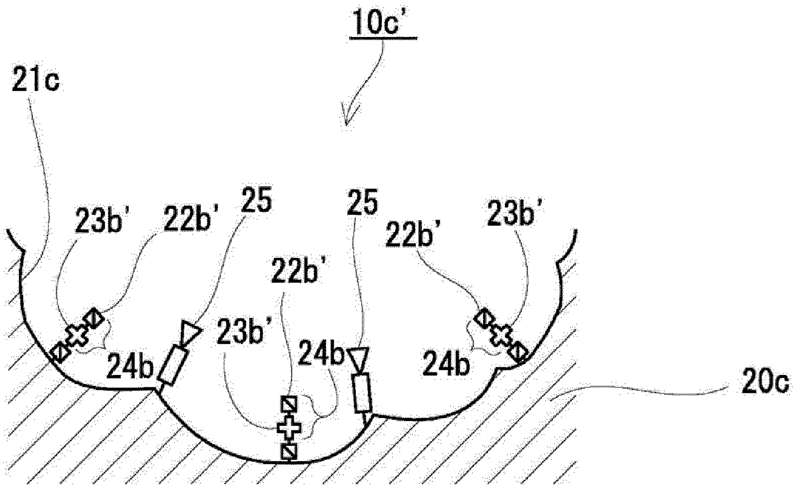

# [Figure 4B]

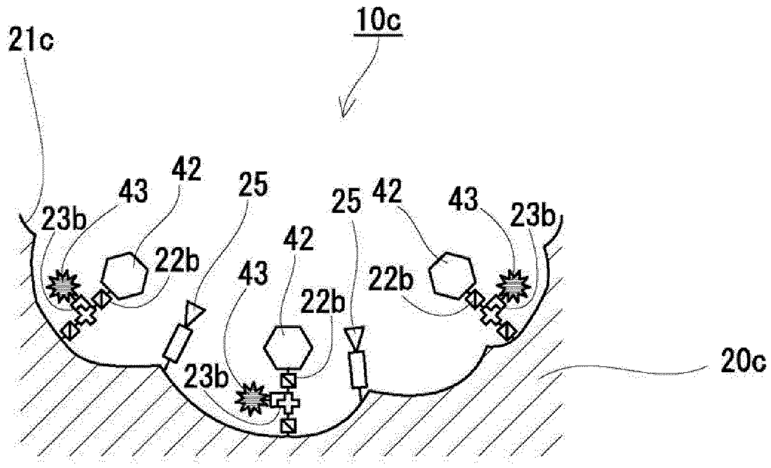


# [Figure 5]

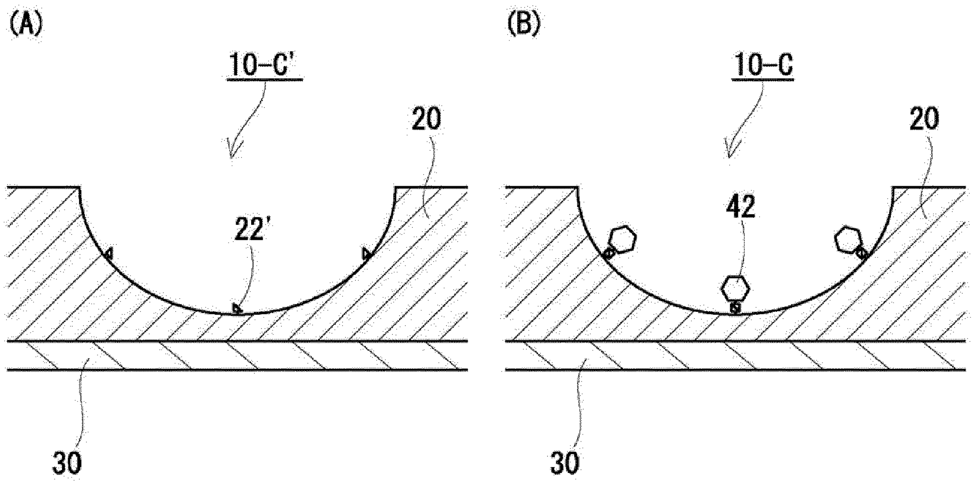


# [Figure 6]

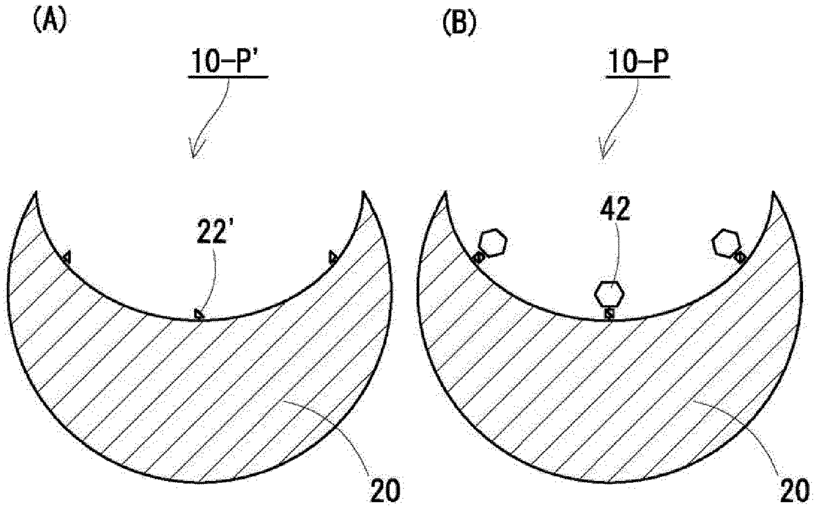

# [Figure 7]

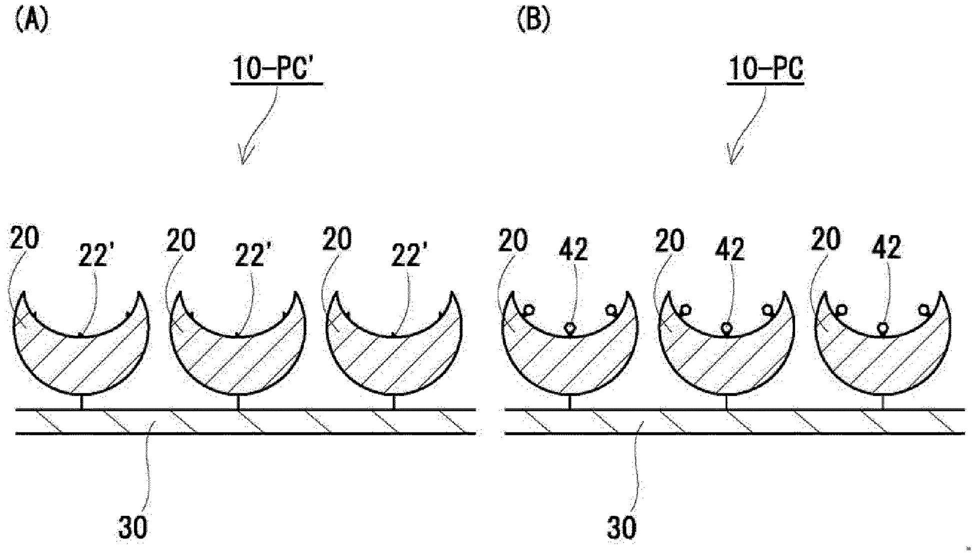


# [Figure 8]

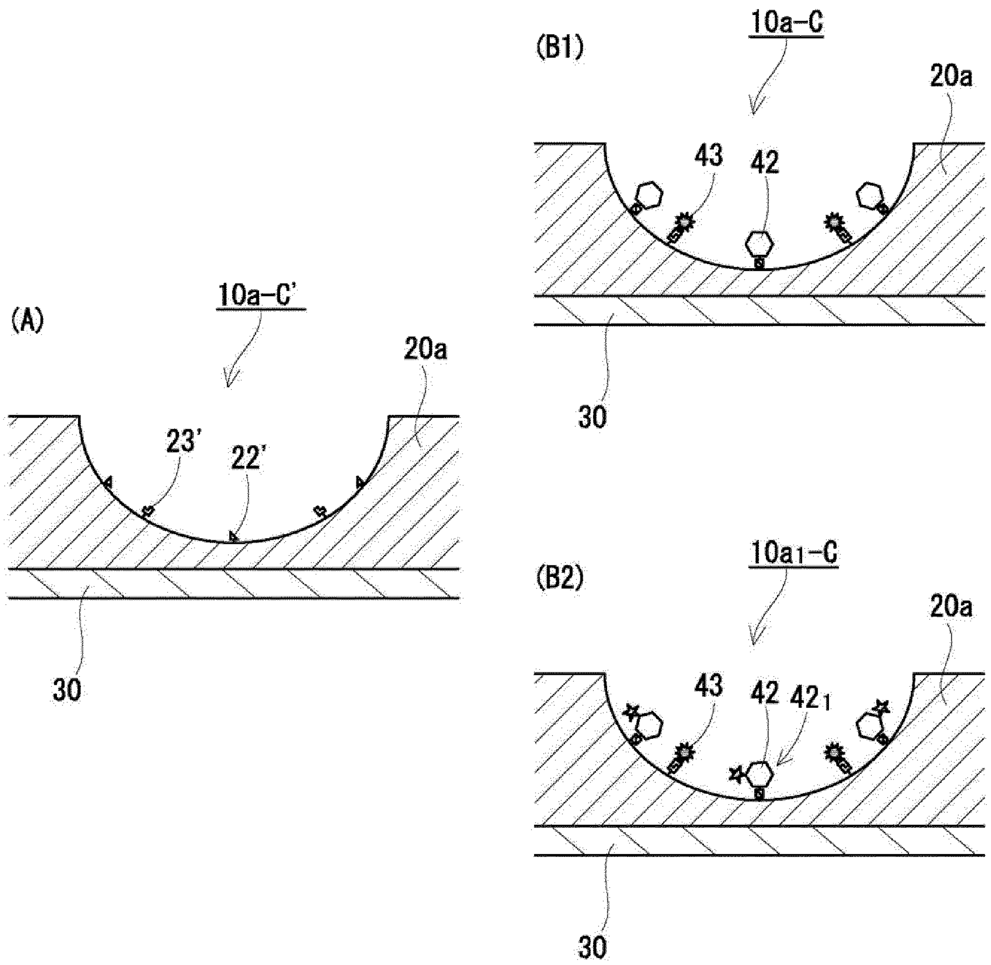

[Figure 9]

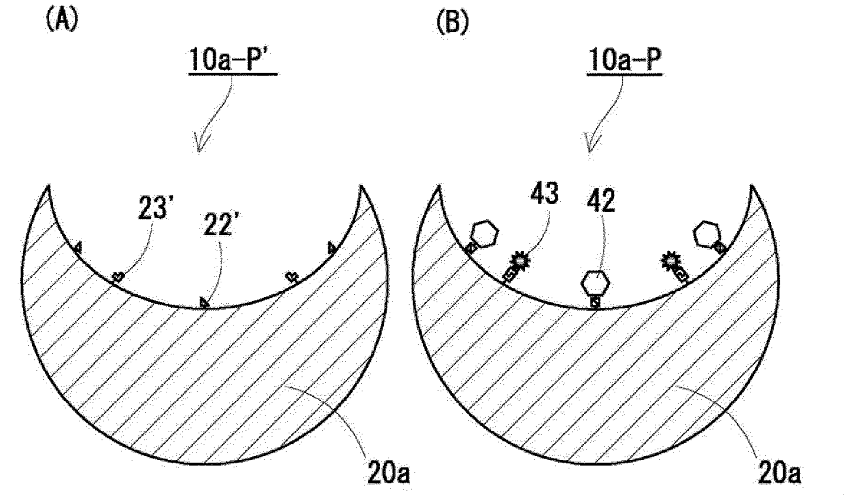


[Figure 10]

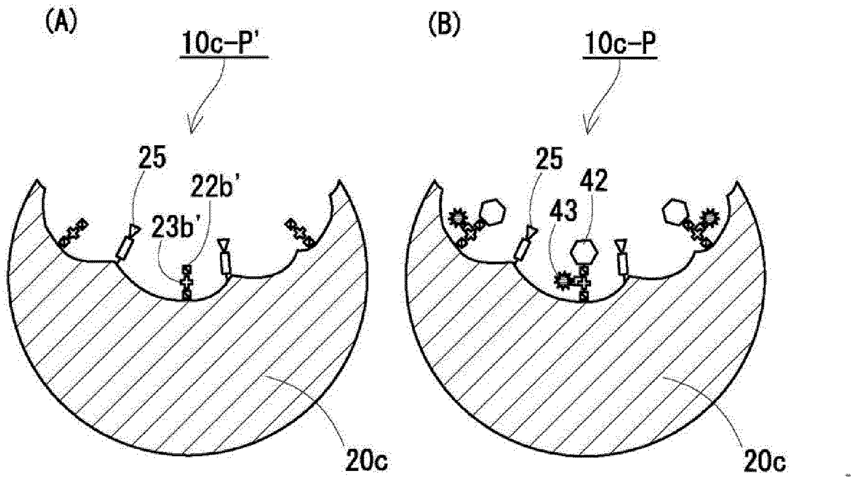


[Figure11]

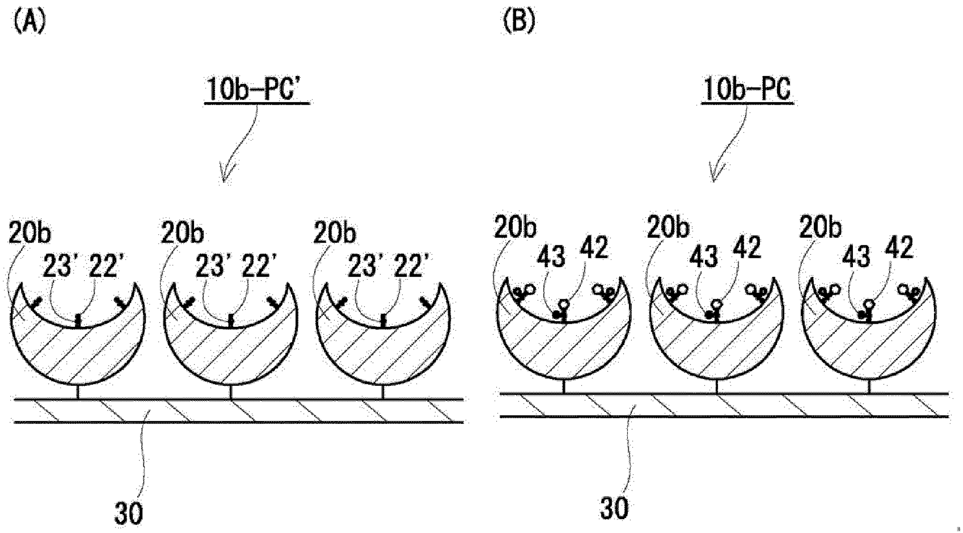

[Figure 12-1a]

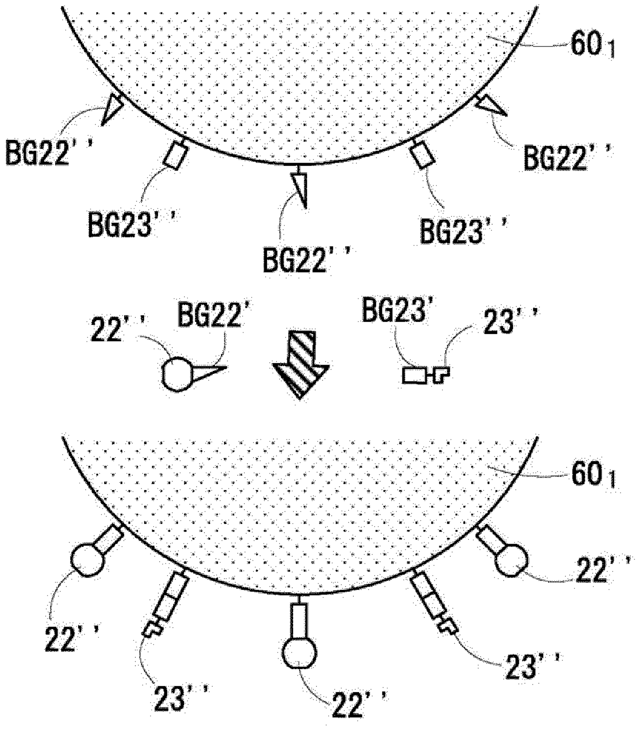


[Figure 12-1b]

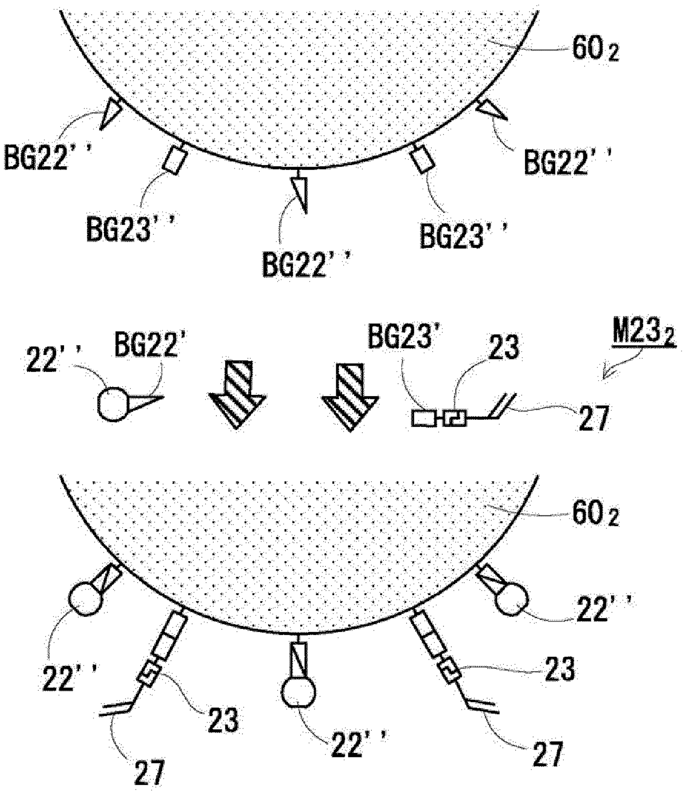

# [Figure 12-2]

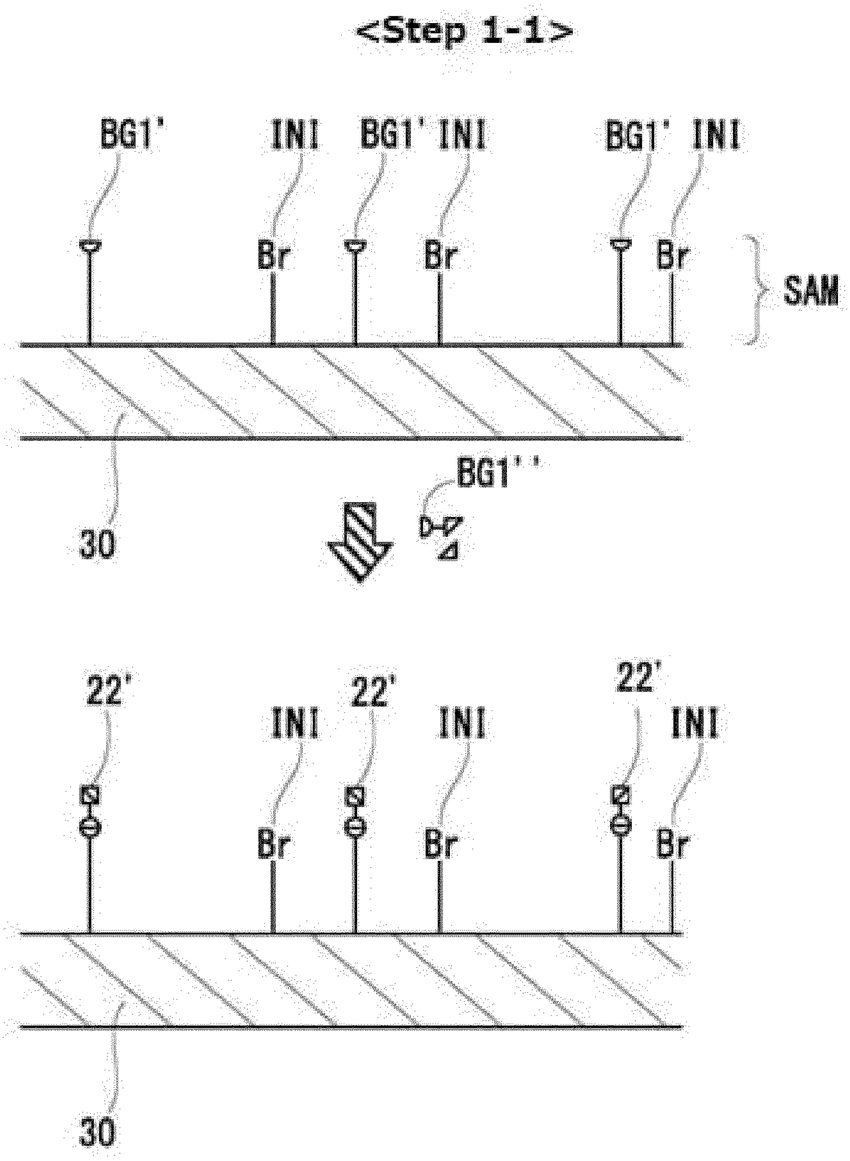
<Step 1-1>
BG1'
INI
BG1'
INI
BG1'
INI
Br
Br
Br
SAM
30
BG1''
22'
22'
22'
INI
INI
INI
Br
Br
Br
30

# [Figure 12-3a]

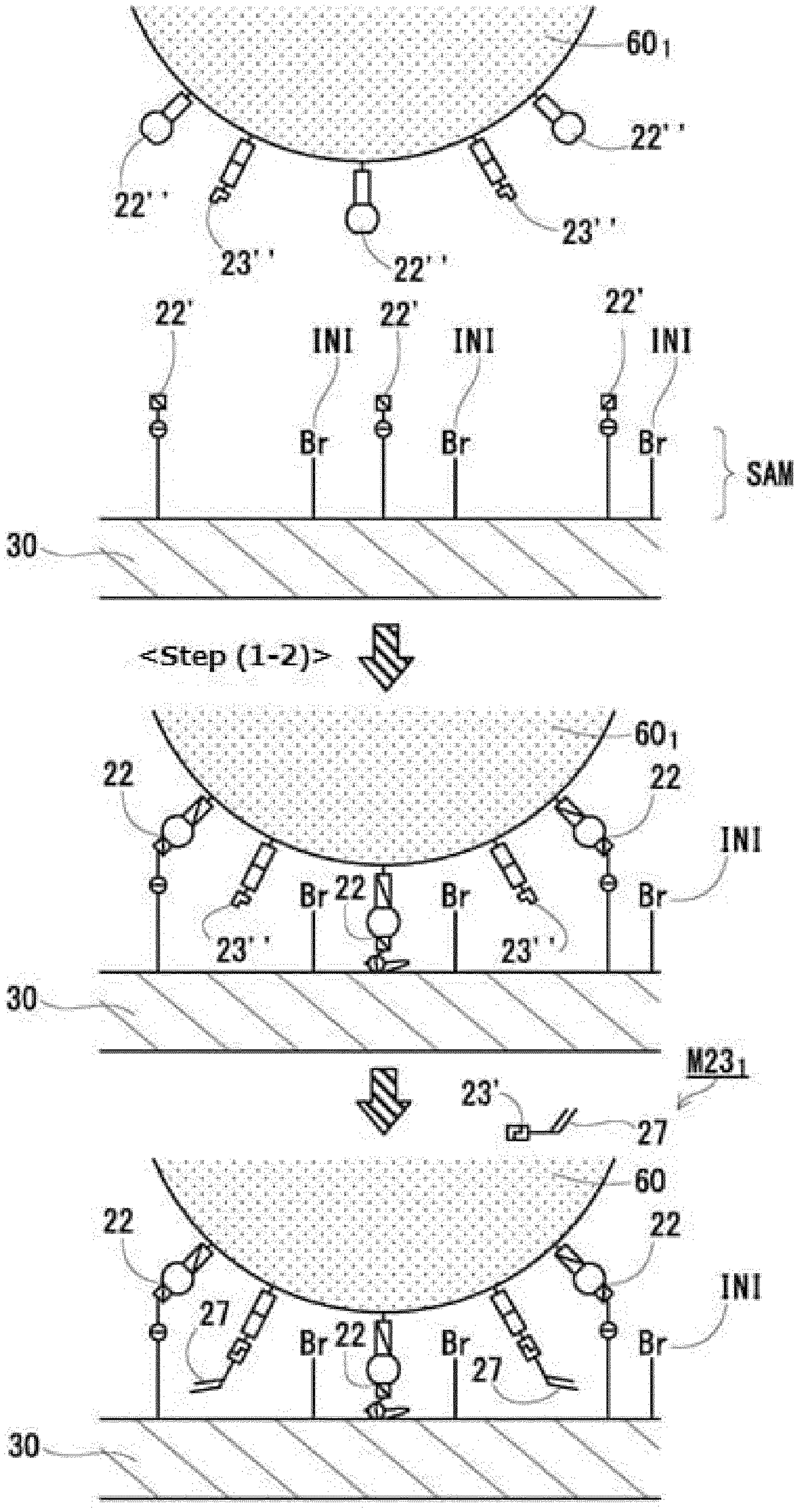

60₁
22''
22''
23''
23''
22''
23''
22'
22'
22'
INI
INI
INI
Br
Br
Br
SAM
30
<Step (1-2)>
60₁
22
22
INI
Br
22
Br
Br
23''
23''
30
M23₁
23'
27
60
22
22
INI
27
Br
22
Br
Br
27
30

# [Figure 12-3b]

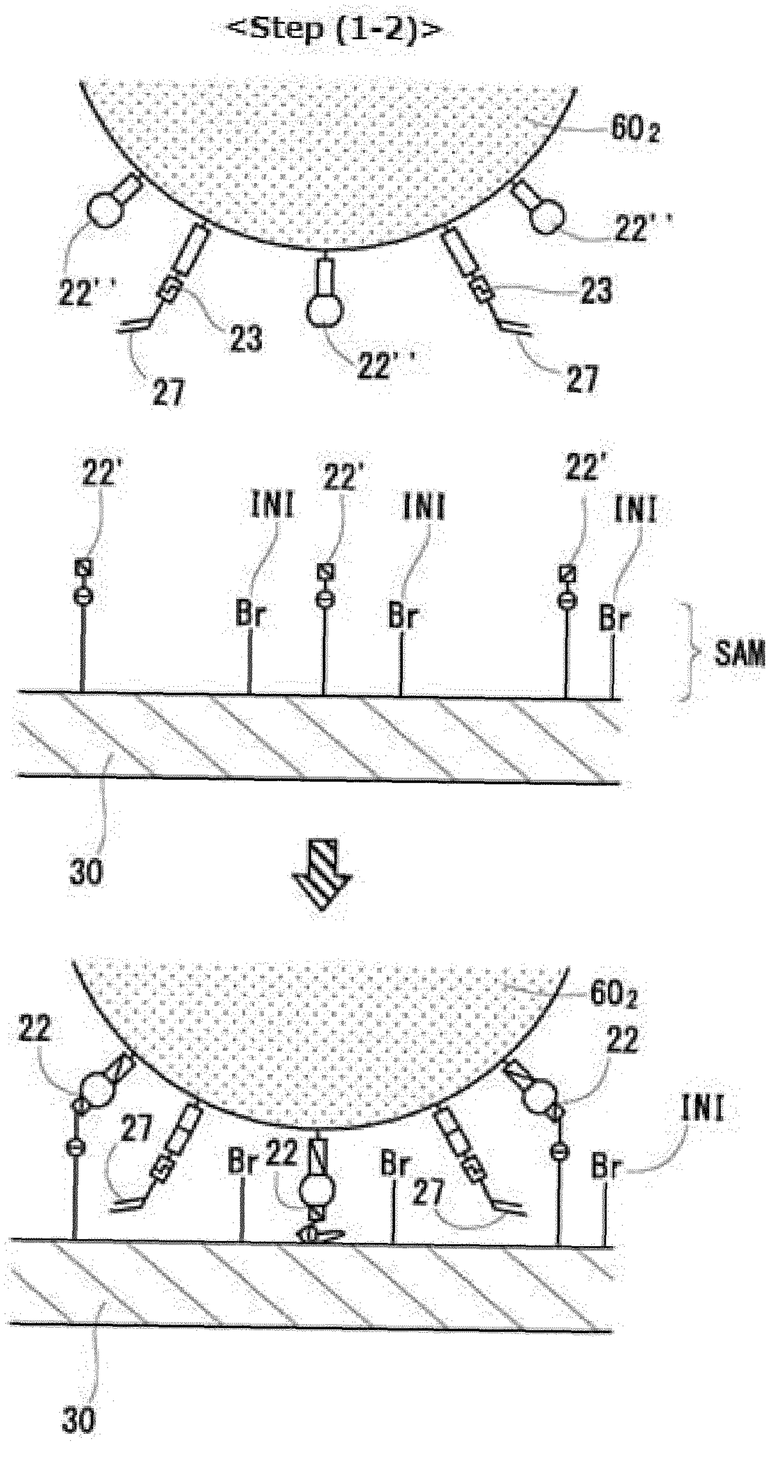
<Step (1-2)>
60₂
22''
22''
23
23
22''
27
27
22'
22'
22'
INI
INI
INI
Br
Br
Br
SAM
30
60₂
22
22
INI
27
Br
22
Br
27
Br
30

[Figure 12-4]

<Step (1-3)>

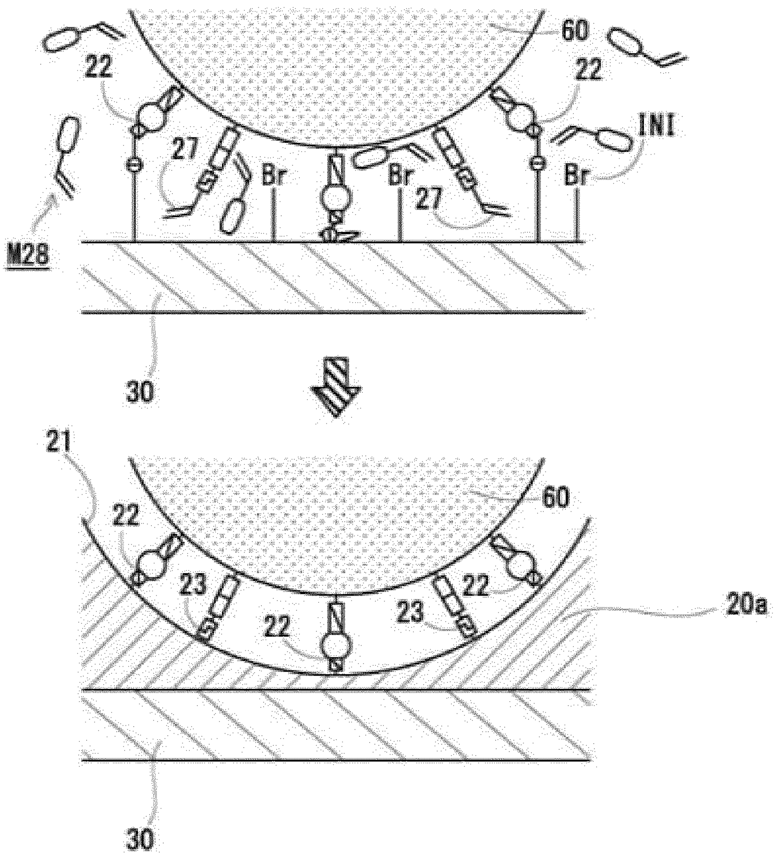


[Figure 12-5]

<Step (1-4)>

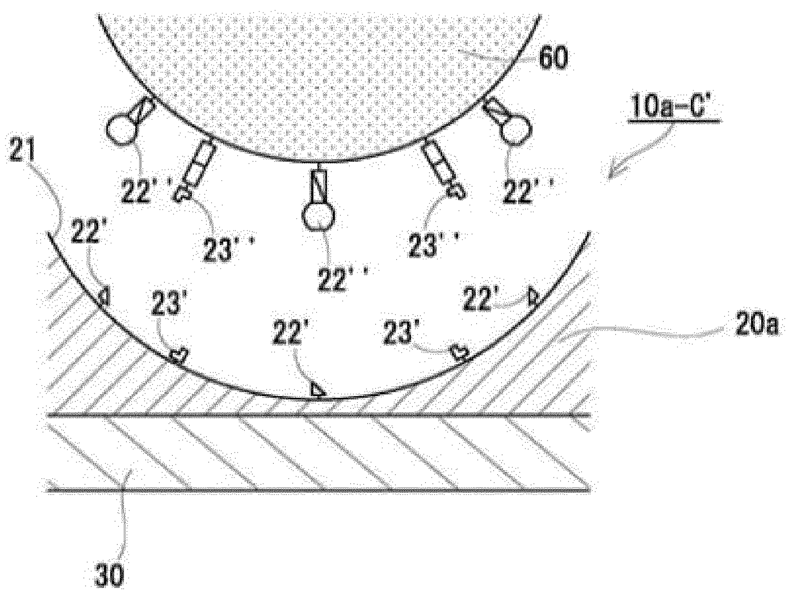

# [Figure 12-6]

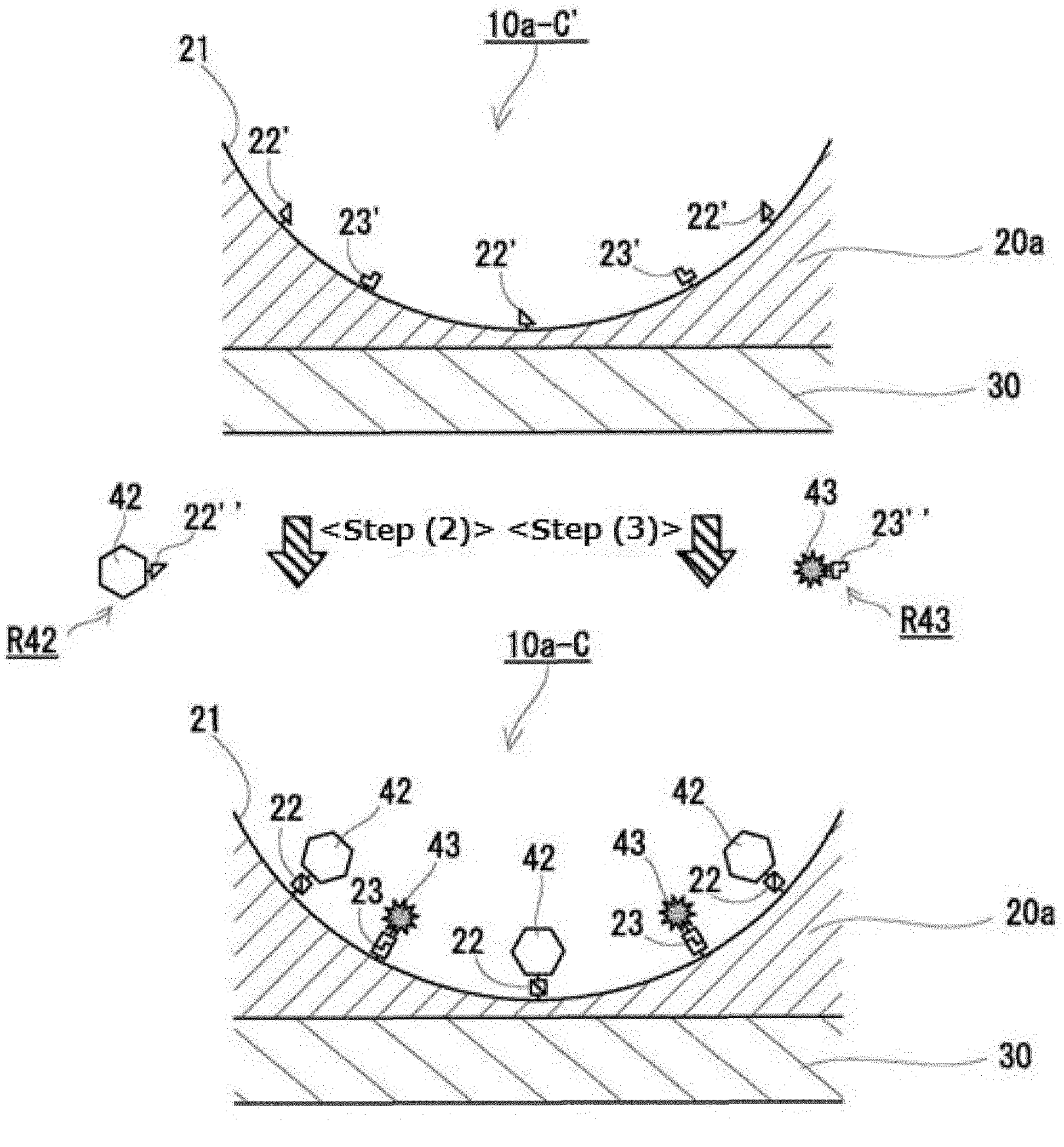
10a-C'
21
22'
23'
22'
22'
23'
20a
30
42
22''
<Step (2)> <Step (3)>
43
23''
R42
R43
10a-C
21
22
42
43
23
42
22
43
42
22
23
20a
30

# [Figure 13-1a]

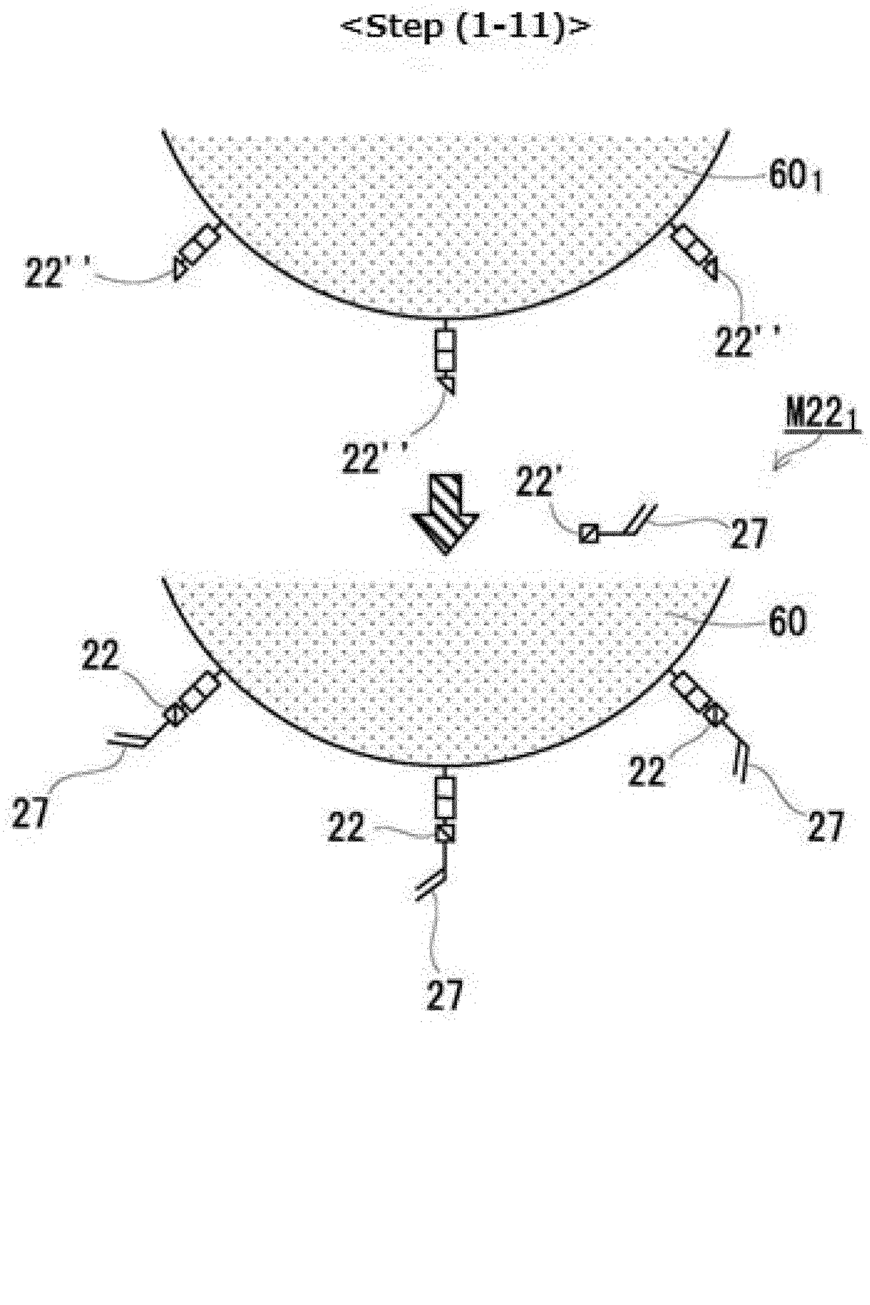

# [Figure 13-1b]

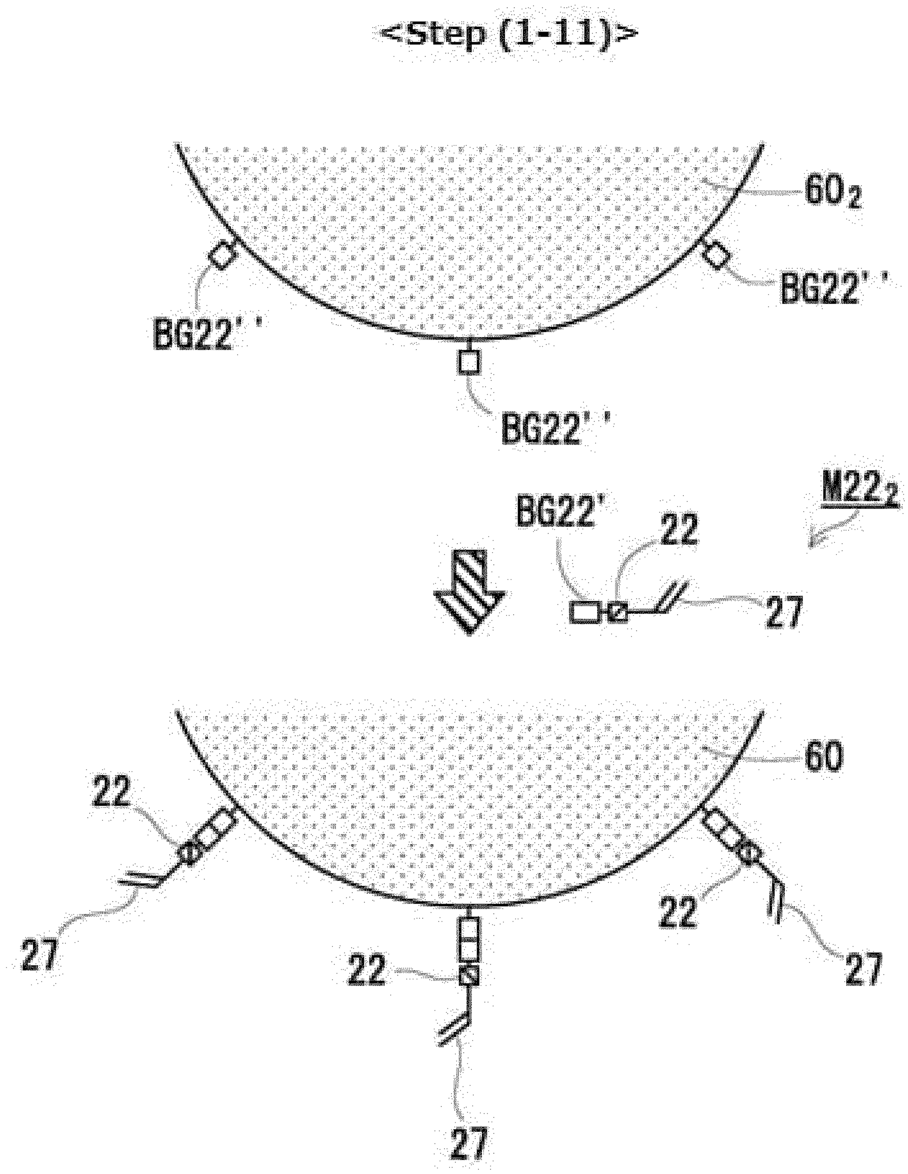

[Figure 13-2]

<Step (1-12)>

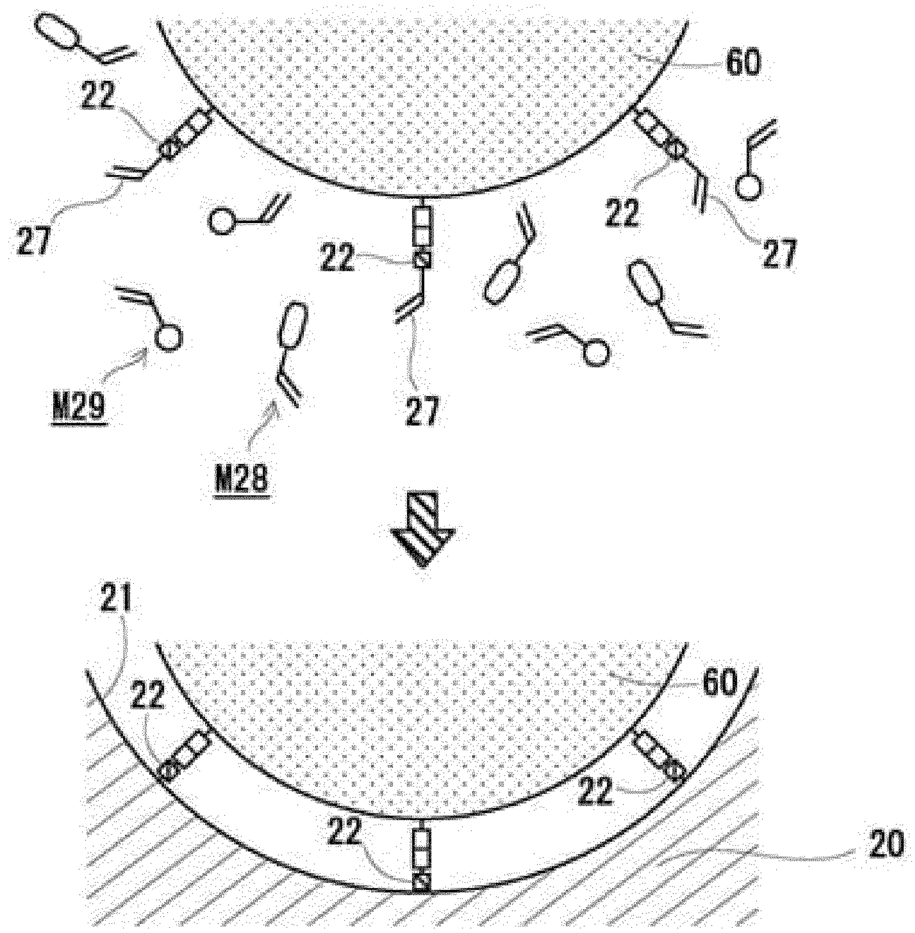

[Figure 13-3]

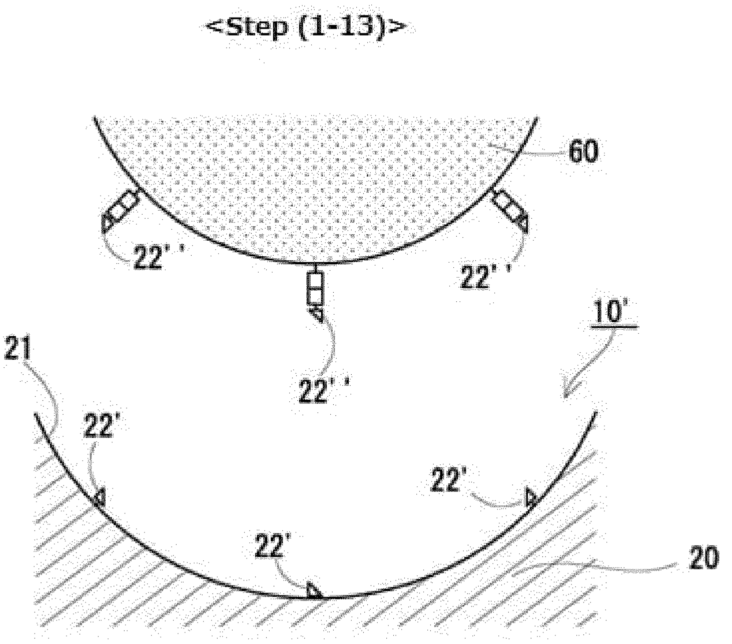


[Figure 13-4]

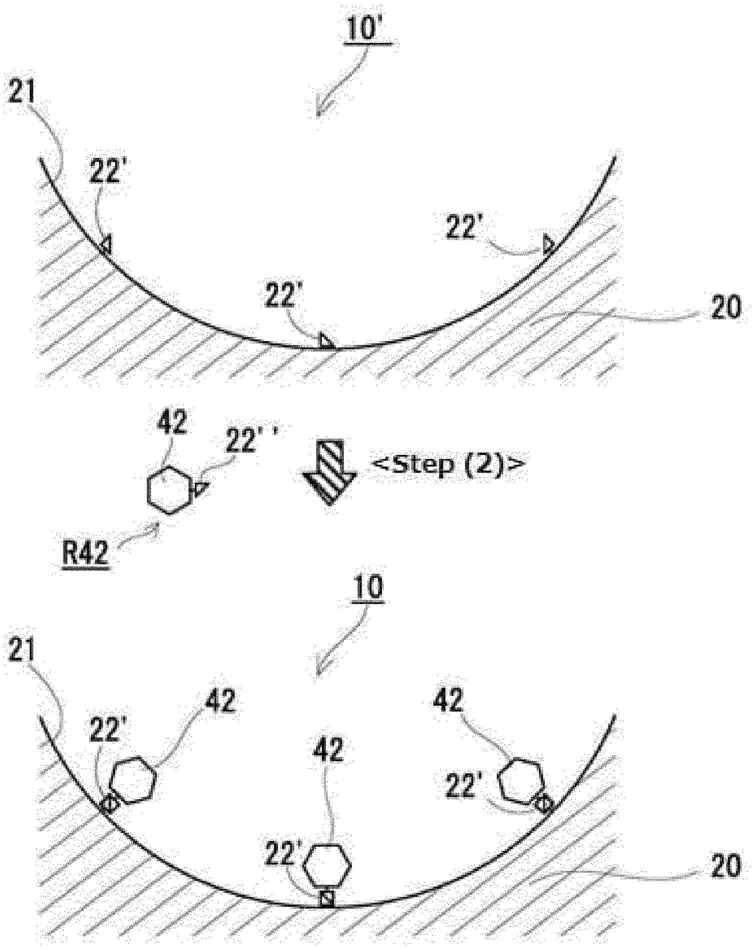

# [Figure 14-1]

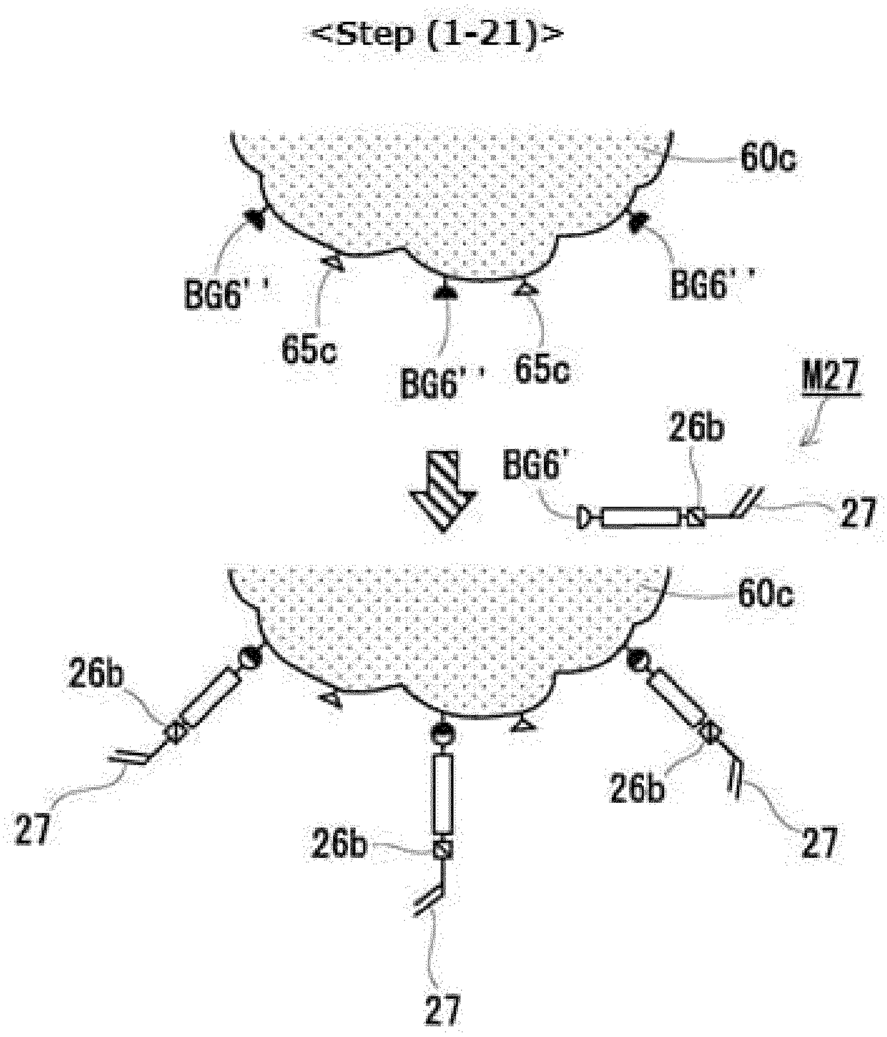
<Step (1-21)>
60c
BG6''
65c
BG6''
65c
BG6''
M27
26b
BG6'
27
60c
26b
27
26b
27
26b
27

# [Figure 14-2]

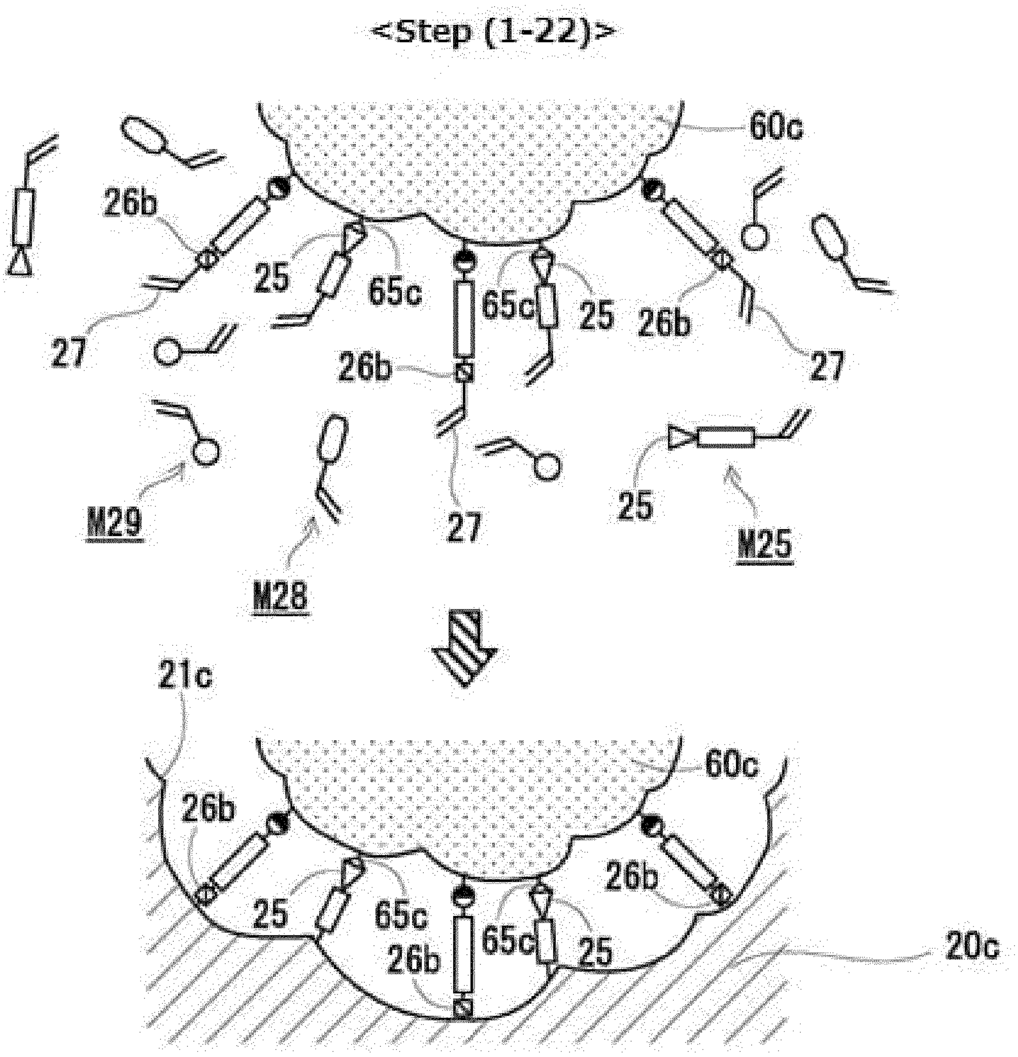
<Step (1-22)>
60c
26b
25
65c
65c
25
26b
27
27
26b
27
M29
M28
27
25
M25
21c
60c
26b
26b
25
65c
65c
26b
25
20c

# [Figure 14-3]

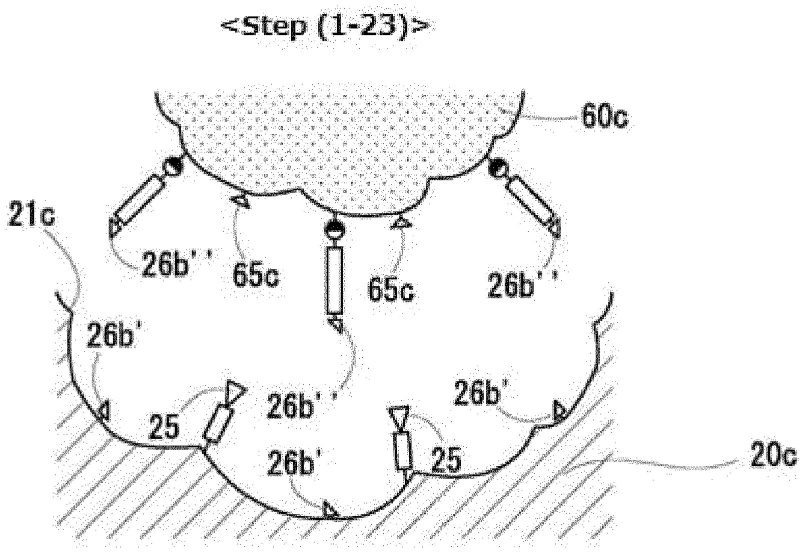


# [Figure 14-4]

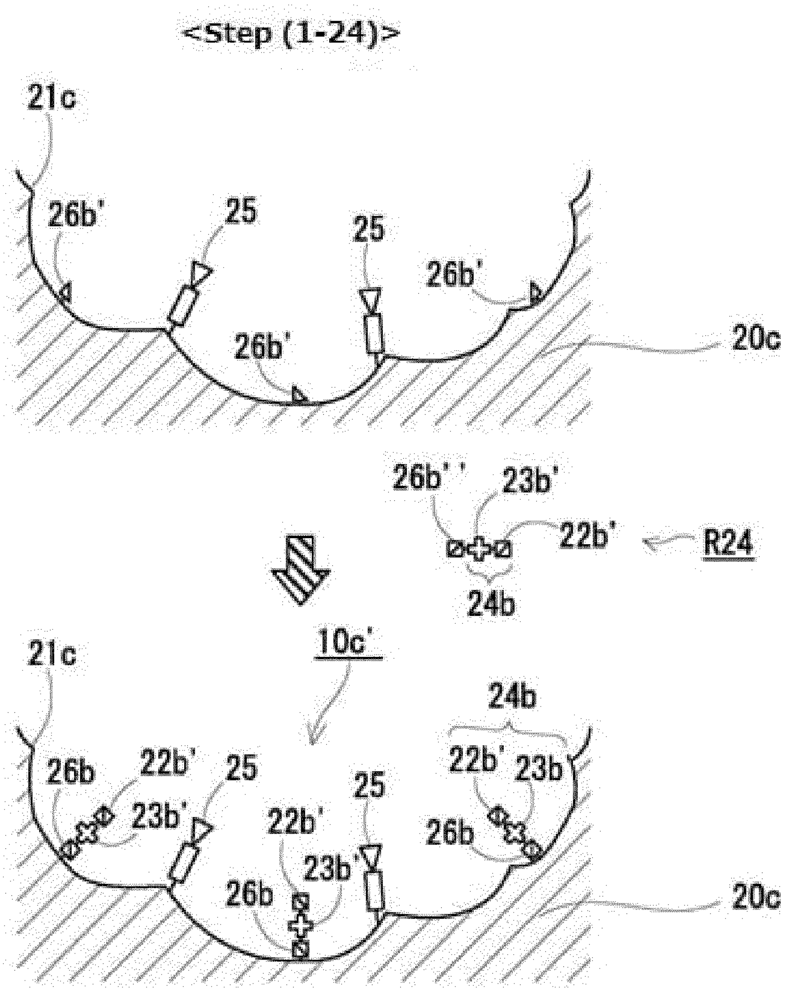

[Figure 14-5]

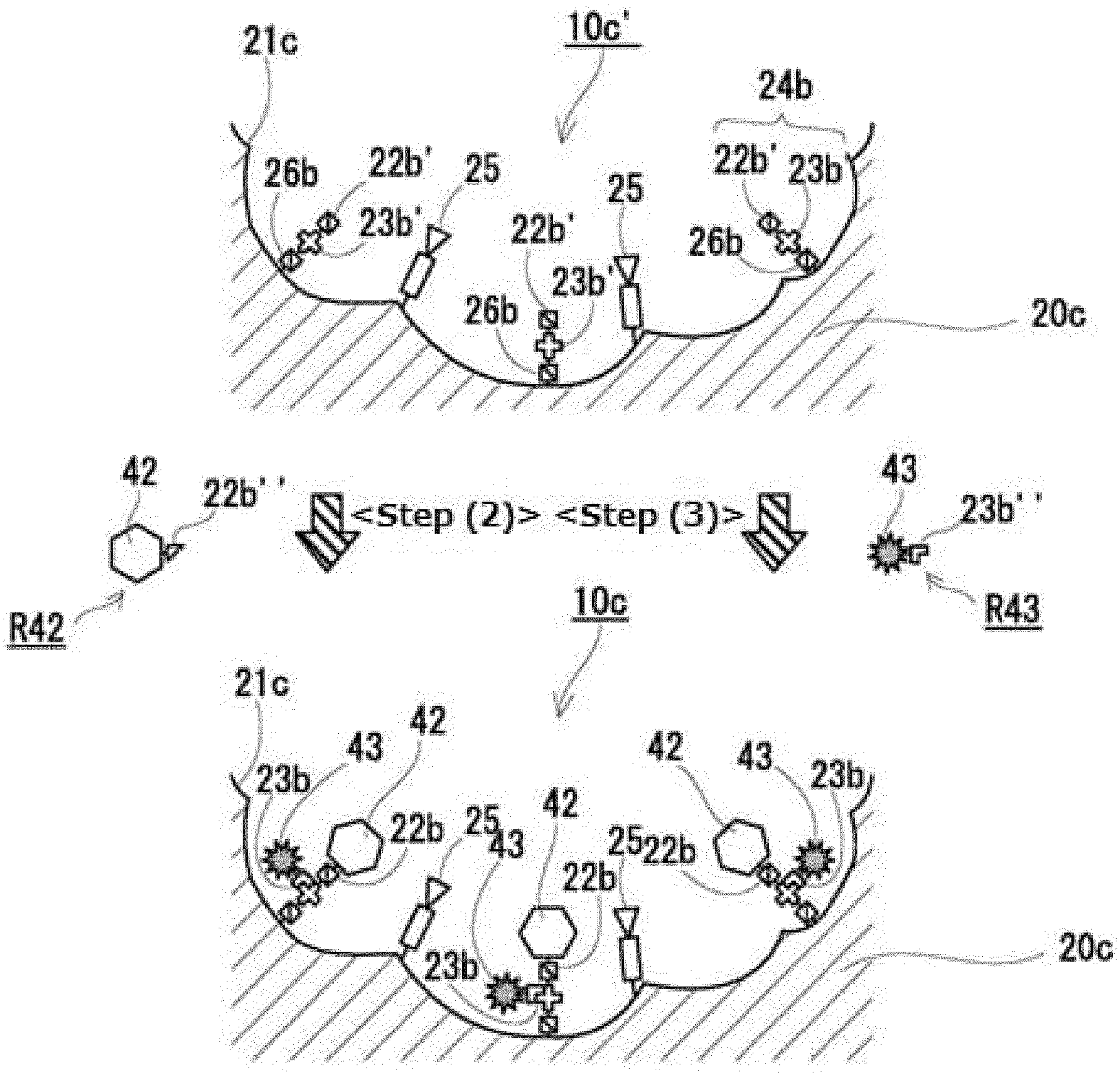
10c'
21c
24b
22b'
23b'
26b
25
22b'
23b'
26b
25
22b'
23b'
26b
20c
42
22b''
R42
<Step (2)> <Step (3)>
43
23b''
R43
10c
21c
23b
43
42
22b
25
43
42
22b
23b
25
22b
42
43
23b
20c

[Figure 15]

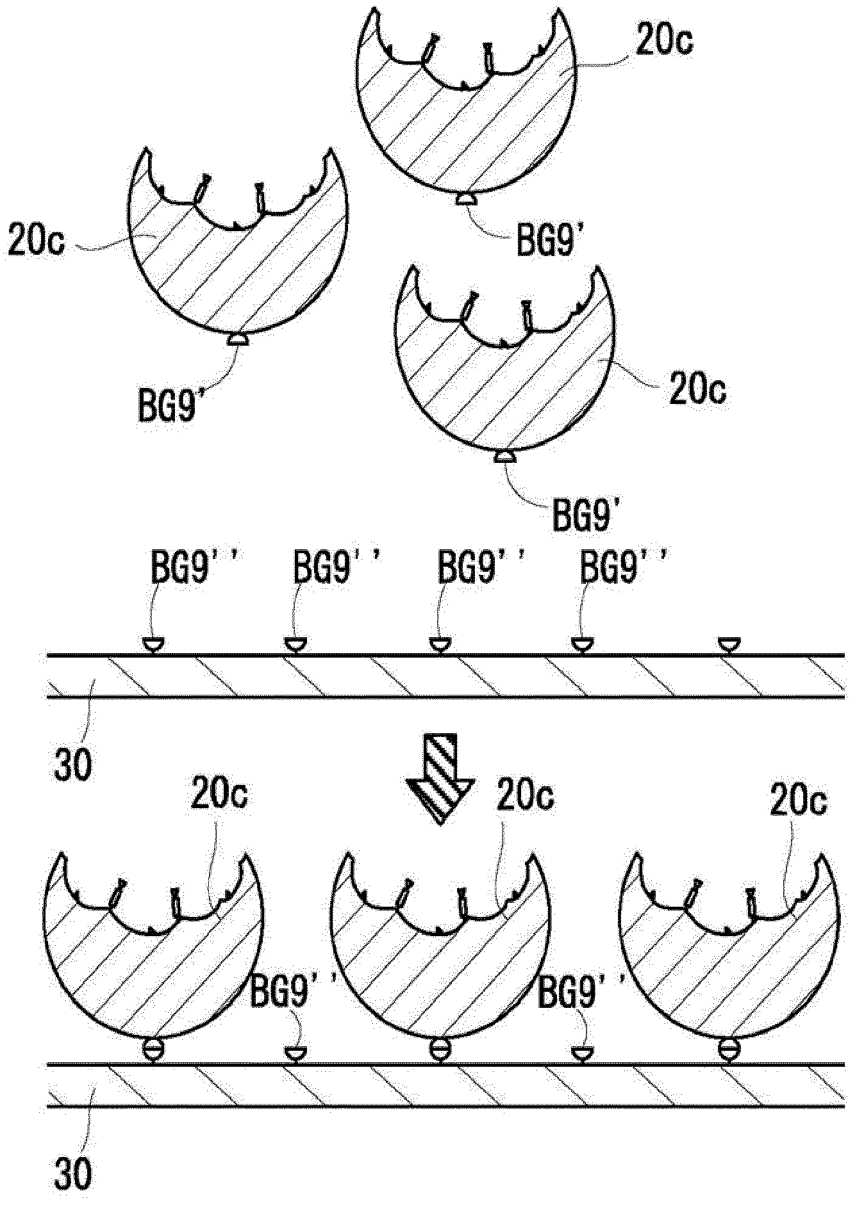

# [Figure 16-1]

(i) (ii)

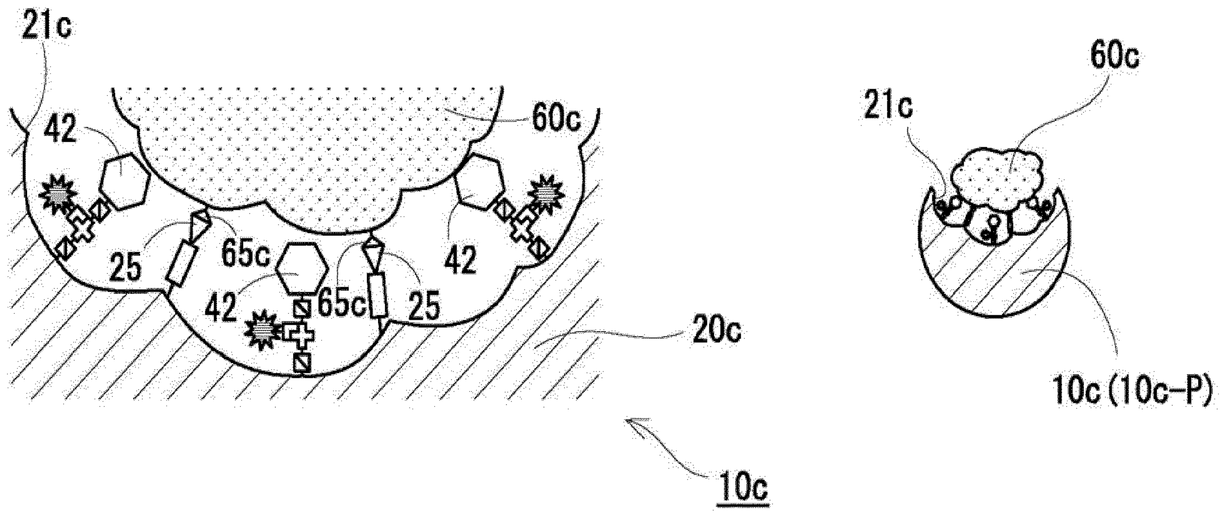


# [Figure 16-2]

(i) (ii)

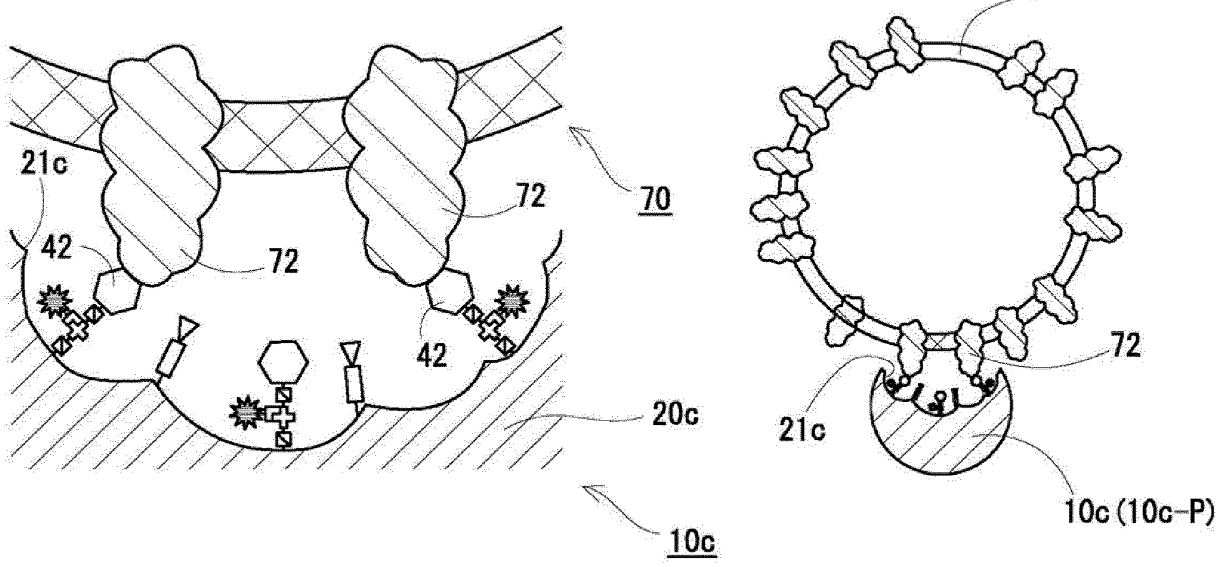

[Figure 17A]

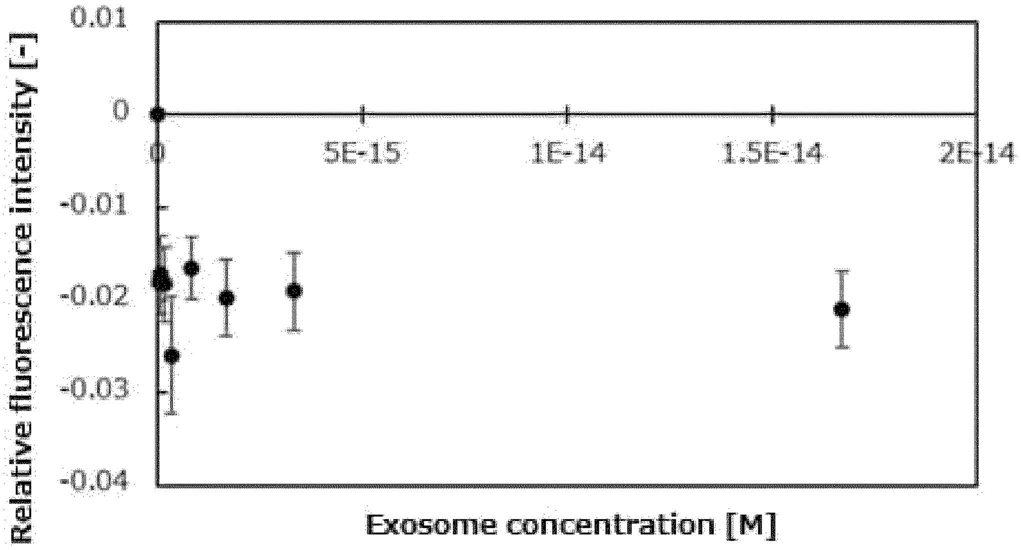


Example 1

[Figure 17B]

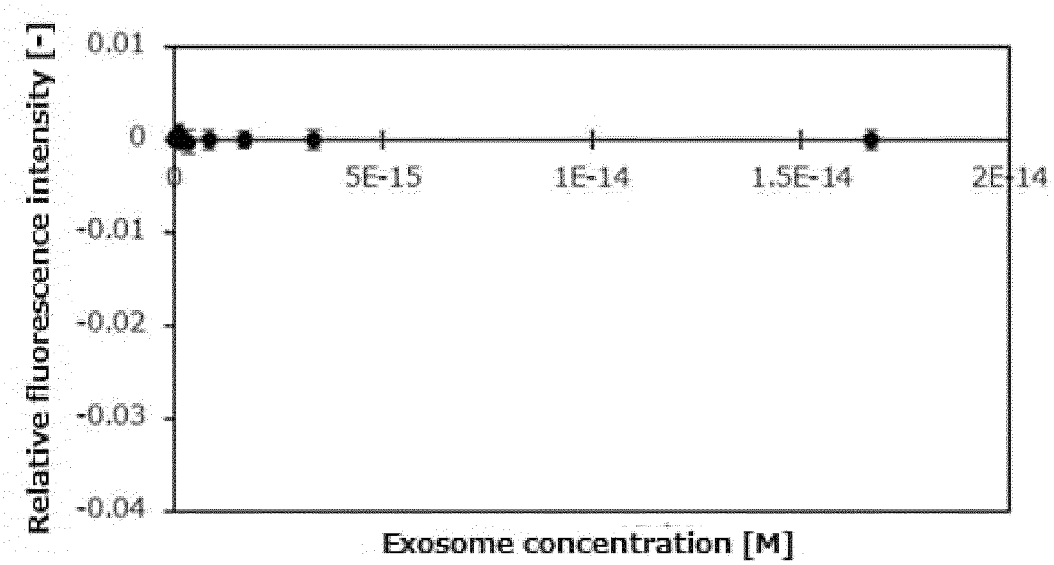


Comparative Example 1

[Figure 18]

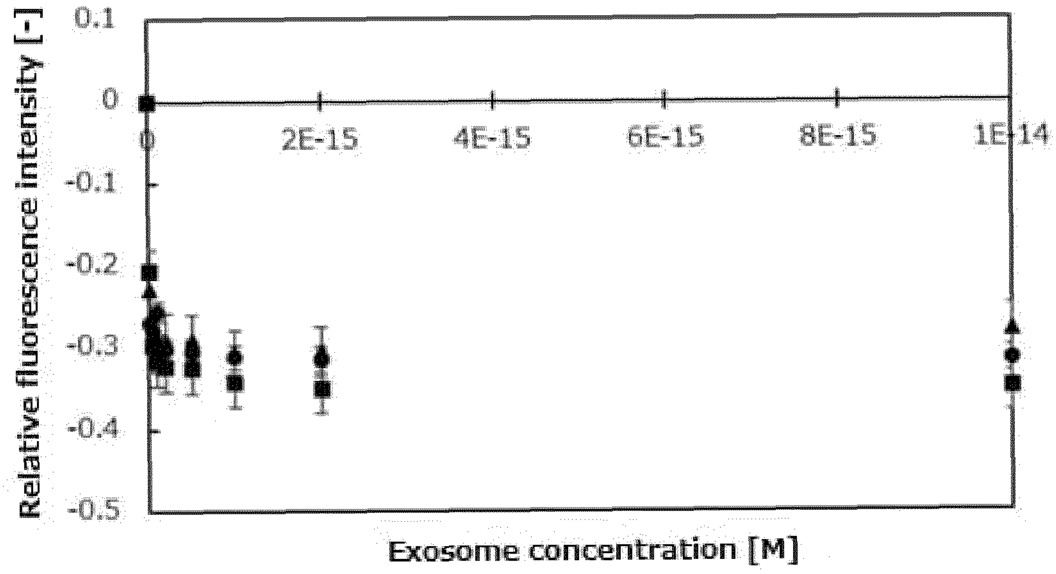


Example 2

[Figure 19]

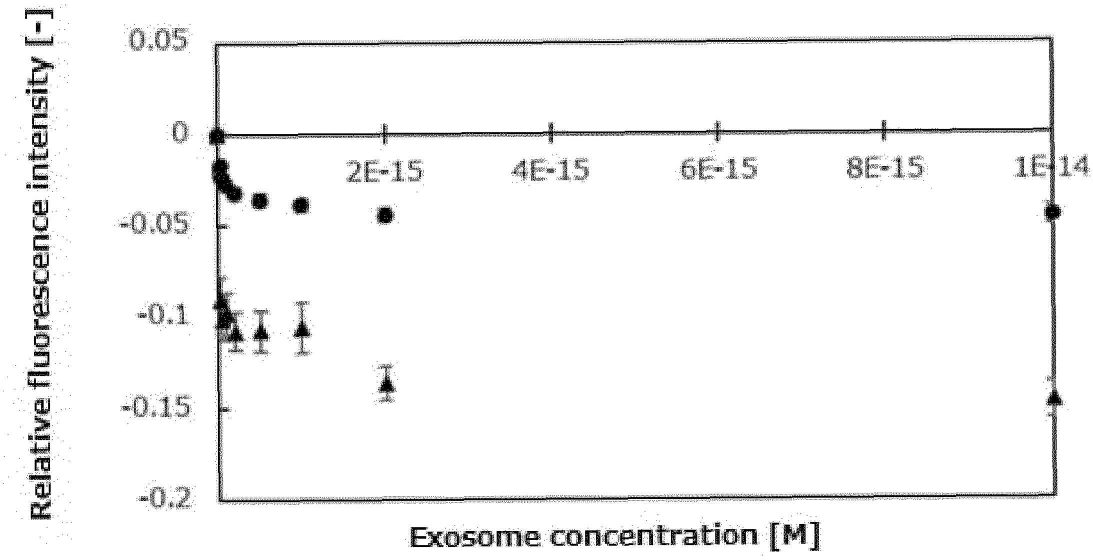


Example 3

# [Figure 20]

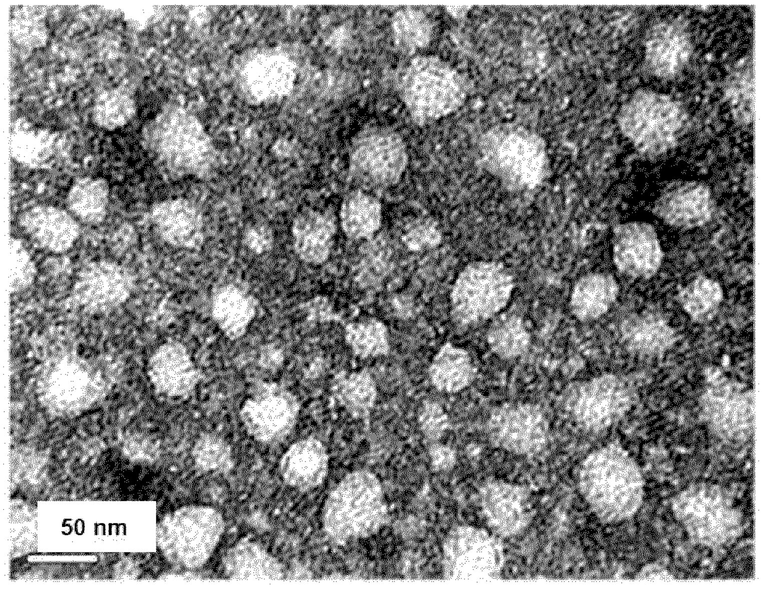


# [Figure 21]

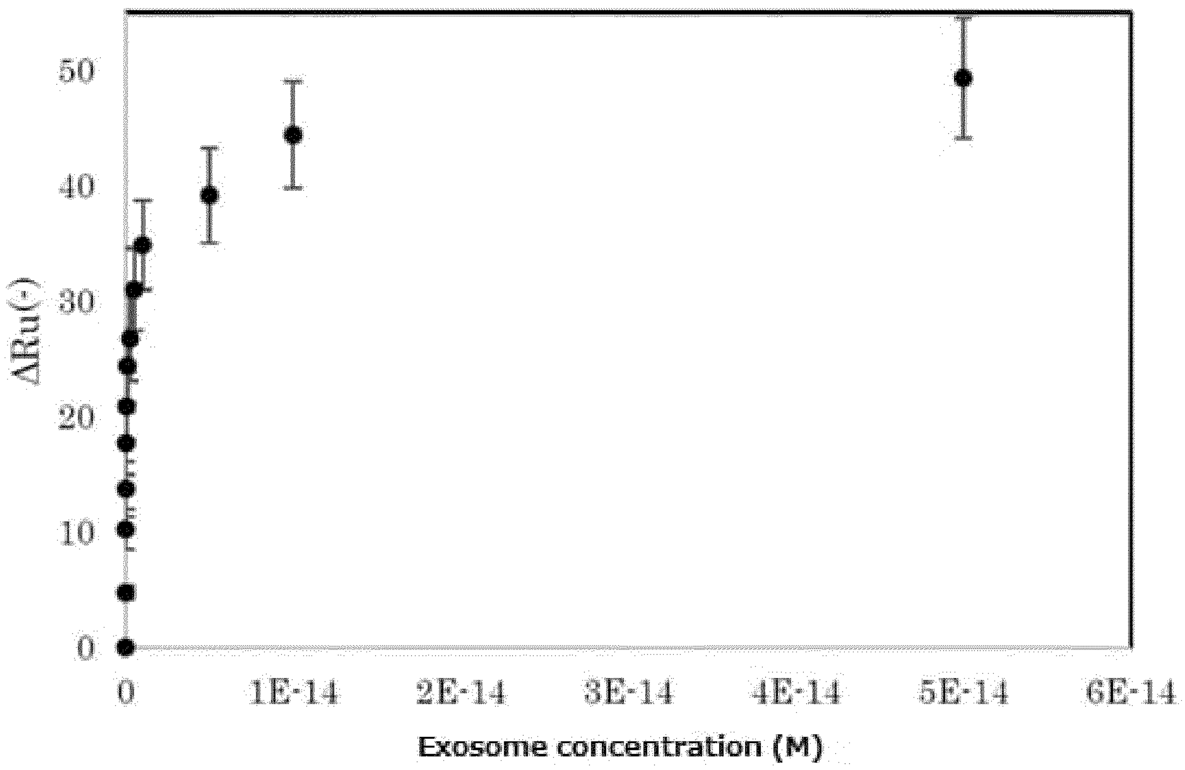


Example 4

# [Figure 22]

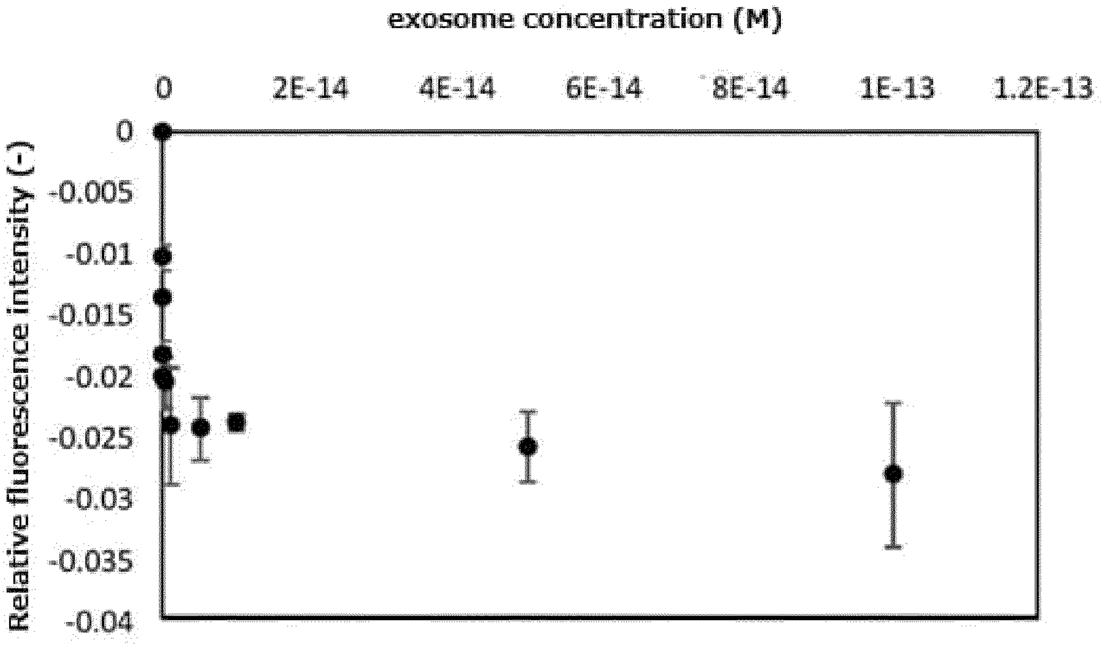


Example 5

# [Figure 23]

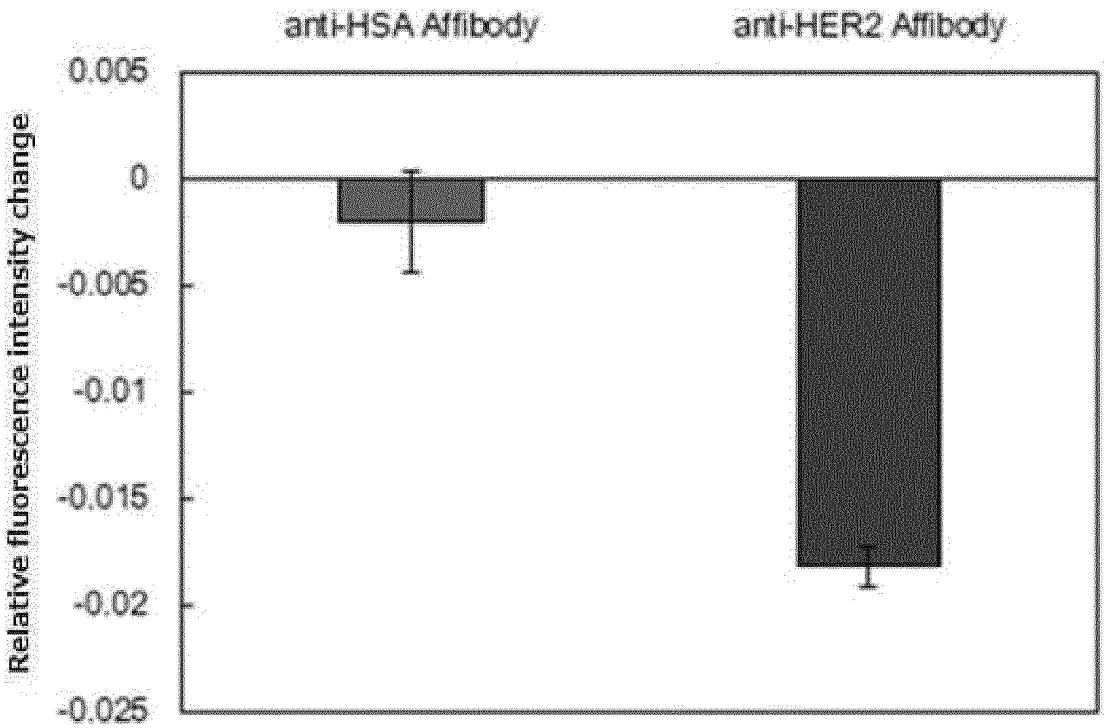

[Figure 24]

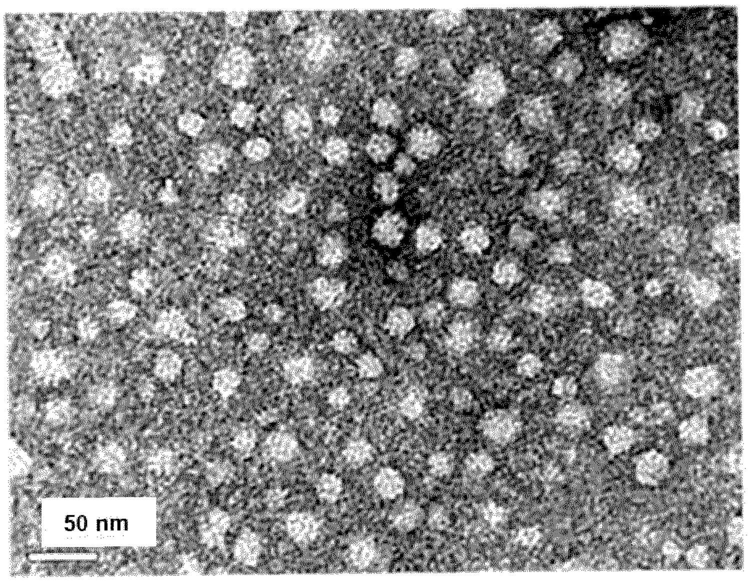


[Figure 25]

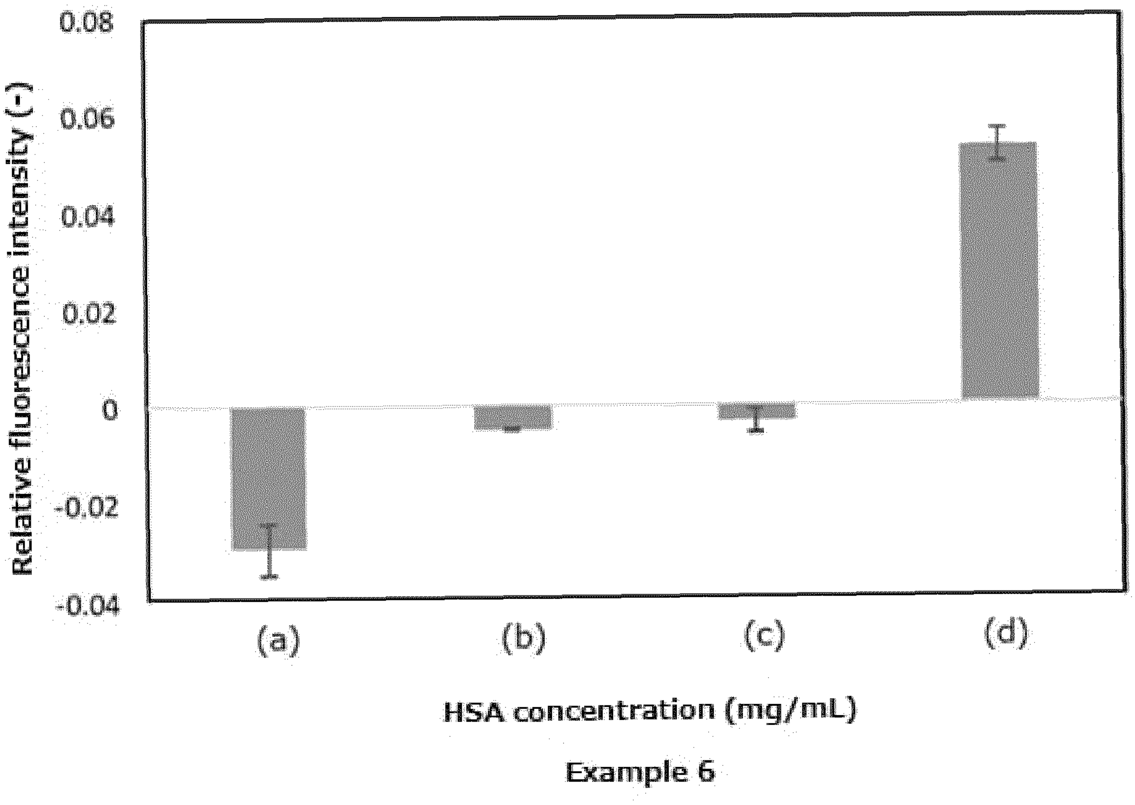

## INTERNATIONAL SEARCH REPORT

International application No. PCT/JP2021/031621

**A. CLASSIFICATION OF SUBJECT MATTER**

***G01N 33/53***(2006.01)i; ***G01N 33/531***(2006.01)i; ***G01N 33/543***(2006.01)i; ***G01N 33/545***(2006.01)i
FI: G01N33/531 B; G01N33/543 501M; G01N33/545 Z; G01N33/53 S

According to International Patent Classification (IPC) or to both national classification and IPC

**B. FIELDS SEARCHED**

Minimum documentation searched (classification system followed by classification symbols)

G01N33/53; G01N33/531; G01N33/543; G01N33/545

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2018/221271 A1 (NATIONAL UNIVERSITY CORP. KOBE UNIVERSITY) 06 December 2018 (2018-12-06) see entire text, all drawings, particularly, claims, paragraphs [0015]-[0042], [0043]-[0085], [0120]-[0131], fig. 1-2, 5-7, 9-12, etc. | 1-34<br>14 |
| X<br>Y | JP 2006-138656 A (KYOWA MEDEX CO., LTD.) 01 June 2006 (2006-06-01) see entire text, all drawings, particularly, claims, paragraphs [0010]-[0038], etc. | 1-2, 7-13, 15<br>14, 16-17 |
| Y | CN 104941602 A (DALIAN CHEMICAL PHYSICS INST.) 30 September 2015 (2015-09-30) see entire text, particularly, paragraphs [0021]-[0033], etc. | 16-17 |
| Y | CN 104744702 A (UNIV. HUAZHONG AGRICULTURAL) 01 July 2015 (2015-07-01) see entire text, particularly, claims, etc. | 16-17 |
| A | WO 2012/124800 A1 (SHIBAURA INST. OF TECHNOLOGY) 20 September 2012 (2012-09-20) see entire text, all drawings, etc. | 1-34 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 November 2021** | **16 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/031621**

| Patent document cited in search report | Publication date (day/month/year) | Patent family member(s) | Publication date (day/month/year) |
|---|---|---|---|
| WO 2018/221271 A1 | 06 December 2018 | US 2020/0110084 A1<br>see entire text, all drawings, particularly, claims, paragraphs [0069]-[0098], [0099]-[0157] , [0199]-[0210], fig. 1-2, 5-7, 9-12, etc.<br>EP 3647786 A1 | |
| JP 2006-138656 A | 01 June 2006 | (Family: none) | |
| CN 104941602 A | 30 September 2015 | (Family: none) | |
| CN 104744702 A | 01 July 2015 | (Family: none) | |
| WO 2012/124800 A1 | 20 September 2012 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020159008 A
- JP 2021007726 W